# EUROPEAN PATENT SPECIFICATION

(11) **EP 2 591 365 B1**
(45) Date of publication and mention of the grant of the patent: **13.01.2016**
(21) Application number: 11731322.1
(22) Date of filing: 06.07.2011
(51) Int. Cl.: G01N 33/68

(54) **MARKERS OF VULNERABILITY OF THE ATHEROSCLEROSIS PLAQUE**
Markers der Verletzlichkeit von Atherosklerotischem Plaque
Marqueurs du vulnérabilité de plaque d'athérome

(30) Priority: 06.07.2010 EP 10305748; 06.07.2010 US 361700 P
(43) Date of publication of application: 15.05.2013
(73) Proprietor: Bio-Rad Innovations, 92430 Marnes-La-Coquette (FR); Centre National de la Recherche Scientifique (CNRS), 75016 Paris (FR)
(72) Inventor: FAREH, Jeannette, F-34160 Saussines (FR); MALAUD, Eric, 30670 AIGUES VIVES (FR)
(74) Representative: Blot, Philippe Robert Emile
(86) International application number: PCT/EP2011/061400
(87) International publication number: WO 2012/004301

(56) References cited:
- EP-A1- 1 394 267
- EP-A1- 2 019 318
- WO-A1-2004/005931
- DE-A1-102006 048 249
- US-A1- 2009 093 005
- MEDBURY H J ET AL: "Monocytes contribute to the atherosclerotic cap by transformation into fibrocytes", INTERNATIONAL ANGIOLOGY, vol. 27, no. 2, April 2008 (2008-04), pages 114-123, XP008125340, ISSN: 0392-9590
- OH HIDEMASA ET AL: "Low expression of calponin in smooth muscle cells of coronary artery lesions identifies the high risk for restenosis after directional coronary atherectomy", JOURNAL OF THE AMERICAN COLLEGE OF CARDIOLOGY, vol. 27, no. 2 SUPPL. A, 1996, pages 112A-113A, XP002596010, & 45TH ANNUAL SCIENTIFIC SESSION OF THE AMERICAN COLLEGE OF CARDIOLOGY; ORLANDO, FLORIDA, USA; MARCH 24-27, 1996 ISSN: 0735-1097
- NEGORO NOBUYUKI ET AL: "Down expression of calponin in smooth muscle of coronary artery lesions identifies a group of lesions at high risk for restenosis after atherectomy: Implications for human gene therapy by direct calponin gene transfer", CIRCULATION, vol. 90, no. 4 PART 2, 1994, page I142, XP008125400, & 67TH SCIENTIFIC SESSIONS OF THE AMERICAN HEART ASSOCIATION; DALLAS, TEXAS, USA; NOVEMBER 14-17, 1994 ISSN: 0009-7322
- KANAKI TATSURO ET AL: "The regulatory expression of procollagen COOH-terminal proteinase enhancer in the proliferation of vascular smooth muscle cells", BIOCHEMICAL AND BIOPHYSICAL RESEARCH COMMUNICATIONS, vol. 270, no. 3, 21 April 2000 (2000-04-21), pages 1049-1054, XP002603519, ISSN: 0006-291X
- PILOP CHRISTIANE ET AL: "Proteomic analysis in aortic media of patients with Marfan syndrome reveals increased activity of calpain 2 in aortic aneurysms.", CIRCULATION, vol. 120, no. 11, 15 September 2009 (2009-09-15), pages 983-991, XP002603520, ISSN: 1524-4539 -& PILOP CHRISTIANE ET AL: "Correction: Proteomic analysis in aortic media of patients with Marfan syndrome reveals increased activity of calpain 2 in aortic aneurysms.", CIRCULATION, vol. 121, no. 6, E38, 2009, XP002607755, DOI: 10.1161/CIR.0b013e3181d41311 -& PILOP CHRISTIANE ET AL: "Data Supplement: Proteomic analysis in aortic media of patients with Marfan syndrome reveals increased activity of calpain 2 in aortic aneurysms.", CIRCULATION, vol. 120, no. 11, 15 September 2006 (2006-09-15), pages 1-7, XP002607756,
- ROGGERIO A ET AL: "Infectious agents, MMP-9 and PPAR alpha are increased in the adventitia and periadventital fat of aortic aneurysms", CIRCULATION, vol. 118, no. 12, September 2008 (2008-09) , page E410, XP008127732, & WORLD CONGRESS OF CARDIOLOGY; BUENOS AIRES, ARGENTINA; MAY 18 -21, 2008 ISSN: 0009-7322
- BELKIN ALEXEY M ET AL: "A novel phosphoglucomutase-related protein is concentrated in adherens junctions of muscle and nonmuscle cells", JOURNAL OF CELL SCIENCE, vol. 107, no. 1, 1994, pages 159-173, XP002603522, ISSN: 0021-9533
- ZHANG C ET AL: "Role of NonO-histone interaction in TNFalpha-suppressed Prolyl-4-hydroxylase alpha1", BIOCHIMICA ET BIOPHYSICA ACTA. MOLECULAR CELL RESEARCH, ELSEVIER SCIENCE PUBLISHERS, AMSTERDAM, NL LNKD- DOI:10.1016/J.BBAMCR.2008.03.011, vol. 1783, no. 8, 1 August 2008 (2008-08-01), pages 1517-1528, XP022731885, ISSN: 0167-4889 [retrieved on 2008-03-29]

## Description

The present invention concerns the detection of the presence of unstable atherosclerotic plaques.

Atherosclerosis is a complex evolutive disease in relation with major cardiovascular risk factors (hypertension, hyperlipidemia, diabetes...). It is the leading cause of death in the developed world, and is predicted to be the leading cause of death in the developing world within the first quarter of the next century. Indeed, in 2005, cardiovascular diseases were responsible for about 35% of all deaths that year (Heart Disease and Stroke Statistics - 2009 Update). For a few years, a light decrease in mortality due to coronary heart disease in the developed world has occurred. Nevertheless, this decrease has not occurred in the developing world, and an exponential increase in tobacco addiction and the adoption of a Western diet high in saturated fats likely predicts the continued increase in death and disability due to coronary heart disease in those countries. Accordingly, there is still a need for methods enabling detecting atherosclerosis in subjects, in particular detecting vulnerable plaques generally responsible for vascular events.

Several histopathological studies investigated plaque characteristics to better understand pathophysiology of atherosclerosis progression, leading to plaque vulnerability. However, the exact mechanisms underlying plaque destabilization, responsible of atherothrombosis, remain complex and not fully understood.

Vulnerable plaque is commonly defined as presenting a thinner fibrous cap and inflammatory infiltrate, with possible intra-plaque hemorrhage (Virmani et al. (2006) J. Am. Coll Cardiol 47:C13-18), while instability status of plaques is less consensual. Microscopic characteristics of carotid plaques revealed differences in symptomatic and asymptomatic patients following endarterectomy. Presence of plaque rupture, thin fibrous cap, cap foam cells, fibrin and hemorrhage intraplaques are the main critical differences (Virmani et al. (2006) J. Am. Coll Cardiol 47:C13-18). Cellular components as macrophage cells (Virmani et al. (2006) J. Am. Coll Cardiol 47:C13-18), leukocytes (Leclercq et al. (2007) J Leukoc Biol. 82(6):1420-9) and erythrocytes (Virmani et al. (2005) Arterioscler Thromb Vasc Biol 25:2054-2061) have been reported in atherosclerotic plaques. Thrombotic plaques are more common in symptomatic patients as compared to asymptomatic patients. Carotid atherosclerotic plaques following patient endarterectomy have been widely studied, leading to the definition of the two following types: fibrous stable plaque and vulnerable/unstable plaque more prone to rupture.

Previous authors reported the implication of markers of inflammation (Tedgui and Mallat (2006) Physiol Rev. 86(2):515-81), proteolytic activity (Sangiorgi et al. (2006) J Am Coll Cardiol 47:2201-2211; Mansilla et al. (2008) Arterioscler Thromb Vasc Biol. 28(10):1844-50; Sluijter et al. (2006) Stroke. 37(1):235-9), intra-plaque hemorrhage (Virmani et al. (2005) Arterioscler Thromb Vasc Biol. 25(10):2054-61; Leclercq et al. (2007) J Leukoc Biol. 82(6):1420-9) and thrombosis (Reininger et al. (2010) J Am Coll Cardiol. 55(11):1147-58). In parallel, several protein analyses were proposed to better explain plaque progression leading to rupture of carotid plaques (Park et al. (2006) Circulation 114:886-893; Lepedda et al. (2009) Atherosclerosis 203:112-118; Donners et al. (2005) J Pathol 206:39-45) or of cultured atherosclerotic plaques (Duran et al. (2003) Proteomics 3:973-978). More specifically, Lepedda *et al.* (2009) tended to correlate histological plaque features to protein expression in stable and unstable human carotid atherosclerotic plaques. A series of markers such as PAPP-A (Sangiorgi et al. (2006) J Am Coll Cardiol 47:2201-2211), MMP-9 (Sluijter et al. (2006) Stroke 37:235-239), soluble Tweak (Blanco-Colio et al. (2007) Arterioscler Thromb Vasc Biol 27(4):916-22), heat shock protein-27 and 70, SOD2 protein, ferritin light subunit, fibrinogen fragment D (Lepedda et al. (2009)), were identified as differentially expressed in carotid plaque depending on the plaque characteristics.

Some of these biochemical markers were used to identify vulnerable atherosclerotic plaques. For example, the international application WO 2007/002677 discloses circulating proteins that were identified as differentially expressed in atherosclerotic patients and their use to classify notably a sample as an atherosclerotic cardiovascular disease sample or as a healthy sample. The European application EP 2 019 318 also discloses methods for predicting the risk of a test subject developing a cardiovascular event comprising typing atherosclerotic plaques by measuring the amount of different biochemical markers in samples of atherosclerotic plaques from a patient.

According to these previous proteomic studies, most of the extracted carotid plaque proteins were of plasma origin (Lepedda *et al.* (2009)), making a larger proteome dynamic range of the atherosclerotic plaque. Thus biomarker discovery using proteomic analysis is limited, especially for the detection of low abundant proteins, as cytokines/chemokines and growth factors hindered by higher abundant species.

The present invention arises from the unexpected finding by the inventors that a specific treatment of protein extract of tissue samples, obtained from patients displaying unstable atherosclerotic plaques, leading to the enrichment of the samples in low abundant proteins, enabled identifying new biochemical markers of the unstable atherosclerotic plaque.

Each biological fluid pre-treatment technique presents its drawback, limiting the detection of all proteome components (Zolotarjova et al. (2005) Proteomics 5:3304-3313). Immunological co-depletion with uncontrolled loss of potential molecule of interest is one of the most important concerns of this pre-analytical process. More recently, reduction of the protein dynamic range based on ligand libraries has been reported to be effective in low abundant protein enrichment in serum (Sennels et al. (2007) J Proteome Res 6:4055-4062), plasmas (Sihlbom et al. (2008) J Proteome Res 7:4191-4198), platelets (Guerrier et al. (2007) J Proteome Res 6:4290-4303) and erythrocytes (Roux-Dalvai (2008) Mol Cell Proteomics 7:2254-2269). The ligand library bead technique has proven to effectively identify larger spots/peak numbers and several new species in different biological support. The hypothesis of the inventors was that the wide protein dynamic range of atherosclerotic carotid plaques (Lepedda *et al.* (2009)) might mask potential biomarkers tagging plaque vulnerability; and that peptide library bead technology applied on vulnerable atherosclerotic plaques might amplify minor species. Nevertheless, one limitation of this approach was the low compatibility of peptide library bead technology with tissue extraction methods especially when using concentrated detergents, thus reducing low abundant proteins capture on beads when compared to others human fluids.

The article Pilop et al. (2009) Circulation 120:983-991 shows that MFAP-4 and calponin-1 are present in higher amounts in aortic media of patients suffering from Marfan syndrome with aortic aneurysm.

The German application DE 10 2006 048 249 discloses the use of markers including MFAP-4 and calponin-1 for diagnosing liver inflammation.

However, the present inventors unexpectedly demonstrated that a particular extraction protocol for soluble proteins (herein after also named "soluble-oriented protein extraction") coupled to ligand library bead treatment on protein diversity provided from carotid atherosclerotic plaques allowed low abundant protein amplification from complex vascular tissues extracts. This specific protocol led to the identification of novel biochemical markers of unstable atherosclerotic plaques enabling the diagnosis or prognosis of vascular or metabolic diseases in an individual.

### Detailed description of the invention

Accordingly, the application describes an *in vitro* method for identifying markers of the presence of an unstable atherosclerotic plaque in an individual, comprising the steps of:
- preparing a protein extract from a biological sample representative of a stable atherosclerotic plaque,
- preparing a protein extract from a biological sample representative of an unstable atherosclerotic plaque,
- decreasing the proportion of the proteins which are the most abundant in the protein extracts in order to obtain depleted protein extracts enriched in scarce proteins,
- separating the proteins present in the depleted protein extracts,
- identifying the markers of the presence of an unstable atherosclerotic plaque, wherein said markers are the proteins that are differently expressed in the biological sample representative of the unstable atherosclerotic plaque compared to the biological sample representative of the stable atherosclerotic plaque.

The invention is defines by the appended claims.

### Atherosclerotic plaque

In the context of the invention, an "atherosclerotic plaque" or "atheroma plaque" refers to a lesion of vessel walls. Preferably, an "atherosclerotic plaque" according to the invention comprises a lipid core and a fibrous cap, said cap being constituted by smooth muscle cells, collagens and an extracellular matrix, and isolating the lipid core from the arterial lumen. As known from the person skilled in the art, an atherosclerotic plaque is generally due to the accumulation and swelling in artery walls where the swelling is caused by for example macrophages, cell debris, lipids such as cholesterol and fatty acids, calcium and fibrous connective tissue. Thus, the process of atheroma development within an individual is called atherogenesis and the overall result of the disease process is termed atherosclerosis. Atherosclerotic plaques may be found in particular in the aorta, in the carotid or in the coronary artery, and also in peripheral vessels. Atherosclerotic plaques may be further divided into "stable" atherosclerotic plaques and "unstable" or "vulnerable" atherosclerotic plaques.

As used herein, the terms "unstable atherosclerotic plaque", "vulnerable atherosclerotic plaque", "complicated atherosclerotic plaque", "complicated plaque" and "CP" are used indifferently and refer to an atherosclerotic plaque which is prone to rupture. Unstable atherosclerotic plaques may in particular be characterized by a thin fibrous cap (<100 µm thick) infiltrated by monocyte/macrophage and sometimes T-cell and a lipid core accounting for 40% of the plaque's total volume. In the context of the invention, unstable atherosclerotic plaques (or vulnerable plaques) encompass thin cap fibroatheroma (TCFA), pathologic intimal thickening, thick cap fibroatheroma and calcified plaque with luminal calcified nodules. In the context of the present invention, when referring to atherosclerotic plaque, the terms "vulnerable", "unstable", "dangerous" and "high-risk" are synonymous and interchangeable (Naghavi et al. (2003) Circulation 108:1664-1672; Naghavi et al. (2003) Circulation 108:1772-1778).

As used herein, the terms "stable atherosclerotic plaque", "non complicated atherosclerotic plaque", "non complicated plaque" and "NCP" are used indifferently and refer to an atherosclerotic plaque which is not prone to rupture and/or which does not show important inflammation and important lipid accumulation.

In particular, an atherosclerotic plaque may contain an area which corresponds to an unstable atherosclerotic plaque and an area which corresponds to a stable atherosclerotic plaque.

As used herein, the expression "marker of the presence of an unstable atherosclerotic plaque" or "marker of the vulnerability of an atherosclerotic plaque" refers to a compound, the presence, absence and/or level of which is characteristic of an unstable atherosclerotic plaque. Preferably, the marker of the presence of an unstable atherosclerotic plaque is a compound which is differently present or absent, or the level of which is different, in the region of an unstable atherosclerotic plaque compared to the region of a stable atherosclerotic plaque.

Advantageously, the marker of the presence of an unstable atherosclerotic plaque described herein is also differently present or absent, or its level is different, in a liquid biological sample, in particular a plasma sample from a subject displaying unstable atherosclerotic plaques compared to a subject which does not display unstable atherosclerotic plaques.

Preferably, the marker is a biological marker, more preferably a biochemical marker. The marker may be in particular a protein, a protein fragment, a peptide, a peptide fragment, a polypeptide, a lipid, an oligosaccharide, a metabolite or a proteic complex.

In the context of the present invention, an "individual" denotes a human or non-human mammal, such as a rodent (rat, mouse, rabbit), a primate (chimpanzee), a feline (cat), a canine (dog). Preferably, the individual is human.

### Biological sample

As used herein, the term "biological sample" refers to a sample of tissue or fluid isolated from an individual. In particular a biological sample may be a liquid biological sample or a tissue sample. Examples of biological samples include, but are not limited to, pieces of organs or of tissues such as kidney, liver, heart, lung, and the like, arteries veins and the like, blood and components thereof such as plasma, platelets, serum, subpopulations of blood cells and the like, tears, urine and saliva. Preferably, when the biological sample is a liquid biological sample, it is selected from the group consisting of blood sample, serum, plasma and urine. More preferably, when the biological sample is a liquid biological sample, it is a plasma sample. Still preferably, when the biological sample is a tissue sample, it is an artery sample.

As used herein, the expression "biological sample representative of stable atherosclerotic plaque" refers to a biological sample which comprises essentially the same components as a stable atherosclerotic plaque. In particular, a biological sample representative of stable atherosclerotic plaque comprises the same proportions of biochemical components as a stable atherosclerotic plaque. Preferably, a biological sample representative of stable atherosclerotic plaque according to the invention is a tissue sample of a stable atherosclerotic plaque.

As used herein, the expression "biological sample representative of unstable atherosclerotic plaque" refers to a biological sample which comprises essentially the same components as an unstable atherosclerotic plaque. In particular, a biological sample representative of unstable atherosclerotic plaque comprises the same proportions of biochemical components as an unstable atherosclerotic plaque. Preferably, a biological sample representative of unstable atherosclerotic plaque according to the invention is a tissue sample of an unstable atherosclerotic plaque.

Preferably, the tissue sample of stable atherosclerotic plaque and the tissue sample of unstable atherosclerotic plaque are taken from an artery.

### Preparation of the protein extract

As used herein, the term "protein extract" or "total protein extract" refers to a mixture comprising at least one protein obtained from a biological sample. Preferably, the protein extract of the invention is obtained from a tissue sample. More preferably, the protein extract of the invention is obtained from an artery sample. Most preferably, the protein extract of the invention is obtained from an atherosclerotic plaque.

Techniques to obtain protein extracts from a biological sample, in particular from a tissue sample, are well-known from the one skilled in the art. Examples of such techniques include liquid nitrogen freezing, fragmentation, disruption in hypotonic buffer or in buffer containing detergents using an homogenizer, sonication, and combinations of these techniques.

Preferably, the protein extract is obtained in a non-selective way, *i.e.* some proteins which are initially present in the biological sample are not selectively obtained in the protein extract compared to other proteins which are initially present in the biological sample. Accordingly, the protein extract as described herein comprises preferably the same proportion of each protein as the biological sample.

Preferably, the step of preparing the protein extract enables obtaining a protein extract which may be directly treated to decrease the proportion of the proteins which are the most abundant.

Typically, the preparation of the protein extract as described herein comprises (i) homogenizing the biological sample with an homogenizer, preferably on ice, adding a buffer, in particular HEPES buffer, preferably cold HEPES buffer, more preferably HEPES buffer containing anti-proteases and/or anti-phosphatases, whereby an homogenate is obtained; (ii) incubating the homogenates, preferably at 4°C, preferably under agitation; (iii) and centrifuging the homogenates, preferably at 4°C, for example at 15,000 - 20,000 g, preferably at 18,000 g.

### Preparation of the depleted protein extract

As described herein, the expression "proteins which are the most abundant in the protein extract" refers to the proteins the level of which is the highest in the total or essentially total protein extract as defined above.

As used herein, the "level" of a protein corresponds to the concentration, the amount or the activity of the protein. Preferably, the level of a protein refers to the amount of the protein, more particularly to the molar or the mass amount of the protein.

It is within the general knowledge of the skilled person to determine the proteins which are the most abundant in the protein extract. The skilled person may in particular refer to Lepedda et al. (2009) Atherosclerosis 203:112-118 to determine these proteins. Preferably, the proteins which are the most abundant in the protein extract correspond to the ten most abundant proteins in the total or essentially total protein extract. More preferably, the proteins which are the most abundant in the protein extract correspond to the nine, still preferably the eight, still preferably the seven, still preferably the six, still preferably the five, still preferably the four, still preferably the three, still preferably the two most abundant protein(s) in the total or essentially total protein extract. Still preferably, the proteins which are the most abundant in the protein extract correspond to the protein, the level of which is the highest in the total or essentially total protein extract.

In the context of the invention, the expression "scarce proteins" refers to the proteins of the protein extract, the level of which is the lowest in the total or essentially total protein extract as defined above.

It is within the general knowledge of the skilled person to determine the scarce proteins in the protein extract. The skilled person may in particular refer to Lepedda et al. (2009) Atherosclerosis 203:112-118 to determine these proteins

Preferably, the scarce proteins in the protein extract correspond to the set of proteins, the level of which represents less than 15% of the total or essentially total level of protein in the total or essentially total protein extract. More preferably, the scarce proteins in the protein extract correspond to the set of proteins, the level of which represents less than 10%, still preferably less than 5%, still preferably less than 1% of the total or essentially total level of protein in the total or essentially total protein extract.

As described herein, the expression "decreasing the proportion of the proteins which are the most abundant in the protein extract" or "decrease of the proportion of the proteins which are the most abundant in the protein extract" refers to the specific partial removal, from the total or essentially total protein extract as defined above, of the proteins which are the most abundant in the protein extract as defined above, whereas the other proteins of the protein extract, in particular the scarce proteins as defined above, are retained. As described herein, the decrease of the proportion of the proteins which are the most abundant in the protein extract leads to obtaining a "depleted protein extract enriched in scarce proteins".

As used herein, the expression "depleted protein extract enriched in scarce proteins" or "depleted protein extract" refers to protein extracts obtained from a total or essentially total protein extract as defined above wherein the proportion of the proteins which are the most abundant in the total protein extract has been decreased while the scarce proteins have been retained. Accordingly, a depleted protein extract enriched in scarce proteins as described herein is a protein extract obtained from a total or essentially total protein extract as defined above, wherein the scarce proteins of the total or essentially total protein extract are present in a higher proportion than in the total or essentially total protein extract. Preferably, the proteins which are the most abundant in the protein extract as defined above are present in a lower proportion in the depleted protein extract compared to the total or essentially total protein extract as defined above.

As used herein, the "proportion" of a protein in a protein extract corresponds to the molar or the mass amount of the protein compared to the total molar or mass amount of proteins in the protein extract.

Preferably, the proteins which are the most abundant in the protein extract are only partially removed from the total or essentially protein extract. Accordingly, a portion of the proteins which are the most abundant in the protein extract may still be present in the depleted protein extract as defined above.

Techniques for removing some proteins from a protein extract are well known from the skilled person and include for example the use of affinity purification, such as the use of ligands specific of the proteins to be removed or retained, in particular of individual or combined antibodies; the use of sequential or simultaneous immunoaffinity depletion (as described in Pieper et al. (2003) Proteomics 3:1345-1364); the use of chromatography such as lectin columns (as described in Hirabayashi et al. (2004) Glycoconj J 21:35-40) and metal chelator or titanium oxide resins (as described in Larsen et al. (2005) Mol Cell Proteomics 4:873-886), thin-layer liquid chromatography, gas chromatography, liquid chromatography, in particular adsorption chromatography, partition chromatography, ion exchange chromatography, gel permeation chromatography and affinity chromatography, and supercritical chromatography; the use of electrophoresis such as SDS-PAGE electrophoresis, 2D electrophoresis, 3D electrophoresis, immunoelectrophoresis, pulse-field gel electrophoresis, capillary electrophoresis, narrow range immobilized pH gradient strips (zoom gels), Off-gel electrophoresis, solution isoelectric focusing (as described in Zuo et al. (2005) Curr Protoc Protein Sci Chapter 22:Unit22.6); the use of gradients and the use of centrifugation. Preferably, the decrease of the proportion of the proteins which are the most abundant in the protein extract is carried out using ligands that preferentially bind to the proteins which are the most abundant in the protein extract.

As used herein, the expression "ligands that preferentially bind to the proteins which are the most abundant in the protein extract" refers to ligands which bind more to the proteins which are the most abundant in the protein extract as defined above than to the other proteins of the protein extracts. In particular, these ligands bind more to the proteins which are the most abundant in the protein extract as defined above than the scarce proteins of the protein extract as defined above.

As also described herein, the decrease of the proportion of the proteins which are the most abundant in the protein extract may be carried out using a combination of ligands, wherein each ligand binds preferentially to a specific protein of the protein extract. Preferably, the combinations of ligands, wherein each ligand binds preferentially to a specific protein of the protein extract, are combinatorial ligands libraries. Still preferably, said ligands are bound to a chromatographic support.

As used herein, the expression "combinatorial ligands libraries" refers to highly diverse libraries of peptides, wherein each peptide theoretically binds to a unique protein sequence. Typically, because the ligands capacity limits binding capacity, proteins which are the most abundant in the protein extract quickly saturate their ligands and excess protein is washed out. In contrast, scarce proteins from the protein extract are concentrated on their specific ligands. Preferably, the peptides of the combinatorial ligands libraries according to the invention consist of less than 20 amino acids, more preferably of less than 15 amino acids, still preferably of less than 10 amino acids, still preferably of less than 9, 8, 7 or 6 amino acids. Most preferably, the peptides of the combinatorial ligands libraries according to the invention are hexapeptides. Preferably, the peptides of the combinatorial ligands peptides are bound to a chromatographic support.

Examples of chromatographic supports are well known from the one skilled in the art and include for example resins and beads. Preferably, the peptides of the combinatorial ligands peptides are bound to beads. Typically, the combinatorial ligands peptides used in the context of the invention are ProteoMiner™ beads commercialized by Bio-Rad and modified combinatorial ligand libraries such as the combinatorial ligand libraries described in Roux-Dalvai et al. (2008) Mol. Cell. Proteomics 7:2254-2269.

As described herin, when the decrease of the proportion of the proteins which are the most abundant in the protein extract is carried out using a combination of ligands, wherein each ligand binds preferentially to a specific protein of the protein extract, the proteins which are bound to the ligands may then be eluted and recovered. Techniques for eluting proteins which are bound to ligands are well known from the skilled person and include the use of elution buffers, in particular denaturing elution buffers or acid elution buffers, such as buffers containing urea or a detergent such as an ionic (anionic or cationic) detergent or a non-ionic (or zwitterionic) detergent. A non-limiting example of anionic detergent is sodium dodecyl sulfate (SDS), a non-limiting example of cationic detergent is cetyltrimethylammonium bromide (CTAB), and a non-limiting example of zwitterionic detergent is 3-[(3-Cholamidopropyl)dimethylammonio]-1-propanesulfonate (CHAPS). Typically, the elution of the proteins which are bound to the ligands is carried out using an elution buffer at a pH of 3 to 5, preferably at pH 3.4, containing urea, optionally with a detergent such as CHAPS.

Typically, the preparation of the depleted protein extract as described herein comprises:
(i) optionally equilibrating a combination of ligands (abbreviated herein ligands), wherein each ligand binds preferentially to a specific protein of the protein extract, in particular combinatorial ligands libraries, preferably combinatorial ligands libraries bound to beads, more preferably ProteoMiner™ beads, in a buffer, preferably in a saline buffer, for example in a phosphate buffer saline, whereby equilibrated ligands are obtained;
(ii) incubating the protein extract with the optionally equilibrated ligands, preferably under gentle agitation, preferably at a temperature of 1°C to 20°C, more preferably at 4°C, for example during 3 hours, into an appropriate container such as a spin-column;
(iii) separating the proteins which did not bind to the ligands from the proteins bound to the ligands, preferably by centrifugation, for example at 800 - 2000 g, preferably at 1020 g;
(iv) optionally, incubating the proteins which did not bind to the ligands with other optionally equilibrated combination of ligands such as modified combinatorial ligand libraries, wherein each ligand binds preferentially to a specific protein of the protein extract, preferably under gentle agitation, preferably at a temperature of 1°C to 20°C, more preferably at 4°C, for example during 3 hours, into an appropriate container such as a spin-column; and separating the proteins which did not bind to the second ligands from the proteins bound to the second ligands, preferably by centrifugation, for example at 800 - 2000 g, preferably at 1020 g;
(v) recovering the proteins bound to the first and optionally to the second ligands, optionally after washing, for example with a phosphate buffer saline, preferably at a neutral pH such as at pH 7.4, wherein the recovery is preferably carried by elution, preferably using an acid elution buffer, typically an elution buffer at pH 3.4, preferably comprising a denaturing agent, such as urea, further preferably comprising a detergent, such as CHAPS, typically an elution buffer pH 3.4 containing 8 M urea, 2% CHAPS and 50 mM acetic acid.

### Separation and identification of the markers

As used herein, the term "separation" or "separating" refers to the differentiation of the proteins of the protein extract the one from the others according to some of their properties, such as their molecular mass, their charge, their isoelectric point.

Examples of techniques of separation are well known from the skilled person and include in particular electrophoresis, such as one dimensional gel (1 D) electrophoresis and two-dimensional (2D) gel electrophoresis; chromatography, such as column chromatography including ion-exchange chromatography, gel-filtration chromatography and affinity chromatography, high pressure liquid chromatography (HPLC) and multidimensional liquid chromatography.

Preferably, the step of separating the proteins as described herein is carried out by electrophoresis, in particular by 2D gel electrophoresis.

Typically, when the proteins are separated by 2D gel electrophoresis, the gels are stained in order to visualize the proteins, for example using Sypro Ruby^{®} commercialized by Bio-Rad, and are preferentially scanned and analyzed by computer in order to detect each spot of proteins on each gel. Suitable software's to detect spots of proteins are well known from the skilled person and include for example Progenesis SameSpots^{®} commercialized by Nonlinear USA Inc. Typically after protein spot detection, new spots are created to determine a mean background value, from which a value corresponding to the mean background value plus 2 standard deviations is calculated for each gel. A protein spot is for example identified as such when the mean value obtained for that spot is higher than the mean background value plus 2 standard deviations. The total number of spots for each gel is then typically calculated and the presence or absence of protein spots in each gel is determined.

The present inventors have demonstrated that the comparison of the separated proteins present in the depleted protein extract from the biological sample of a stable atherosclerotic plaque as defined above and the separated proteins present in the depleted protein extract from the biological sample of an unstable atherosclerotic plaque as defined above, enables identifying proteins which are differentially expressed in the biological sample representative of the unstable atherosclerotic plaque compared to the biological sample representative of the stable atherosclerotic plaque. These proteins are markers of the presence of an unstable atherosclerotic plaque.

As used herein, the expression "proteins which are differentially expressed in the biological sample representative of the unstable atherosclerotic plaque compared to the biological sample representative of the stable atherosclerotic plaque" refers to the proteins the level of which, or the proportion of which, is different in the total, essentially total or depleted protein extract from the biological sample representative of the unstable atherosclerotic plaque compared to the total, essentially total or depleted protein extract from the biological sample representative of the stable atherosclerotic plaque. In particular, the proteins which are differentially expressed in the biological sample representative of the unstable atherosclerotic plaque compared to the biological sample representative of the stable atherosclerotic plaque may display an increased level in the total, essentially total or depleted protein extract from the biological sample representative of the unstable atherosclerotic plaque compared to the total, essentially total or depleted protein extract from the biological sample representative of the stable atherosclerotic plaque. On the contrary, the proteins which are differentially expressed in the biological sample representative of the unstable atherosclerotic plaque compared to the biological sample representative of the stable atherosclerotic plaque may display a decreased level in the total, essentially total or depleted protein extract from the biological sample representative of the unstable atherosclerotic plaque compared to the total, essentially total or depleted protein extract from the biological sample representative of the stable atherosclerotic plaque.

Preferably the step of identifying the markers of the presence of an unstable atherosclerosis plaque is carried out by a technique selected from the group consisting of Western and dot Blot, mass spectrometry and immunoassays.

The above described method enabled the inventors to identify new biochemical markers which are characteristic of the presence of an unstable atherosclerotic plaque.

The application also describes a method, preferably an *in vitro* method, for determining the presence of an unstable atherosclerotic plaque in an individual, comprising the steps of:
a1) measuring the presence and/or level of at least one biochemical marker in a biological sample obtained from said individual, said biochemical marker being selected from the group consisting of:
   - calponin-1,
   - alpha B crystallin,
   - phosphatidylethanolamine-binding protein 1,
   - aciculin,
   - mature cathepsin K,
   - osteoglycin,
   - peroxiredoxin-2,
   - dihydropyrimidinase-like 3,
   - serum amyloid P component,
   - serpin B9,
   - serpin B9-granzyme B complex,
   - pro-cathepsin D,
   - thrombospondin-2,
   - thrombospondin-1,
   - pro-collagen C-endopeptidase enhancer-1,
   - microfibril-associated protein-4,
   - DJ-1 oncogene,
   - tissue inhibitor of metalloproteinase 1,
   - heat shock protein 27,
   - IL-12,
   - vascular endothelial growth factor,
   - IL-8,
   - IL-1ra,
   - granulocyte colony-stimulating factor,
   - growth-regulated alpha protein,
   - IL-13,
   - leukemia inhibitory factor,
   - IL-1 alpha,
   - IL-1 beta,
   - interferon alpha-2, and
   - stromal cell derived factor 1 alpha;
b1) based on the result of the measurement in step a1), determining the presence of an unstable atherosclerotic plaque in the individual.

Preferably, said at least one biochemical marker is selected from the group consisting of: calponin-1, alpha B crystallin, phosphatidylethanolamine-binding protein 1, aciculin, mature cathepsin K, dihydropyrimidinase-like 3, serum amyloid P component, serpin B9-granzyme B complex, pro-cathepsin D, thrombospondin-1, pro-collagen C-endopeptidase enhancer-1, IL-12, IL-1ra, granulocyte colony-stimulating factor, leukemia inhibitory factor, IL-1 alpha, IL-1 beta, interferon alpha-2, stromal cell derived factor 1 alpha, microfibril-associated protein-4, DJ-1 oncogene and tissue inhibitor of metalloproteinase 1. More preferably, said at least one biochemical marker is selected from the group consisting of: calponin-1, phosphatidylethanolamine-binding protein 1, aciculin, pro-collagen C-endopeptidase enhancer-1, IL-12, IL-1ra, granulocyte colony-stimulating factor, leukemia inhibitory factor, IL-1 alpha, IL-1 beta, interferon alpha-2, stromal cell derived factor 1 alpha, microfibril-associated protein-4, DJ-1 oncogene, tissue inhibitor of metalloproteinase 1, osteoglycin, peroxiredoxin-2, thrombospondin-2, heat shock protein 27, growth-regulated alpha protein, IL-8, IL-13 and vascular endothelial growth factor. Most preferably, said at least one biochemical marker is selected from the group consisting of: calponin-1, IL-8, DJ-1 oncogene, vascular endothelial growth factor and pro-collagen C-endopeptidase enhancer-1.

The present invention concerns a method for determining the presence of an unstable atherosclerotic plaque in an individual, comprising the steps of:
a1) measuring the presence and/or level of at least one biochemical marker in a biological sample obtained from said individual, said biochemical marker being microfibril-associated protein-4;
b1) based on the result of the measurement in step a1), determining the presence of an unstable atherosclerotic plaque in the individual.

The invention is further defined by the set of claims.

As disclosed herein, in the above defined method, step a1) may comprise or consist in measuring, in said biological sample, the presence and/or level of one of the above defined biochemical biomarkers, more preferably two of the above defined biochemical markers, still preferably three of the above defined biochemical markers, still preferably four of the above defined biochemical markers, still preferably five of the above defined biochemical markers, still preferably six of the above defined biochemical markers, still preferably seven of the above defined biochemical markers, still preferably eight of the above defined biochemical biomarkers, still preferably nine of the above defined biochemical biomarkers, and still preferably ten of the above defined biochemical biomarkers.

As disclosed herein, in the above defined method, step a1) may comprise measuring the presence and/or level of :
(i) calponin-1 and
(ii) at least one biochemical marker in a biological sample obtained from said individual, said biochemical marker being selected from the group consisting of:
   - alpha B crystallin,
   - phosphatidylethanolamine-binding protein 1,
   - aciculin,
   - mature cathepsin K,
   - osteoglycin,
   - peroxiredoxin-2,
   - dihydropyrimidinase-like 3,
   - serum amyloid P component,
   - serpin B9,
   - serpin B9 -granzyme B complex,
   - pro-cathepsin D,
   - thrombospondin-2,
   - thrombospondin-1,
   - pro-collagen C-endopeptidase enhancer-1,
   - DJ-1 oncogene,
   - tissue inhibitor of metalloproteinase 1,
   - microfibril-associated protein-4,
   - heat shock protein 27,
   - IL-12,
   - vascular endothelial growth factor,
   - IL-8,
   - IL-1ra,
   - granulocyte colony-stimulating factor,
   - growth-regulated alpha protein,
   - IL-13,
   - leukemia inhibitory factor,
   - IL-1 alpha,
   - IL-1 beta,
   - interferon alpha-2, and
   - stromal cell derived factor 1 alpha.

Preferably, said at least one biochemical marker (ii) is selected from the group consisting of: phosphatidylethanolamine-binding protein 1, aciculin, pro-collagen C-endopeptidase enhancer-1, IL-12, IL-1ra, granulocyte colony-stimulating factor, leukemia inhibitory factor, IL-1 alpha, IL-1 beta, interferon alpha-2, stromal cell derived factor 1 alpha, microfibril-associated protein-4, DJ-1 oncogene, tissue inhibitor of metalloproteinase 1, osteoglycin, peroxiredoxin-2, thrombospondin-2, heat shock protein 27, growth-regulated alpha protein, IL-8, IL-13 and vascular endothelial growth factor. More preferably, said at least one biochemical marker (ii) is selected from the group consisting of: phosphatidylethanolamine-binding protein 1, aciculin, pro-collagen C-endopeptidase enhancer-1, IL-12, stromal cell derived factor 1 alpha, microfibril-associated protein-4, DJ-1 oncogene, tissue inhibitor of metalloproteinase 1, osteoglycin, heat shock protein 27, growth-regulated alpha protein, IL-8 and vascular endothelial growth factor.

Preferably, in the method disclosed herein, step a1) comprises measuring the presence and/or level of one of the following combinations of biochemical markers :
- calponin-1 and IL-8,
- calponin-1 and aciculin,
- calponin-1 and stromal cell derived factor 1 alpha,
- calponin-1 and DJ-1 oncogene,
- calponin-1 and vascular endothelial growth factor,
- calponin-1 and tissue inhibitor of metalloproteinase,
- calponin-1 and heat shock protein 27,
- calponin-1 and growth-regulated alpha protein,
- calponin-1 and IL-12,
- calponin-1 and microfibril-associated protein-4,
- calponin-1 and phosphatidylethanolamine-binding protein 1,
- calponin-1 and pro-collagen C-endopeptidase enhancer-1,
- calponin-1 and osteoglycin.

As disclosed herein, in the above defined method, step a1) may comprise or consist in measuring, in said biological sample, the presence and/or the level of one of the following combinations of biochemical markers:
- IL-8 and granulocyte colony-stimulating factor,
- tissue inhibitor of metalloproteinase 1 and granulocyte colony-stimulating factor,
- tissue inhibitor of metalloproteinase 1 and IL-13,
- IL-13 and granulocyte colony-stimulating factor,
- pro-collagen C-endopeptidase enhancer-1 and granulocyte colony-stimulating factor,
- IL-1 beta and IL-13,
- IL-12 and granulocyte colony-stimulating factor,
- vascular endothelial growth factor and granulocyte colony-stimulating factor,
- DJ-1 oncogene and granulocyte colony-stimulating factor,
- pro-collagen C-endopeptidase enhancer-1, IL-8 and granulocyte colony-stimulating factor,
- IL-8, IL-13 and granulocyte colony-stimulating factor,
- DJ-1 oncogene, IL-8 and granulocyte colony-stimulating factor,
- tissue inhibitor of metalloproteinase 1, IL-13 and granulocyte colony-stimulating factor,
- tissue inhibitor of metalloproteinase 1, IL-8 and granulocyte colony-stimulating factor,
- growth-regulated alpha protein, IL-8 and granulocyte colony-stimulating factor,
- IL-8, IL-12 and granulocyte colony-stimulating factor, and
- tissue inhibitor of metalloproteinase 1, IL-13 and growth-regulated alpha protein.

As disclosed herein, step a1) may comprise measuring the presence and/or level of:
(i) calponin-1,
(ii) a second biochemical marker selected from the group consisting of:
   - alpha B crystallin,
   - phosphatidylethanolamine-binding protein 1,
   - aciculin,
   - mature cathepsin K,
   - osteoglycin,
   - peroxiredoxin-2,
   - dihydropyrimidinase-like 3,
   - serum amyloid P component,
   - serpin B9,
   - serpin B9 - granzyme B complex,
   - pro-cathepsin D,
   - thrombospondin-2,
   - thrombospondin-1,
   - pro-collagen C-endopeptidase enhancer-1,
   - DJ-1 oncogene,
   - tissue inhibitor of metalloproteinase 1,
   - microfibril-associated protein-4,
   - heat shock protein 27,
   - IL-12,
   - vascular endothelial growth factor,
   - IL-8,
   - IL-1ra,
   - granulocyte colony-stimulating factor,
   - growth-regulated alpha protein,
   - IL-13,
   - leukemia inhibitory factor,
   - IL-1 alpha,
   - IL-1 beta,
   - interferon alpha-2, and
   - stromal cell derived factor 1 alpha; and
(iii) at least one other biochemical marker selected from the group consisting of:
   - alpha B crystallin,
   - phosphatidylethanolamine-binding protein 1,
   - aciculin,
   - mature cathepsin K,
   - osteoglycin,
   - peroxiredoxin-2,
   - dihydropyrimidinase-like 3,
   - serum amyloid P component,
   - serpin B9,
   - serpin B9 - granzyme B complex,
   - pro-cathepsin D,
   - thrombospondin-2,
   - thrombospondin-1,
   - pro-collagen C-endopeptidase enhancer-1,
   - DJ-1 oncogene,
   - tissue inhibitor of metalloproteinase 1,
   - microfibril-associated protein-4,
   - heat shock protein 27,
   - IL-12,
   - vascular endothelial growth factor,
   - IL-8,
   - IL-1ra,
   - granulocyte colony-stimulating factor,
   - growth-regulated alpha protein,
   - IL-13,
   - leukemia inhibitory factor,
   - IL-1 alpha,
   - IL-1 beta,
   - interferon alpha-2, and
   - stromal cell derived factor 1 alpha;
      said at least one other biochemical marker (iii) being different from said second biochemical marker (ii).

Preferably, said at least one biochemical marker (ii) is selected from the group consisting of: phosphatidylethanolamine-binding protein 1, aciculin, pro-collagen C-endopeptidase enhancer-1, IL-12, IL-1ra, granulocyte colony-stimulating factor, leukemia inhibitory factor, IL-1 alpha, IL-1 beta, interferon alpha-2, stromal cell derived factor 1 alpha, microfibril-associated protein-4, DJ-1 oncogene, tissue inhibitor of metalloproteinase 1, osteoglycin, peroxiredoxin-2, thrombospondin-2, heat shock protein 27, growth-regulated alpha protein, IL-8, IL-13 and vascular endothelial growth factor. More preferably, said at least one biochemical marker (ii) is selected from the group consisting of IL-8, aciculin, phosphatidylethanolamine-binding protein 1, pro-collagen C-endopeptidase enhancer-1, heat shock protein 27, stromal cell derived factor 1 alpha and vascular endothelial growth factor. Still preferably, said at least one biochemical marker (ii) is selected from IL-8 and aciculin. Still preferably, said at least one biochemical marker (ii) is IL-8.

Preferably, said at least one other biochemical marker (iii) is selected from the group consisting of: phosphatidylethanolamine-binding protein 1, aciculin, pro-collagen C-endopeptidase enhancer-1, IL-12, IL-1ra, granulocyte colony-stimulating factor, leukemia inhibitory factor, IL-1 alpha, IL-1 beta, interferon alpha-2, stromal cell derived factor 1 alpha, microfibril-associated protein-4, DJ-1 oncogene, tissue inhibitor of metalloproteinase 1, osteoglycin, peroxiredoxin-2, thrombospondin-2, heat shock protein 27, growth-regulated alpha protein, IL-8, IL-13 and vascular endothelial growth factor.

Preferably, as disclosed herein, step a1) comprises measuring the presence and/or level of one of the following combinations of biochemical markers :
- calponin-1, IL-8 and microfibril-associated protein-4,
- calponin-1, IL-8 and heat shock protein 27,
- calponin-1, IL-8 and thrombospondin-2,
- calponin-1, IL-8 and pro-collagen C-endopeptidase enhancer-1,
- calponin-1, IL-8 and tissue inhibitor of metalloproteinase 1,
- calponin-1, IL-8 and DJ-1 oncogene,
- calponin-1, IL-8 and growth-regulated alpha protein,
- calponin-1, IL-8 and IL-1 beta,
- calponin-1, IL-8 and IL-13,
- calponin-1, IL-8 and vascular endothelial growth factor,
- calponin-1, IL-8 and IL-12,
- calponin-1, IL-8 and IL-1 alpha,
- calponin-1, IL-8 and leukemia inhibitory factor,
- calponin-1, IL-8 and aciculin,
- calponin-1, IL-8 and interferon alpha-2,
- calponin-1, aciculin and vascular endothelial growth factor,
- calponin-1, IL-8 and IL-1 ra,
- calponin-1, IL-8 and osteoglycin,
- calponin-1, IL-8 and stromal cell derived factor 1 alpha,
- calponin-1, aciculin and DJ-1 oncogene,
- calponin-1, IL-8 and phosphatidylethanolamine-binding protein 1,
- calponin-1, IL-8 and granulocyte colony-stimulating factor,
- calponin-1, IL-8 and peroxiredoxin-2,
- calponin-1, aciculin and phosphatidylethanolamine-binding protein 1,
- calponin-1, phosphatidylethanolamine-binding protein 1 and IL-1ra,
- calponin-1, vascular endothelial growth factor and phosphatidylethanolamine-binding protein 1,
- calponin-1, vascular endothelial growth factor and pro-collagen C-endopeptidase enhancer-1,
- calponin-1, vascular endothelial growth factor and IL-13,
- calponin-1, phosphatidylethanolamine-binding protein 1 and IL-12,
- calponin-1, pro-collagen C-endopeptidase enhancer-1 and IL-1 ra,
- calponin-1, phosphatidylethanolamine-binding protein 1 and stromal cell derived factor 1 alpha,
- calponin-1, vascular endothelial growth factor and stromal cell derived factor 1 alpha,
- calponin-1, heat shock protein 27 and IL-1 ra,
- calponin-1, heat shock protein 27 and stromal cell derived factor 1 alpha,
- calponin-1, stromal cell derived factor 1 alpha and IL-1 ra,
- calponin-1, phosphatidylethanolamine-binding protein 1 and heat shock protein 27,
- calponin-1, phosphatidylethanolamine-binding protein 1 and thrombospondin-2,
- calponin-1, vascular endothelial growth factor and heat shock protein 27,
- calponin-1, phosphatidylethanolamine-binding protein 1 and microfibril-associated protein-4,
- calponin-1, pro-collagen C-endopeptidase enhancer-1 and stromal cell derived factor 1 alpha.

As disclosed herein, in the above defined method, step a1) may comprise or consist in determining the ratio of the level of a first biochemical marker with the level of a second biochemical marker. In this case, said first biochemical maker is preferably selected from the group consisting of IL-1ra, vascular endothelial growth factor, mature cathepsin K, peroxiredoxin-2, serum amyloid P component, IL-12, IL-8, granulocyte colony-stimulating factor, growth-regulated alpha protein, IL-13, leukemia inhibitory factor and IL-1 beta, and said second biochemical marker is preferably selected from the group consisting of alpha B crystallin, calponin-1, phosphatidylethanolamine-binding protein 1, aciculin, osteoglycin, dihydropyrimidinase-like 3, serpin B9, serpin B9-granzyme B complex, pro-cathepsin D, thrombospondin-2, thrombospondin-1, pro-collagen C-endopeptidase enhancer-1, microfibril-associated protein-4, DJ-1 oncogene, IL-1 alpha, interferon alpha-2, stromal cell derived factor 1 alpha, tissue inhibitor of metalloproteinase 1 and heat shock protein 27.

### Biochemical markers

As used herein, the expression "biochemical marker" refers to a molecule, the presence and/or level of which is characteristic of a condition. Preferably, a biochemical marker according to the invention is a protein or a peptide.

In the context of the invention, the terms "calponin-1", "calponin H1 smooth muscle", "smooth muscle troponin-like protein" and "basic calponin" are used indistinctively and refer to a calcium binding protein which inhibits the ATPase activity of myosin in smooth muscle. Phosphorylation of calponin by a protein kinase, which is dependent upon calcium binding to calmodulin, releases the calponin's inhibition of the smooth muscle ATPase. Calponin-1 is for example described in Winder and Walsh (1993) Cell Signal 5:677-686. In particular, human calponin-1 is constituted of 297 amino acids. The sequence of human calponin-1 is referenced under Swiss Prot Number Q53FP8.

In the context of the invention, the terms "alpha-B crystallin", "α-crystallin B chain", "α(B)-crystallin", "Rosenthal fiber component", "heat shock protein β-5", "renal carcinoma antigen" and "NY-REN-27" are used indistinctively and refer to a 20 kDa heat shock protein which is expressed constitutively in the lens together with α-A-crystallin, where the two proteins serve as structural proteins. Alpha-B crystallin is for example described in Horwitz (2003) Exp Eye Res 76:145-153. The sequence of human alpha-B crystallin is referenced under Swiss Prot Number P02511.

In the context of the invention, the terms "phosphatidylethanolamine-binding protein 1 ", "prostatic-binding protein", "neuropolypeptide h3", "Raf kinase inhibitor protein", "PEBP-1", "HCNPpp" and "RKIP" are used indistinctively and refer to a phospholipid-binding protein encoded by the *PEBP-1* gene and which interacts with MAP2K1, C-Raf and MAPK1. Phosphatidylethanolamine-binding protein 1 is for example described in Vance (2003) Prog Nucleic Acid Res Mol Biol 75:69-111. In particular, human phosphatidylethanolamine-binding protein 1 is constituted of 187 amino acids. The sequence of human phosphatidylethanolamine-binding protein 1 is referenced under Swiss Prot Number P30086.

In the context of the invention, the terms "aciculin", "phosphoglucomutase-like protein 5", "phosphoglucomutase-related protein", "PGMRP" and "PGM5" are used indistinctively and refer to a 567 amino acid protein that belongs to the phosphoglucomutase family of phosphotransferases and plays an important role in the interconversion of glucose-1-phosphate and glucose-6-phosphate. Localized to the cell junction and expressed at high levels in smooth and cardiac muscle, aciculin binds magnesium as a cofactor and interacts with dystrophin and utrophin, possibly playing a role in cytoskeletal organization and function. Aciculin is for example described in Moiseeva et al. (1996) Eur. J Biochem. 235:103-113. The sequence of human aciculin is referenced under Swiss Prot Number Q15124.

In the context of the invention, the terms "cathepsin K", "cathepsin O", "cathepsin X", "cathepsin O2" and "CTSK" are used indistinctively and refer to a lysosomal cysteine protease involved in bone remodeling and resorption. This protein, which is a member of the peptidase C1 protein family, is predominantly expressed in osteoclasts. It is a protease, which is defined by its high specificity for kinins and is involved in bone resorption. The enzyme's ability to catabolize elastin, collagen, and gelatin allows it to break down bone and cartilage. Cathepsin K is for example described in Lecaille et al. (2008) Biochimie 90:208-226. Cathepsin K is synthesized as an inactive pre-proenzyme which contains 329 amino acids. It includes a 15-amino acid signal sequence, a 99-amino acid pro-peptide and a region of 215 amino acids containing the overall organization of the catalytic site. The mature form of cathepsin K or "mature cathepsin K" corresponds to the region of 215 amino acids containing the overall organization of the catalytic site. The sequence of human cathepsin K is referenced under Swiss Prot Number P43235.

In the context of the invention, the terms "osteoglycin", "mimecan", "osteoinductive factor", "corneal keratan sulfate proteoglycan" and "OGN" are used indistinctively and refer to a small proteoglycan which contains tandem leucine-rich repeats and which induces ectopic bone formation in conjunction with transforming growth factor β. Osteoglycin is described for example in Funderburgh et al. (1997) J. Biol. Chem. 272:28089-28095. The sequence of human osteoglycin is referenced under Swiss Prot Number P20774.

In the context of the invention, the terms "peroxiredoxin-2", "thioredoxin peroxidase 1", "thioredoxin-dependent reductase 1", "thioredoxin-dependent reductase 1", "thiol-specific antioxidant protein", "natural killer cell-enhancing factor A" and "PRDX2" are used indistinctively and refer to a member of the peroxiredoxin family of antioxidant enzymes, which reduces hydrogen peroxide and alkyl hydroperoxides. Peroxiredoxin-2 typically plays an antioxidant protective role in cells, and may contribute to the antiviral activity of CD8⁺ T-cells. Peroxiredoxin-2 is described for example in Low et al. (2008) Antioxid. Redox. Signal. 10:1621-1630. The sequence of human peroxiredoxin-2 is referenced under Swiss Prot Number P32119.

In the context of the invention, the terms "dihydropyrimidinase-like 3", "collapsin response mediator 4", "CRMP4", "ULIP protein", "Unc-33-like phosphoprotein" and "DPYSL3 protein" are used indistinctively and refer to a member of the CRMP family constituted of 570 amino acids, involved in neuronal differentiation and regulation of synaptic rearrangement. As known from the skilled person, dihydropyrimidinase-like 3 is typically both cytosolic and membrane associated and is expressed transiently in post mitotic neurons. Dihydropyrimidinase-like 3 shares homology with the nematode gene *unc-33,* mutations of which led to aberrant patterns of axonal outgrowth. Dihydropyrimidinase-like 3 is described for example in Alabed et al. ((2007) J Neurosci. 27:1702-1711. The sequence of human dihydropyrimidinase-like 3 protein is referenced under Swiss Prot Number Q6DEN2.

As used herein, the terms "serum amyloid P component" and "9.5S α-1-glycoprotein" are used indistinctively and refer to a 25 kDa pentameric protein first identified as the pentagonal constituent of *in vivo* pathological deposits called amyloid. Serum amyloid P component is a member of the pentraxins family, characterised by calcium dependent ligand binding and distinctive flattened β-jellyroll structure similar to that of the legume lectins. Serum amyloid P component is described for example in de Haas (1999) FEMS Immunol. Med. Microbiol. 26:197-202. The sequence of human serum amyloid P component is referenced under Swiss Prot Number Q01995.

In the context of the invention, the terms "serpin B9", "cytoplasmic antiproteinase 3" and "peptidase inhibitor 9" are used indistinctively and refer to a protein that belongs to the large superfamily of serine proteinase inhibitors (serpins), which bind to and inactivates serine proteinases. Serpin B9 specifically inhibits granzyme B. Serpin B9 is described for example in Sprecher et al. (1995) J. Biol. Chem. 270:29854-29861. The sequence of human serpin B9 is referenced under Swiss Prot Number P50453.

In the context of the invention, the terms "granzyme B", "granzyme-2", "T-cell serine protease 1-3E", "Cytotoxic T-lymphocyte proteinase 2", "SECT", "Cathepsin G-like 1", "CTLA-1" and "Fragmentin-2 Human lymphocyte protein" are used indistinctively and refer to a protein that is an important serine protease necessary for target cell lysis in cell-mediated immune responses and implicated in apoptosis through its ability to activate caspase cascade. Granzyme B is described for example in Boivin et al. (2009) Lab Invest. 89:1195-1220. Granzyme B is synthesized as a precursor which contains 247 amino acids and includes an 18-amino acid signal sequence, a propeptide of 2 amino acids and a mature chain of 227 amino acids. The sequence of human granzyme B is referenced under Swiss Prot Number P10144.

The protein complex formed by the interaction between serpin B9 and granzyme B is hereinafter indifferently designated "serpin B9 - granzyme B complex", "serpin B9/granzyme B complex", "granzyme B - serpin B9 complex" or "granzyme B/serpin B9 complex".

In the context of the invention, the terms "cathepsin D" and "CTSD" are used indistinctively and refer to a lysosomal aspartic protease of the pepsin family composed of a dimer of disulfide-linked heavy and light chains, both produced from a single protein precursor. Cathepsin D is synthesized as a pre-proenzyme which contains 412 amino acids. Cathepsin D is described for example in Zaidi et al. (2008) Biochem. Biophys. Res. Commun. 376:5-9. It includes a 18-amino acid signal sequence, a 46-amino acid propeptide and a mature chain of 348 amino acids. Pro-cathepsin D results from the cleavage of the signal sequence of the pre-proenzyme, and corresponds to the peptide of 52 kDa consisting of the propeptide (46 amino acid residues) linked to the mature chain (348 amino acid residues). The mature chain can be processed further to the light chain of 97 amino acids and the heavy chain of 244 amino acids. The sequence of human cathepsin D is referenced under Swiss Prot Number P07339.

In the context of the invention, the terms "thrombospondin-2", "TSP2" and "THBS2" are used indistinctively and refer to a matricellular glycoprotein which is a member of the thrombospondin family. Thrombospondin-2 is synthesized as a precursor which contains 1172 amino acids. The mature secreted protein comprises 1154 amino acids and assembles into a disulfide linked homotrimer. Secreted thrombospondin-2 is a glycoprotein with a molecular mass of 150-160 kDa that contains approximately 7 potential asparagine-linked oligosaccharide attachment sites and variable numbers of C-mannosylated tryptophane residues in the type 1 repeats. Thrombospondin-2 is for example described in Carlson et al. (2008) Cell. Mol. Life Sci. 65:672-686. The sequence of human thrombospondin-2 is referenced under Swiss Prot Number P35442.

In the context of the invention, the terms "thrombospondin-1", "TSP1" and "THBS1" are used indistinctively and refer to a member of the thrombospondin family which is a subunit of a disulfide-linked homotrimeric protein that is an adhesive glycoprotein that mediates cell-to-cell and cell-to-matrix interactions. Thrombospondin-1 is for example described in Lawler and Hynes (1986) J. Cell. Biol. 103:1635-1648. The sequence of human thrombospondin-1 is referenced under Swiss Prot Number P07996.

In the context of the invention, the terms "pro-collagen C-endopeptidase enhancer-1", "pro-collagen COOH-terminal proteinase enhancer 1", "pro-collagen C proteinase enhancer 1 ", "type 1 pro-collagen C-proteinase enhancer protein" and "PCPE-1" are used indistinctively and refer to a protein that binds to the C-terminal propeptide of type I procollagen and enhances procollagen C-proteinase activity. Pro-collagen C-endopeptidase enhancer 1 is for example described in Takahara et al. (1994) J. Biol. Chem. 269:26280-26285. The sequence of human pro-collagen C-endopeptidase enhancer-1 is referenced under Swiss Prot Number Q15113.

In the context of the invention, the terms " DJ-1 oncogene", "protein DJ-1", "Parkinson disease protein 7" and "PARK7 protein" are used indistinctively and refer to an ubiquitously expressed protein identified as an oncogene product, involved in breast cancer, lung cancer and prostate cancer. Although the exact function of DJ-1 remains unclear, increasing studies revealed that DJ-1 has multiple roles in various biological processes. In particular, DJ-1 scavenges oxidative stress, has chaperone activity that prevents the aggregation of α-synuclein, and plays an important role in anti-apoptosis. Besides those biological functions, DJ-1 was reported to function as an important transcriptional regulator in multiple pathways. DJ-1 is for example described in Vasseur et al. (2009) Proc. Natl. Acad. Sci. USA 106:1111-1116. The sequence of human oncogene DJ-1 is referenced under Swiss Prot Number Q99497.

In the context of the invention, the terms " tissue inhibitor of metalloproteinase 1", "TIMP-1" ,"Metalloproteinase inhibitor 1 ","Fibroblast collagenase inhibitor" and "Erythroid-potentiating activity" are used indistinctively and refer to a member of the metalloproteinase inhibitor family. Formation of complexes with metalloproteinases leads to the inactivation of the metalloproteinases by binding to their catalytic zinc cofactor. Tissue inhibitor of metalloproteinase 1 is for example described in Osthues et al. (1992) FEBS Lett. 296:16-20. The sequence of human tissue inhibitor of metalloproteinase 1 is referenced under Swiss Prot Number P01033.

In the context of the invention, the term "microfibril-associated protein-4" and "MFAP-4" are used indistinctively and refer to a protein with similarity to a bovine microfibril-associated protein, which has binding specificities for both collagen and carbohydrate. It is thought to be an extracellular matrix protein which is involved in cell adhesion or intercellular interactions. MFAP-4 is for example described in Lausen et al. (1999) J. Biol. Chem. 274:32234-32240. The sequence of human MFAP-4 is referenced under Swiss Prot Number P55083.

In the context of the invention, the terms "heat shock protein 27", "heat shock 27 kDa protein", "heat shock protein β1", "stress-responsive protein 27", "estrogen-regulated 24 kDa protein", "28 kDa heat shock protein" and "HSP27" are used indistinctively and refer to a 27 kDa small heat shock protein constituted of 205 amino acids that functions as a chaperone molecule and has been shown to be involved in thermotolerance, inhibition of apoptosis, regulation of cell development, cell differentiation and signal transduction. HSP27 is described for example in Ciocca et al. (1993) J. Natl. Cancer Inst. 85:1558-1570. The sequence of human HSP27 is referenced under Swiss Prot Number P04792.

In the context of the invention, the terms "interleukin 12", "IL-12" or "p70" are used indistinctively and refer to an interleukin that is naturally produced by dendritic cells, macrophages and human B-lymphoblastoid cells (NC-37) in response to antigenic stimulation. IL-12 is composed of a bundle of four α helices. IL-12 is described for example in Del Vecchio et al. (2007) Clin. Cancer Res. 13:4677-4685. It is a heterodimeric cytokine encoded by two separate genes, IL-12A (also called natural killer cell stimulatory factor 1, cytotoxic lymphocyte maturation factor 1 or p35) and IL-12B (also called natural killer cell stimulatory factor 2, cytotoxic lymphocyte maturation factor 2 or p40). IL-12A is synthesized as a precursor which contains 253 amino acids and includes a 56-amino acid signal sequence and a mature chain of 197 amino acids. The sequence of human IL-12A is referenced under Swiss Prot Number P29459. IL-12B is synthesized as a precursor which contains 328 amino acids and includes a 22-amino acid signal sequence and a mature chain of 306 amino acids. The sequence of human IL-12B is referenced under Swiss Prot Number P29460.

In the context of the invention, the terms "vascular endothelial growth factor", "vascular permeability factor" and "VEGF" are used indistinctively and refer to a homodimeric 34-42 kDa, heparin-binding glycoprotein with potent angiogenic, mitogenic and vascular permeability-enhancing activities specific for endothelial cells. VEGF is described for example in Nieves et al. (2009) Biofactors 35:332-337. The sequence of human VEGF is referenced under Swiss Prot Number P15692.

In the context of the invention, the terms "interleukin 8", "IL-8", "CXC motif chemokine 8", "CXCL8", "monocyte-derived neutrophil chemotactic factor", "T-cell chemotactic factor", "neutrophil-activating protein 1", "protein 3-10C", "granulocyte chemotactic protein 1", "monocyte-derived neutrophil-activating peptide" and "emoctakin" are used indistinctively and refer to a chemokine produced by macrophages and other cell types such as epithelial cells, and which is a member of the CXC chemokine family. IL-8 is described for example in Baggiolini et al. (1995) Int. J. Immunopharmacol. 17:103-108. IL-8 is synthesized as a precursor which contains 99 amino acids and includes a 20-amino acid signal sequence and a mature chain of 79 amino acids. The sequence of human IL-8 is referenced under Swiss Prot Number P10145.

In the context of the invention, the terms "interleukin-1 receptor antagonist protein", "IL-1 ra", "IL1 inhibitor", "ICIL-1 RA" and "anakinra" are used indistinctively and refer to an agent which binds to the same receptor on the cell surface as IL-1 (IL1R), and thus prevents IL-1 from sending a signal to that cell. IL-1ra is a member of the interleukin 1 cytokine family. IL-1ra is described for example in Dinarello (1998) Int. Rev. Immunol. 16:457-499. The sequence of human IL-1ra is referenced under Swiss Prot Number P18510.

In the context of the invention, the terms "granulocyte colony-stimulating factor", "pluripoietin", "filgrastim", "lenograstim" and "G-CSF" are used indistinctively and refer to a glycoprotein, growth factor or cytokine produced by a number of different tissues to stimulate the bone marrow to produce granulocytes and stem cells. G-CSF then stimulates the bone marrow to release them into the blood. It also stimulates the survival, proliferation, differentiation, and function of neutrophil precursors and mature neutrophils. G-CSF is described for example in Dale (1998) Trans. Am. Clin. Climatol. Assoc. 109:27-36. G-CSF is synthesized as a precursor which contains 204 amino acids and includes a 30-amino acid signal sequence and a mature chain of 174 amino acids. The sequence of human G-CSF is referenced under Swiss Prot Number P09919.

In the context of the invention, the terms "GRO alpha", "growth-regulated alpha protein", "CXC motif chemokine 1", "melanoma growth stimulatory activity" and "neutrophil-activating protein 3" are used indistinctively and refer to a member of the chemokine alpha family that is characterized by the separation, with one amino acid, of the first two cysteine residues, C-X-C, in the amino acid sequence. It is typically a chemoattractant for both T-cells and neutrophils and may be one of the chemokines involved in cell maintenance. GRO alpha is described for example in Dhawan and Richmond (2002) J. Leukoc. Biol. 72:9-18. GRO alpha is synthesized as a precursor which contains 107 amino acids and includes a 34-amino acid signal sequence and a mature chain of 73 amino acids. The sequence of human GRO alpha is referenced under Swiss Prot Number P09341.

In the context of the invention, the terms "interleukin 13" or "IL-13" are used indistinctively and refer to a cytokine secreted by many cell types, but especially T helper type 2 cells, that is an important mediator of allergic inflammation and disease. IL-13 is described for example in McKenzie and Zurawski (1995) Cancer Treat. Res. 80:367-378. IL-13 is synthesized as a precursor which contains 132 amino acids and includes a 20-amino acid signal sequence and a mature chain of 112 amino acids. The sequence of human IL-13 is referenced under Swiss Prot Number P35225.

In the context of the invention, the terms "leukemia inhibitory factor", "LIF", "differentiation-stimulating factor", "melanoma-derived LPL inhibitor" and "emfilermin" are used indistinctively and refer to an interleukin 6 class cytokine which affects cell growth and development. LIF derives its name from its ability to induce the terminal differentiation of myeloid leukaemic cells. Other properties attributed to LIF include: growth promotion and cell differentiation of different types of target cells, influence on bone metabolism, cachexia, neural development, embryogenesis and inflammation. LIF binds to the specific LIF receptor (LIFR-α) which forms a heterodimer with a specific subunit common to all members of that family of receptors, the GP130 signal transducing subunit. LIF is described for example in Metcalf (1992) Growth Factors 7:169-173. LIF is synthesized as a precursor which contains 202 amino acids and includes a 22-amino acid signal sequence and a mature chain of 180 amino acids. The sequence of human LIF is referenced under Swiss Prot Number P15018.

In the context of the invention, the terms "interleukin 1α", "IL-1 alpha", 'IL-1A" and "hematopoietin-1" are used indistinctively and refer to a member of the interleukin 1 cytokine family, which is a pleiotropic cytokine involved in various immune responses, inflammatory processes, and hematopoiesis. IL-1 alpha is produced by monocytes and macrophages as a 33 kDa proprotein, which is proteolytically processed by calpain and released in response to cell injury, and thus induces apoptosis. IL-1 alpha is for example described in Furutani (1994) Eur. Cytokine Netw. 5:533-538. Typically, IL-1 alpha is synthesized as a precursor which contains 271 amino acids and includes a 112-amino acid N-terminal region which is actively transported into the cell nucleus and a mature chain of 159 amino acids. The sequence of human IL-1 alpha is referenced under Swiss Prot Number P01583.

In the context of the invention, the terms "interleukin 1β", "IL-1 beta", "IL-1B" and "catabolin" are used indistinctively and refer to a member of the interleukin 1 cytokine family which is produced by activated macrophages as a proprotein, which is proteolytically processed to its active form by caspase 1. IL-1 beta is an important mediator of the inflammatory response, and is involved in a variety of cellular activities, including cell proliferation, differentiation, and apoptosis. IL-1 beta is for example described in Veerapandian et al. (1992) Proteins 12:10-23. Typically, IL-1 beta is synthesized as a precursor which contains 269 amino acids and includes a 116-amino acid N-terminal region and a mature chain of 153 amino acids. The sequence of human IL-1 beta is referenced under Swiss Prot Number P01584.

In the context of the invention, the terms "interferon alpha-2" and "IFN α2" are used indistinctively and refer to a type I interferon that is involved in the innate immune response against viral infections. Interferons are proteins which produce antiviral and antiproliferative responses in cells. On the basis of their sequence, interferons are classified into five groups: α, α-2 (or ε), β, δ (or trophoblast). IFN α2 is for example described in Kontseková et al. (1999) Int. J. Biol. Macromol. 24:11-14. The sequence of human interferon alpha-2 is referenced under Swiss Prot Number P01563.

In the context of the invention, the terms "stromal cell derived factor 1 alpha", "CXC motif chemokine 12α", "CXCL12α" and "SDF-1a" are used indistinctively and refer to small cytokine belonging to the chemokine family that is officially designated Chemokine (C-X-C motif) ligand 12 (CXCL12). SDF-1α belongs in particular to the intercrine family, members of which activate leukocytes and are often induced by proinflammatory stimuli such as lipopolysaccharide, TNF, or IL1. The intercrines are characterized by the presence of four conserved cysteines which form two disulfide bonds. SDF-1α is for example described in Kucia et al. (2004) J. Mol. Histol. 35:233-245. Typically, SDF-1α is synthesized as a precursor which contains 89 amino acids and includes a 21-amino acid signal peptide and a mature chain of 68 amino acids. The sequence of human stromal cell derived factor 1 alpha is referenced under Swiss Prot Number P48061.

In the context of the invention, the above cited Swiss Prot references are those that were available on June 11, 2010.

### Measurement of the presence and/or level of the biochemical markers

The first step of the method of determining the presence of an unstable atherosclerotic plaque defined above consists in measuring the presence and/or level of at least one biochemical marker as defined above in a biological sample obtained from the individual.

As used herein, the "level" of a biochemical marker corresponds to the concentration, the amount or the activity of the biochemical marker. Preferably, the level of a biochemical marker refers to the amount or concentration of the biochemical marker, more particularly to the molar or the mass amount or to the molar or the mass concentration of the biochemical marker.

Techniques to measure the level of a biochemical marker in a biological sample are well-known from one skilled in the art. Examples of suitable techniques include mass spectrometry, but also ligand-based methods such as immunological assays (simplex or multiplex), in particular enzyme-linked immunosorbent assays (ELISA), immunofluorescent assays (IFA), radioimmunoassays (RIA), competitive binding assays, and the like. Other suitable techniques include Multiple Reaction Monitoring (MRM), Stable Isotope Standards and Capture by Anti-Peptide Antibodies (SISCAPA; Anderson et al. (2004) J. Proteome Res. 3:235-244; Anderson et al. (2004) J. Proteome Res. 3:228-234) or imaging technologies such as immunostaining of tissue samples and confocal laser scanning microscopy. In particular the levels of the biochemical markers are measured using ligands specific for said biochemical markers.

A "ligand" relates to a molecule which has binding affinity towards a marker. In particular it is preferred that the ligand binds to the marker in a specific manner, that is the ligand preferentially binds to the marker it is specific for as compared to other components of the biological sample. The specific ligands are in particular selected from the group consisting of polyclonal, monoclonal and recombinant antibodies, or fragments thereof, such as Fab fragments, F(ab')₂ fragments and scFv fragments; phage antibodies (PhAbs); llama and camel antibodies; peptide ligands (as derived from protein scaffold like the Sac 7 D protein) and aptamers. Preferably, the specific ligands are monoclonal antibodies.

Still preferably, the specific ligands are antibodies that enable detecting the biochemical markers according to the invention in a liquid biological sample, in particular in a plasma sample. Such specific ligands are for example antibodies comprised in the following commercially available kits: HSP27 ELISA Kit (Merck), ELISA Kit for Peroxiredoxin-2 (USCN Life Science Inc.), Human TIMP-1 Quantikine ELISA Kit (R&D Systems), ELISA Kit for PCPE-1 (USCN Life Science Inc.), Circulex Human DJ-1/PARK7 ELISA Kit (MBL International Corporation), ELISA Kit for osteoglycin (USCN Life Science Inc.) and Human thrombospondin-2 Quantikine ELISA Kit (R&D Systems). Such specific ligands may also be obtained by conventional techniques known from the skilled person, typically comprising immunisation of mice with the recombinant protein to be detected, lymphocyte fusion between spleen cells of the immunised mice and myeloma SP2 cells thereby obtaining hybridomas producing monoclonal antibodies, and testing of the obtained antibodies for their sensitivity and specificity to detect proteins of interest in human plasmas. Techniques to test the sensitivity and specificity of antibodies to detect proteins in human plasmas are well-known from the skilled person.

The level of the biochemical marker may also be measured by determining the level of mRNA expression for said biochemical marker. Suitable techniques to measure the level of such mRNA include for example RT-PCR and qRT-PCR.

Preferably, the step of measuring the presence and/or the level of the at least one biochemical marker is carried out by an immunological assay, mass spectrometry, Multiple Reaction Monitoring (MRM), Stable Isotope Standards and Capture by Anti-Peptide Antibodies (SISCAPA), RT-PCR, imaging technology such as immunostaining of tissue samples, or *in vivo* immunoimaging.

The step of measuring the presence and/or the level of the at least one biochemical marker may in particular be carried out by an ELISA assay. Preferably, the step of measuring the presence and/or the level of the at least one biochemical marker is carried out by an ELISA assay enabling detecting said at least one biochemical marker in a liquid biological sample, in particular in a plasma sample. Such ELISA assays are well-known from the skilled person and may be typically performed as follows: plates are coated with a capture monoclonal antibody which is specific of the biochemical marker to be detected and is able to bind to said biochemical marker in a liquid biological sample, in particular in a plasma sample. Standard range of recombinant protein of the biochemical marker to be detected or liquid biological samples, in particular plasma samples, are mixed with a detection monoclonal antibody coupled with a detectable label, such as peroxidase enzyme and which is specific of said biochemical marker to be detected and is able to bind to said biochemical marker in a liquid biological sample, in particular in a plasma sample. This mixed solution is then dispensed on the coated plates and incubated during a suitable period to enable the formation of immunocomplexes. A chromogenic or fluorogenic solution is then typically added to allow the detection of the immunocomplexes so obtained. The level and/or the presence of the biochemical marker is then measured typically by measuring optical densities and using a dose response curve of the standard of the biochemical marker to be detected.

As intended herein in the methods of determining the presence of an unstable atherosclerotic plaque according to the invention, the expression "biological sample" encompasses all samples which can be taken from a patient, such as tissue biopsies or samples of body fluids. In particular, the biological sample may be a liquid biological sample. The biological sample may thus be selected from the group consisting of blood sample, serum, plasma, saliva, urine and tears, in particular blood sample, serum, plasma and urine. It is particularly preferred in the invention that the liquid biological samples are blood-based samples, such as whole-blood samples, serum or plasma samples. In case of blood based samples, any anticoagulant, such as EDTA, citrate or heparin, can be added. In particular, the biological sample may consist of cells taken from a subject or a sample of a body fluid of an individual which may be derived from blood. Thus, the biological sample may consist of erythrocytes, isolated mononuclear cells, neutrophiles and/or mixtures thereof. Additionally, the liquid biological sample may be a native body fluid sample or a pre-treated body fluid sample.

The biological sample may also be a tissue sample, preferably an artery sample.

Furthermore, the levels of the biochemical markers can be measured from different samples taken from a same patient or from a unique sample.

Moreover, the levels of biochemical markers in the samples can be determined individually, in simplex assays, or optionally, the levels of some or all of the biochemical markers can be determined simultaneously, in multiplex assays.

The level of a biochemical marker can be determined once or repeatedly, notably according to a predefined time-course.

It is preferred that the level of a biochemical marker is determined using an automated assay system, such as an automated system suitable for single assays or an automated system suitable for multiplex assays, such as the BioPlex™ 2200 system.

Preferably, in the methods of the invention wherein the level of at least two biochemical markers is measured in step a1), a "virtual" marker transposing biochemical markers from the multivariate conditions into an univariate setting is determined by linearly combining the measured levels of said at least two biochemical markers. Techniques to determine such "virtual" markers are well-known from the skilled person. Examples of such techniques include methods wherein Receiver operating characteristic (ROC) curves, obtained for each biochemical marker of the combination, are regrouped by the best linear combination, which maximises the area under the ROC curve under the hypothesis of a multivariate distribution as described in Kramar et al. (2001) Computer Methods and Programs in Biomedicine 66:199-207 and Su and Liu (1993) J. American Statistical Association 88:1350-1355; or methods of iterative marginal optimization (IMO) as described in Wang (2007) Computational Statistics & Data Analysis 512803-2812. Such "virtual" markers may for example be calculated using the multiple Receiving-Operating-Characteristic (mROC) software (Kramar *et al.* 2001).

### Comparison with a predetermined value

In a particular embodiment of the method of detecting the presence of an unstable atherosclerotic plaque as defined above, step a1) is followed by a step a2) of comparing the presence and/or level of the at least one biochemical marker with at least one predetermined value, and step b1) consists in determining the presence of an unstable atherosclerotic plaque in the individual, based on the comparison in step a2).

In another particular embodiment of the methods according to the invention wherein the level of at least two biochemical markers is determined, the level of a virtual marker, obtained from the levels of said at least two biochemical markers as defined above, may be compared with at least one predetermined value.

The predetermined value may be a threshold value such as a median or mean or a range of values such as a confidence interval.

In particular, the predetermined value may correspond to the mean level of the biochemical marker in a population of individuals known to have no unstable atherosclerotic plaque. The predetermined value may also correspond to the range of values of the level of the biochemical marker which is the most observed in a population of individuals known to have no unstable atherosclerotic plaque.

In the context of the invention, a "population of individuals known to have no unstable atherosclerotic plaque" refers to a population of individuals wherein no unstable atherosclerotic plaque has been detected. Accordingly, a population of individuals known to have no unstable atherosclerotic plaque may comprise healthy individuals and/or individuals who have stable atherosclerotic plaques.

As used herein, a "healthy individual" means an individual who has not previously had a cardiovascular event associated with a vascular disease. Healthy individuals also do not otherwise exhibit symptoms of disease. In other words, such individuals, if examined by a medical professional, would be characterized as healthy and free of symptoms of disease.

As used herein, an "individual who has stable atherosclerotic plaques" means an individual, who has been diagnosed as having at least one stable atherosclerotic plaque, but no unstable atherosclerotic plaque.

The predetermined value may also correspond to the level of the biochemical marker measured in a biological sample taken from the individual prior to the time of sampling of the biological sample in which the biochemical marker is measured in step a1). For example, the predetermined value may be the level of said marker measured in a sample taken from the individual at a time t₀, whereas the level of the biochemical marker determined in step a1) is measured in a biological sample taken from the same individual at a time tₓ, wherein to and tₓ are different and tₓ is posterior to t₀. The order of magnitude of the period Δ between tₓ and to (Δ = tₓ-t₀) is preferably in months (preferably 24 months, still preferably 20 months, still preferably 18 months, still preferably 16 months, still preferably 14 months, still preferably 12 months, still preferably 11 months, still preferably 10 months, still preferably 9 months, still preferably 8 months, still preferably 7 months, still preferably 6 months, still preferably 5 months, still preferably 4 months, still preferably 3 months, still preferably 2 months, still preferably 1 month). More preferably, the order of magnitude of Δ is in weeks (preferably 6 weeks, more preferably 5 weeks, still preferably 4 weeks, still preferably 3 weeks, still preferably 2 weeks, still preferably one week). Still preferably, the order of magnitude of Δ is in days (preferably 45 days, more preferably 30 days, still preferably 25 days, still preferably 20 days, still preferably 18 days, still preferably 15 days, still preferably 12 days, still preferably 10 days, still preferably 8 days, still preferably 6 days, still preferably 5 days, still preferably 4 days, still preferably 3 days, still preferably 2 days and still preferably 1 day). Still preferably, the order of magnitude of Δ is in hours (preferably 72 hours, more preferably 60 hours, still preferably 48 hours, still preferably 36 hours, still preferably 24 hours, still preferably 18 hours, still preferably 12 hours, still preferably 10 hours, still preferably 8 hours, still preferably 6 hours, still preferably 4 hours, still preferably 3 hours, still preferably 2 hours, still preferably 1 hour). Still preferably, Δ is 90 minutes, still preferably 60 minutes, still preferably 45 minutes and still preferably 30 minutes. Preferably, t₀ corresponds to the time of the sampling at the admission of the patient.

As known from the skilled person, the predetermined value is dependent on the biological sample type and on the method used for measuring the level of the biochemical marker in the biological sample. Thus, the predetermined value is preferably provided by using the same assay technique as is used for measurement of the individual's biochemical marker levels, to avoid any error in standardization.

Preferably, in the methods for determining the presence of an unstable atherosclerotic plaque of the invention, it is further determined whether the level of the at least one biochemical marker is increased or decreased compared to the at least one predetermined value.

Preferably, when the level of the biochemical marker is increased compared to the predetermined value, its level is significantly higher than the predetermined value.

Preferably, when the level of the biochemical marker is decreased compared to the predetermined value, its level is significantly lower than the predetermined value.

The present inventors have demonstrated that the level of some of the above defined biochemical markers was increased when an unstable atherosclerotic plaque was present, whereas the level of some of the above defined biochemical markers was decreased when an unstable atherosclerotic plaque was present.

Accordingly, preferably, in the methods for determining the presence of an unstable atherosclerotic plaque described herein, measuring, in the biological sample, in particular in the tissue sample, an increased level of expression of at least one biochemical marker, is indicative of the presence of an unstable atherosclerotic plaque, said at least one biochemical marker being selected from the group consisting of:
- IL-1ra,
- vascular endothelial growth factor,
- mature cathepsin K,
- peroxiredoxin-2,
- serum amyloid P component,
- IL-12,
- IL-8,
- granulocyte colony-stimulating factor,
- growth-regulated alpha protein,
- IL-13,
- leukemia inhibitory factor, and
- I L-1 beta.

Preferably, said at least one biochemical marker is selected from the group consisting of mature cathepsin K, serum amyloid P component, IL-12, IL-1ra, granulocyte colony-stimulating factor, leukemia inhibitory factor and IL-1 beta.

Still preferably, in the methods for determining the presence of an unstable atherosclerotic plaque described herein, measuring, in the biological sample, in particular in the tissue sample, a decreased level of expression of at least one biochemical marker, is indicative of the presence of an unstable atherosclerotic plaque, said at least one biochemical marker being selected from the group consisting of:
- alpha B crystallin,
- calponin-1,
- phosphatidylethanolamine-binding protein 1,
- aciculin,
- osteoglycin,
- dihydropyrimidinase-like 3,
- serpin B9,
- serpin B9-granzyme B complex,
- pro-cathepsin D,
- thrombospondin-2,
- thrombospondin-1,
- pro-collagen C-endopeptidase enhancer-1,
- microfibril-associated protein-4,
- DJ-1 oncogene,
- IL-1 alpha,
- interferon alpha-2,
- stromal cell derived factor 1 alpha,
- tissue inhibitor of metalloproteinase 1, and
- heat shock protein 27.

Preferably, said at least one biochemical marker is selected from the group consisting of calponin-1, alpha B crystallin, phosphatidylethanolamine-binding protein 1, aciculin, dihydropyrimidinase-like 3, serpin B9-granzyme B complex, pro-cathepsin D, thrombospondin-1, pro-collagen C-endopeptidase enhancer-1, IL-1 alpha, interferon alpha-2, stromal cell derived factor 1 alpha, microfibril-associated protein-4, DJ-1 oncogene and tissue inhibitor of metalloproteinase 1.

As described herein, in the methods for determining the presence of an unstable atherosclerotic plaque disclosed herein, measuring, in the liquid biological sample, in particular in the plasma sample, an increased level of expression of at least one biochemical marker, may be indicative of the presence of an unstable atherosclerotic plaque, said at least one biochemical marker being calponin-1.

As described herein, in the methods for determining the presence of an unstable atherosclerotic plaque disclosed herein, measuring, in the liquid biological sample, in particular in the plasma sample, a decreased level of expression of at least one biochemical marker, is indicative of the presence of an unstable atherosclerotic plaque, said at least one biochemical marker being selected from the group consisting of IL-8, DJ-1 oncogene and vascular endothelial growth factor.

As known from the skilled person, unstable atherosclerotic plaques often lead to complications due to sudden plaque rupture. Accordingly, determining the presence of an unstable atherosclerotic plaque in an individual is an important step for diagnosing vascular or metabolic disease or an increased predisposition to an adverse outcome.

### Methods of diagnosis of a vascular or metabolic disease or of an increased predisposition to an adverse outcome

The present invention thus also relates to a method, preferably an *in vitro* method, for diagnosing a condition in an individual, comprising the step of determining the presence of an unstable atherosclerotic plaque using the method of the invention, the presence of an unstable atherosclerotic plaque being indicative of said condition, wherein said condition is (i) a vascular or metabolic disease or (ii) an increased predisposition to an adverse outcome.

As intended herein, the term "diagnosis" or "diagnosing" refers to the pathology afflicting an individual (*e.g*., a disease, disorder, syndrome, medical condition and/or a symptom thereof), determining a severity of the pathology, monitoring the progression of a pathology (*e.g.* therapeutic follow-up), forecasting an increased predisposition to an adverse outcome (*e.g*. prognosis) and determining a probability to experience certain adverse outcome to an individual (e.g. risk stratification).

As used herein, the term "prognosis" refers to an increased probability or predisposition that a certain course or outcome will occur; that is, that a course or outcome is more likely to occur in a patient exhibiting a given characteristic, such as the presence or level of a prognostic indicator, in particular one of the biochemical markers defined above, when compared to those individuals not exhibiting the characteristic.

A prognosis is often determined by examining one or more "prognostic indicators" or "prognostic tools". These are markers, the presence or amount of which in an individual (or a sample obtained from the individual) signals a probability or a predisposition that a given course or outcome will occur. For example, preferred prognostic indicators in the present invention are at least one of the biochemical markers defined above.

The skilled artisan will understand that associating a prognostic indicator with a predisposition to an adverse outcome is a statistical analysis. Statistical significance is often determined by comparing two or more populations, and determining a confidence interval and/or a p value. See, *e.g.,* Dowdy and Wearden, Statistics for Research, John Wiley & Sons, New York, 1983.

In the context of the invention, the term "outcome" refers to the health status of an individual following treatment for a disease or disorder, or in the absence of treatment. Outcomes are in particular adverse outcomes. Adverse outcomes include in particular death, heart failure, stroke and myocardial infarction.

As used herein, the term "risk stratification" refers to an arraying of known clinical risk factors to allow physicians to classify patients into a low, moderate, high or highest risk of developing a particular disease, disorder or condition.

As used herein, the term "therapeutic follow-up" refers to the monitoring of the efficacy of a treatment against a particular pathology with a known active compound or with a candidate drug compound.

In the context of the invention, a "vascular disease" refers to a disease that involves the heart or blood vessels (arteries and veins). More particularly, a vascular disease according to the invention denotes a disease, lesion or symptom associated with an atherogenesis process that affects the cardiovascular system. It includes especially the conditions in which an atheroma plaque develops as well as the complications due to the formation of an atheroma plaque (stenosis, ischemia) and/or due to its evolution (plaque rupture inducing atherothrombosis) towards an acute ischemic event (thrombosis, embolism, infarction, arterial rupture).

Vascular diseases include chronic heart disease, acute coronary syndrome, coronary artery disease, coronary heart disease, hypertension, atherosclerosis, in particular coronary or carotid atherosclerosis, peripheral atherosclerotic disease (PAD) including iliac or femoral atherosclerosis, aortic dissection, unstable angina, thrombosis, heart failure, stroke, vascular aneurysm, vascular calcification, myocardial infarction, vascular stenosis and infarction, thrombotic disorder and vascular dementia. Preferably, the vascular disease according to the invention is selected from the group consisting of chronic heart disease, acute coronary syndrome, stroke, peripheral atherosclerotic disease and thrombotic disorder.

As intended herein, the term "chronic heart disease" refers to any illness that seriously compromises the heart's natural ability to function.

As intended herein, "acute coronary syndrome" (ACS) relates to a group of coronary disorders that result from ischemic insult to the heart. ACS includes unstable angina, non-ST-elevation non-Q wave myocardial infarction (NSTEMI), ST-elevation non-Q wave myocardial infection (STEMI), and transmural (Q-wave) myocardial infarction. ACS can be divided into non-ST-elevation ACS (NSTEACS) and ST-elevation ACS (STEACS).

The expression "non-ST-elevation acute coronary syndrome" refers to those ACS not associated with an elevated ST component in an electrocardiogram. Non-ST-elevation ACS include unstable angina and NSTEMI (see *e.g.* Nyman et al. (1993) J. Intern. Med. 234:293-301; Patel et al. (1996) Heart 75:222-228; Patel et al. (1998) Eur. Heart J. 19:240-49; and Lloyd-Jones et al. (1998) Am. J. Cardiol. 81:1182-86).

As used herein, a "coronary artery disease" denotes a disease corresponding to the end result of the accumulation of atheromatous plaques within the walls of the arteries that supply the myocardium with oxygen and nutrients. It is one of the most common causes of coronary heart disease.

As used herein, a "coronary heart disease" denotes a progressive disease, due to a bad irrigation of the heart muscle, consecutive to the narrowing (stenosis) or calcification (sclerosis) of a coronary artery. The complete obstruction of a coronary artery leads to myocardial infarction.

As used herein, "hypertension", also referred to as "high blood pressure", "HTN" or "HPN", denotes a medical condition in which the blood pressure is chronically elevated.

As used herein, "aortic dissection" refers to the condition wherein a tear in the inner wall of the aorta causes blood to flow between the layers of the wall of the aorta and force the layers apart.

As used herein, "atherosclerosis" denotes a disease affecting arterial blood vessels. Atherosclerosis can be characterized by a chronic inflammatory response in the walls of arteries, mainly due to the accumulation of macrophages and promoted by low density lipoproteins without adequate removal of fats and cholesterol from macrophages by functional high density lipoproteins.

As used herein, "angina pectoris" or "angina" denotes a severe chest pain due to ischemia of the heart muscle and includes stable and unstable angina. "Stable angina" or "effort angina" refers to the more common understanding of angina related to myocardial ischemia. Typical presentations of stable angina is that of chest discomfort and associated symptoms precipitated by some activity (running, walking, etc.) with minimal or non-existent symptoms at rest. "Unstable angina" or "crescendo angina" is defined as angina pectoris that changes or worsens.

As used herein, "thrombosis" denotes the formation of a blood clot inside a blood vessel.

As used herein, "heart failure" denotes a condition in which a problem with the structure or function of the heart impairs its ability to supply sufficient blood flow to meet the body's needs.

As used herein, a "stroke" denotes the rapidly developing loss of brain functions due to a disturbance in the blood vessels supplying blood to the brain. Strokes can be classified in particular into ischemic stroke and hemorrhagic stroke. Ischemic strokes are those that are due to interruption of the blood supply, while hemorrhagic strokes are the ones which are due to rupture of a blood vessel or an abnormal vascular structure. As used herein, the term "stroke" also includes transient ischemic attacks, also called TIA or "mini stroke", which is a change in the blood supply to a particular area of the brain, resulting in brief neurologic dysfunction that persists, by definition, for less than 24 hours.

As used herein, a "vascular aneurysm" or "aneurysm" denotes a localized, blood-filled dilation of a blood vessel caused by a disease or weakening of the vessel wall. Aneurysms most commonly occur in arteries at the base of the brain and in the aorta.

As used herein, an "infarction" denotes the process resulting in a macroscopic area of necrotic tissue in some organ caused by loss of adequate blood supply. In particular, a "myocardial infarction" denotes the interruption of blood supply to part of the heart.

As used herein, a "thrombotic disorder" denotes any disorder or condition characterized by excessive or unwanted coagulation or hemostatic activity, or a hypercoagulable state. Thrombotic disorders include diseases or disorders involving platelet adhesion and thrombus formation, and may manifest as an increased propensity to form thromboses, e.g., an increased number of thromboses, thrombosis at an early age, a familial tendency towards thrombosis, and thrombosis at unusual sites.

As used herein, a "vascular dementia" denotes a degenerative cerebrovascular disease that leads to a progressive decline in memory and cognitive functioning. It includes in particular multi-infarct dementia (multiple large and complete infarcts), post-hemorrhage dementia, subcortical vascular dementia (small vessel disease) and mixed dementia (combination of Alzheimer disease and vascular dementia).

In the context of the invention, a "metabolic disease" denotes a disease that disrupts normal metabolism. Preferably, the metabolic disease according to the invention is selected from the group consisting of diabetes, renal insufficiency, dyslipidemia and metabolic syndrome.

As used herein, "diabetes" denotes a syndrome of disordered metabolism, usually due to a combination of hereditary and environmental causes, resulting in abnormally high blood sugar levels.

As used herein, "renal insufficiency" or "renal failure" refers to when an individual's kidneys no longer have enough kidney function to maintain a normal state of health. Renal insufficiency includes both acute and chronic renal failure, including end-stage renal disease (ESRD).

As used herein, "dyslipidemia" denotes a disruption in the amount of lipids in the blood.

As used herein, "metabolic syndrome" denotes a combination of medical disorders that increase the risk of developing cardiovascular disease and diabetes. It is typically defined by the World Health Organization as a condition wherein diabetes mellitus, impaired glucose tolerance, impaired fasting glucose or insulin resistance are present, and two of the following features are observed in the individual: (i) blood pressure: ≥ 140/90 mmHg; (ii) dyslipidemia: triglycerides (TG) ≥ 1.695 mmol/L and high-density lipoprotein cholesterol (HDL-C) ≤ 0.9 mmol/L (male), ≤ 1.0 mmol/L (female); (iii) central obesity: waist:hip ratio > 0.90 (male); > 0.85 (female), or body mass index > 30 kg/m²; and (iv) microalbuminuria: urinary albumin excretion ratio ≥ 20 µg/min or albumin:creatinine ratio ≥ 30 mg/g.

Preferably, the vascular or metabolic disease according to the invention is selected from the group consisting of chronic heart disease, coronary artery disease, acute coronary syndrome, unstable angina, stroke, peripheral atherosclerotic disease, aortic dissection, aneurism, diabetes, renal insufficiency, dyslipidemia, thrombotic disorder and metabolic syndrome.

Determining the presence of an unstable atherosclerotic plaque as defined above may also be useful as marker of effectiveness of a drug or a treatment, in particular in theranostics.

The term "theranostics" describes the use of diagnostic testing to diagnose the disease, choose the correct treatment regimen according to the results of diagnostic testing and/or monitor the individual response to therapy according to the results of diagnostic testing. Theranostic tests can be used to select individuals for treatments that are particularly likely to benefit them and unlikely to produce side-effects. They can also provide an early and objective indication of treatment efficacy in individual patients, so that (if necessary) the treatment can be altered with a minimum of delay.

The present invention also relates to a method, preferably an *in vitro* method, for evaluating the likelihood that an individual will benefit from a treatment with an agent to reduce the risk of cardiovascular disease, comprising the step of determining the presence of an unstable atherosclerotic plaque by the method of the invention, the presence of an unstable atherosclerotic plaque being indicative of the likelihood of a benefit from the treatment.

Treatments with an agent to reduce the risk of cardiovascular disease are well-known from the skilled person and include for example treatments with benazepril; benzthiazide; beta-blockers; bumetanide; captopril; chlorothiazide; chlorthalidone; clonidine; enalapril; fosinopril; furosemide; hydralazine; hydralazine and hydrochlorothiazide; hydralazine, hydrochlorothiazide and reserpine; hydrochlorothiazide; hydrochlorothiazide and triamterene; hydroflumethiazide; indapamide; methyclothiazide; methyldopa; metolazone; moexipril; perindopril erbumine; polythiazide; potassium chloride; quinapril; quinethazone; ramipril; torsemide; trandolapril; triamterene and trichlormethiazide.

### Kit

Is also described herein a kit for carrying out the methods defined above, said kit comprising means for detecting at least two biochemical markers selected from the group consisting of:
- alpha B crystallin,
- calponin-1,
- phosphatidylethanolamine-binding protein 1,
- aciculin,
- mature cathepsin K,
- osteoglycin,
- peroxiredoxin-2,
- dihydropyrimidinase-like 3,
- serum amyloid P component,
- serpin B9,
- serpin B9-granzyme B complex,
- pro-cathepsin D,
- thrombospondin-2,
- thrombospondin-1,
- pro-collagen C-endopeptidase enhancer 1,
- DJ-1 oncogene,
- tissue inhibitor of metalloproteinase,
- microfibril-associated protein-4,
- heat shock protein 27,
- IL-12,
- vascular endothelial growth factor,
- IL-8,
- IL-1ra,
- granulocyte colony-stimulating factor,
- growth-regulated alpha protein,
- IL-13,
- leukemia inhibitory factor,
- IL-1 alpha,
- IL-1 beta,
- interferon alpha-2, and
- stromal cell derived factor 1 alpha,
   as defined above.

Preferably, in the kit described herein, said at least two biochemical markers include at least one biochemical marker selected from the group consisting of calponin-1, alpha B crystallin, phosphatidylethanolamine-binding protein 1, aciculin, mature cathepsin K, dihydropyrimidinase-like 3, serum amyloid P component, serpin B9-granzyme B complex, pro-cathepsin D, thrombospondin-1, pro-collagen C-endopeptidase enhancer-1, IL-12, IL-1 ra, granulocyte colony-stimulating factor, leukemia inhibitory factor, IL-1 alpha, IL-1 beta, interferon alpha-2, stromal cell derived factor 1 alpha, microfibril-associated protein-4, DJ-1 oncogene and tissue inhibitor of metalloproteinase 1.

More preferably, said kit described herein comprises means for detecting a first biochemical marker and means for detecting a second biochemical marker, wherein said first biochemical maker is selected from the group consisting of IL-1ra, vascular endothelial growth factor, mature cathepsin K, peroxiredoxin-2, serum amyloid P component, IL-12, IL-8, granulocyte colony-stimulating factor, growth-regulated alpha protein, IL-13, leukemia inhibitory factor and IL-1 beta, and said second biochemical marker is selected from the group consisting of alpha B crystallin, calponin-1, phosphatidylethanolamine-binding protein 1, aciculin, osteoglycin, dihydropyrimidinase-like 3, serpin B9, serpin B9-granzyme B complex, pro-cathepsin D, thrombospondin-2, thrombospondin-1, pro-collagen C-endopeptidase enhancer-1, microfibril-associated protein-4, DJ-1 oncogene, IL-1 alpha, interferon alpha-2, stromal cell derived factor 1 alpha, tissue inhibitor of metalloproteinase 1 and heat shock protein 27.

As described herein, the expression "means for detecting a marker" refers in particular to ligands specific for said biochemical markers as defined in the paragraph *"Measurement of the presence and*/*or level of the biochemical markers".*

Preferably, the kit described herein further comprises instructions for using the means for detecting markers included in the kit as defined above to carry out the methods defined above.

"Instructions for use" typically include a tangible expression describing the technique to be employed in using the components of the kit and explaining the conditions to be used to carry out the methods defined above.

The invention will be further illustrated by the following figures and examples.

### Description of the figures

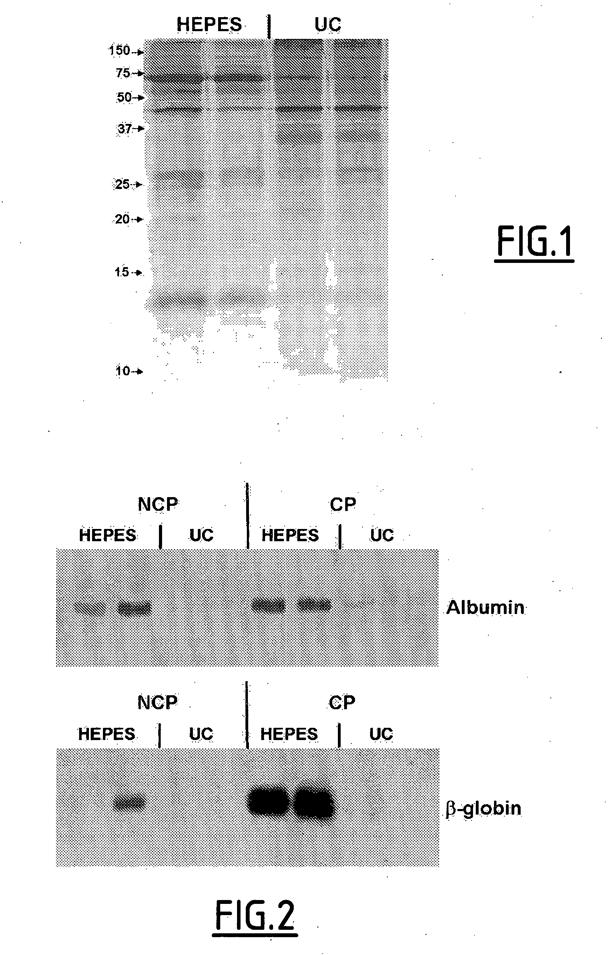

**Figure 1** shows the differential profiling obtained by SDS-PAGE and Sypro Ruby staining in two independent non complicated atherosclerotic carotid plaques after sequential protein extraction with HEPES buffer (HEPES) and Urea/CHAPS buffer (UC). 2 µg of protein extract were loaded by lane. The figures on the left of the blots correspond to the molecular weight ladder.
**Figure 2** shows the differential profiling obtained by Western Blot analysis in two independent non complicated atherosclerotic carotid plaques (NCP) and two independent complicated atherosclerotic carotid plaques (CP) after sequential protein extraction with HEPES buffer (HEPES) and Urea/CHAPS buffer (UC). The upper blot displays the results obtained using anti-HSA (albumin) antibodies, 0.5 µg of protein extract being loaded by lane. The lower blot displays the results obtained using anti-β globin antibodies, 1 µg of protein extract being loaded by lane.

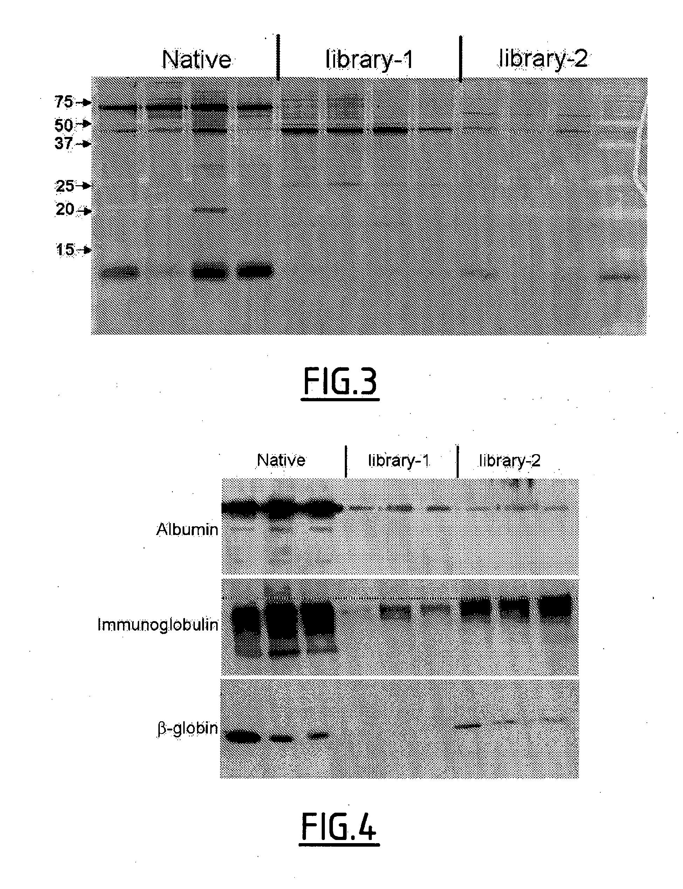

**Figure 3** shows the differential profiling obtained in native and peptides library beads treated carotid atherosclerotic plaques. SDS-PAGE analysis was performed on atherosclerotic plaques protein extracts before (Native = HEPES fraction) and after sequential bead treatment (Library-1 and Library-2 fractions). Proteins from four independent CP protein extracts and bead experiments were separated by SDS polyacrylamide gel and stained with Sypro Ruby. The figures on the left of the blots correspond to the molecular weight ladder.
**Figure 4** shows the results of a Western blot analysis displaying the reduction of high abundant proteins following bead treatment in atherosclerotic carotid plaques. Western blot analyses were performed with anti-HSA (albumin), anti-immunoglobulins and anti-β globin antibodies using CP protein extracts from native, Library-1 and Library-2 fractions (n=3 per group). For the blot with anti-HSA antibodies, 0.5 µg of proteins was loaded. For blots with anti-immunoglobulins and anti-β globin antibodies, 1 µg of proteins was loaded.

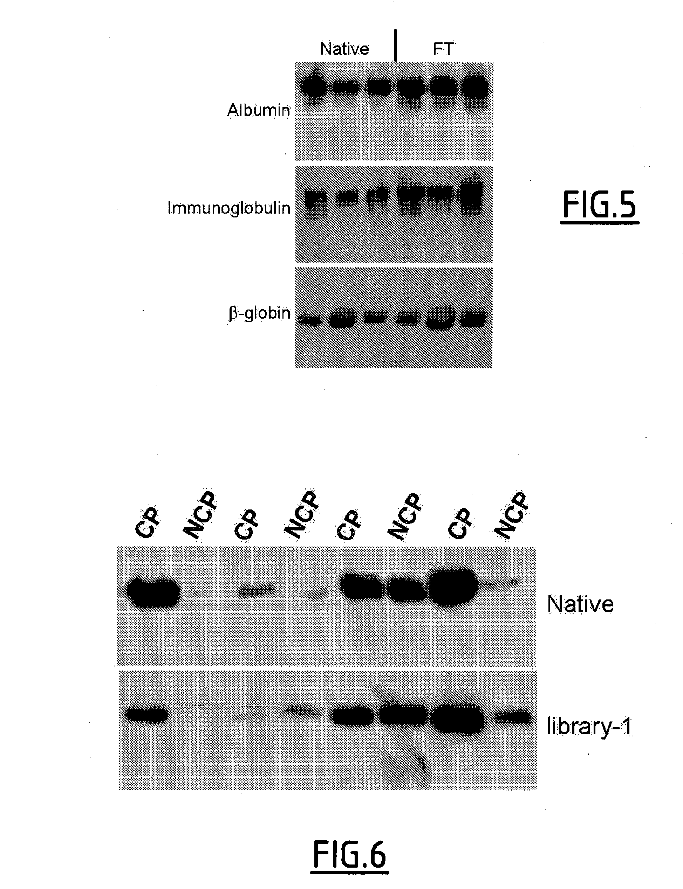

**Figure 5** shows the results of a Western blot analysis displaying the reduction of high abundant proteins following bead treatment in atherosclerotic carotid plaques. Western blot analyses were performed with anti-HSA (albumin), anti-immunoglobulins and ant-β globin antibodies using CP protein extracts from native and flow through (FT) fractions (n=3 per group). For the blot with anti-HSA antibodies, 0.5 µg of proteins was loaded. For blots with anti-immunoglobulins and anti-β globin antibodies, 1 µg of proteins was loaded.
**Figure 6** shows the results of a Western blot analysis displaying hemoglobin expression after peptide beads treatment in carotid plaques. The Western blot was carried out with anti-β globin antibodies using CP and NCP protein extracts from native and Library-1 fractions (n=4 per experimental group).

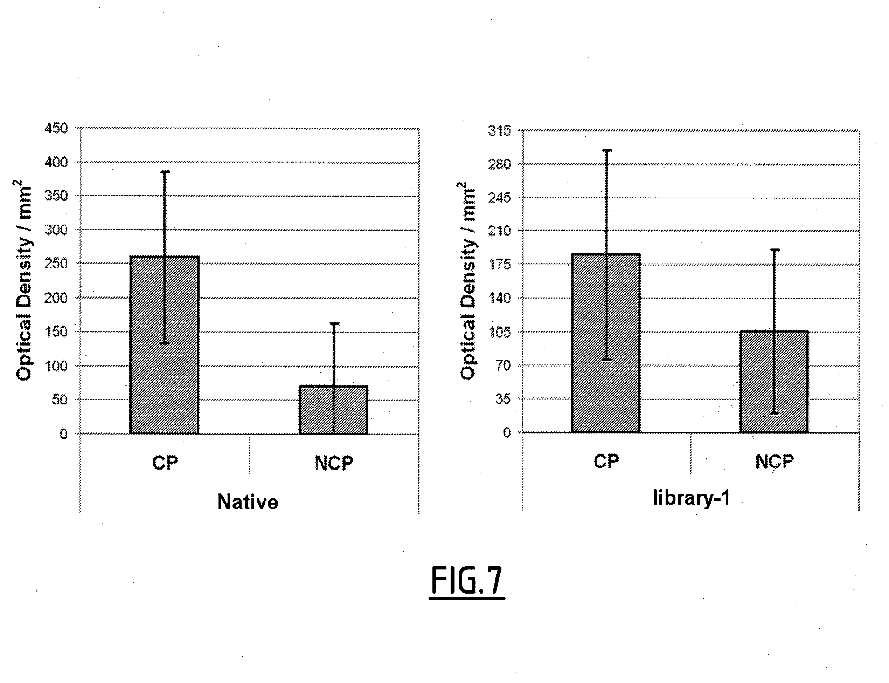

**Figure 7** shows histograms displaying the quantification of bands presented in Figure 6. The quantification of the bands was performed by measuring the optical density and revealed a higher amount of hemoglobin in CP as compared to NCP, the differential expression of hemoglobin being maintained after Library-1 treatment between both plaques.

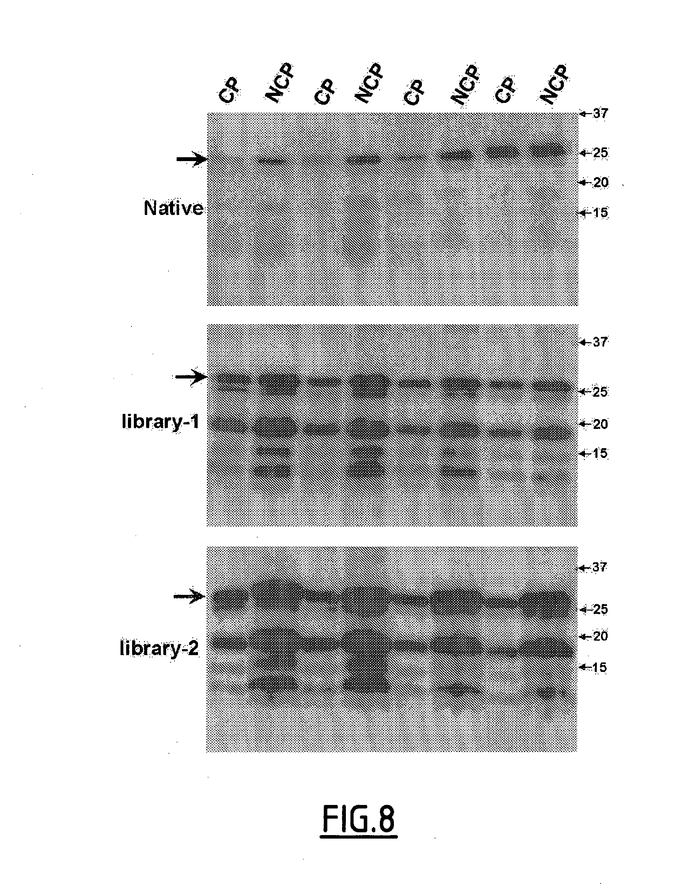

**Figure 8** shows the results of a Western blot analysis performed on CP and NCP protein extracts from native, Library-1 and Library-2 fractions using anti-HSP27 antibodies. For the three protein fractions, blots were realized in parallel in the same experiment. Protein quantities (5 µg) and chemiluminescence revelation times (20 min) were identical for the native, Library-1 and Library-2 fractions. Arrows show protein band expressions corresponding to HSP27 protein. The figures on the right of the blots correspond to the molecular weight ladder.

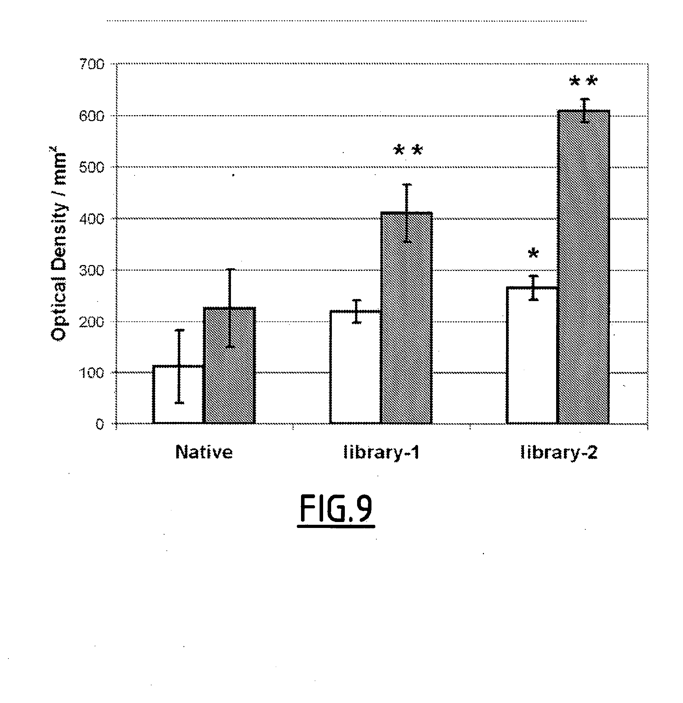

**Figure 9** shows histograms displaying the quantification of the bands corresponding to HSP27 protein and indicated by the arrows in Figure 8. The quantification of the bands was performed by measuring the optical density for Native, Library-1 and Library-2 fractions. Open and grey bars represent respectively CP and NCP samples. Asterisk (*) indicates p<0.05 when comparing native CP to Library-2 CP extracts. Double asterisk (**) indicates p<0.05 when comparing Library-2 NCP extracts to native NCP. For all blots, data are expressed as mean values ± SEM for four CP and NCP protein extracts.

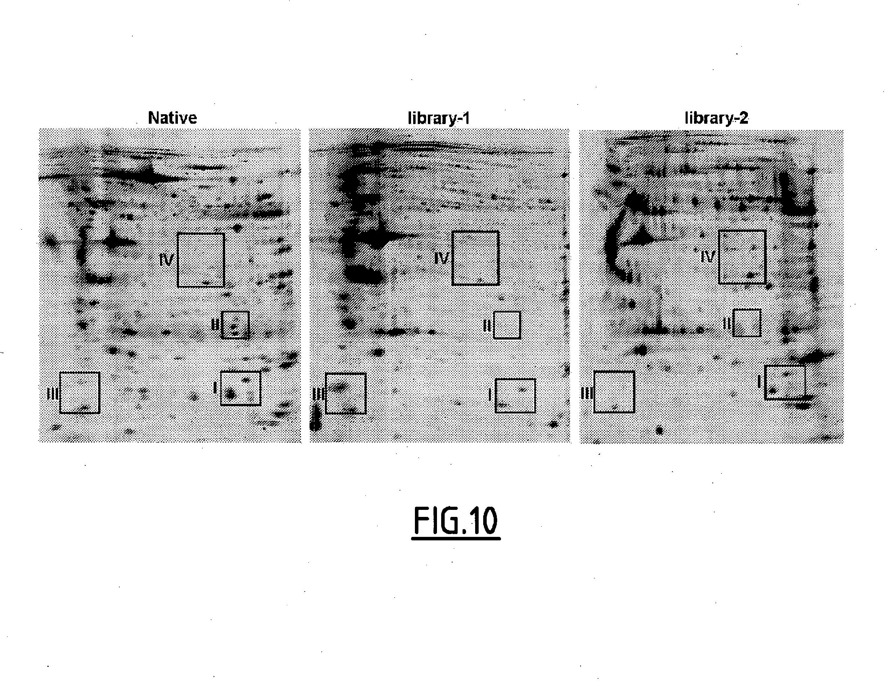

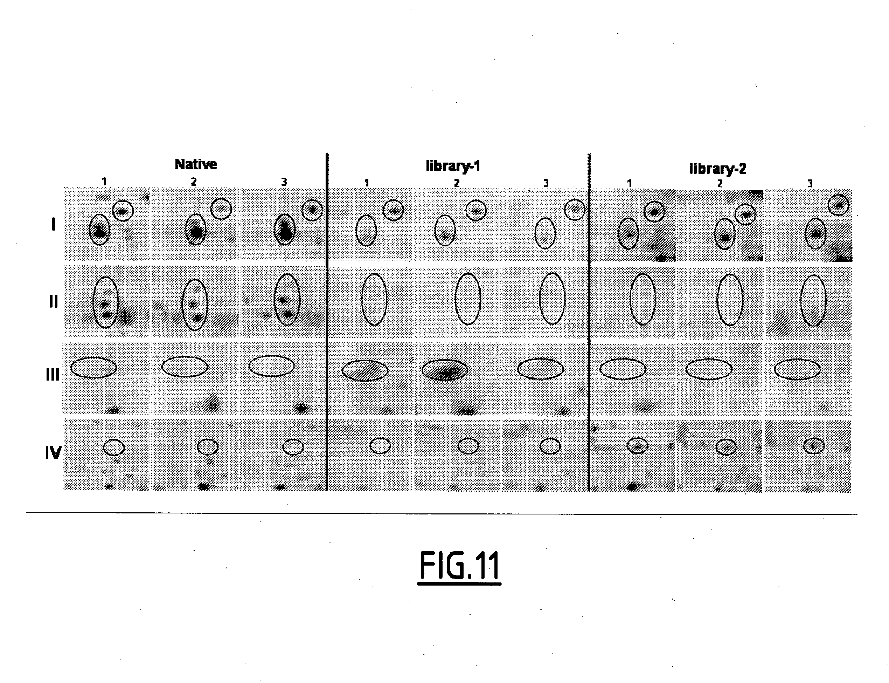

**Figure 10** shows the results of 2-D polyacrylamide gels analysis of native, Library-1 and Library-2 fractions for atherosclerotic plaque proteins (CP samples). Open squares (I, II, III and IV) indicate the 2-D gel areas which are enlarged in Figure 11.
**Figure 11** shows the enlargement of the four areas (I, II, III and IV), corresponding to protein spots, indicated in Figure 10 for the Native, Library-1 and Library-2 fractions. Each selected protein spot is shown for three independent CP samples (1, 2 and 3).

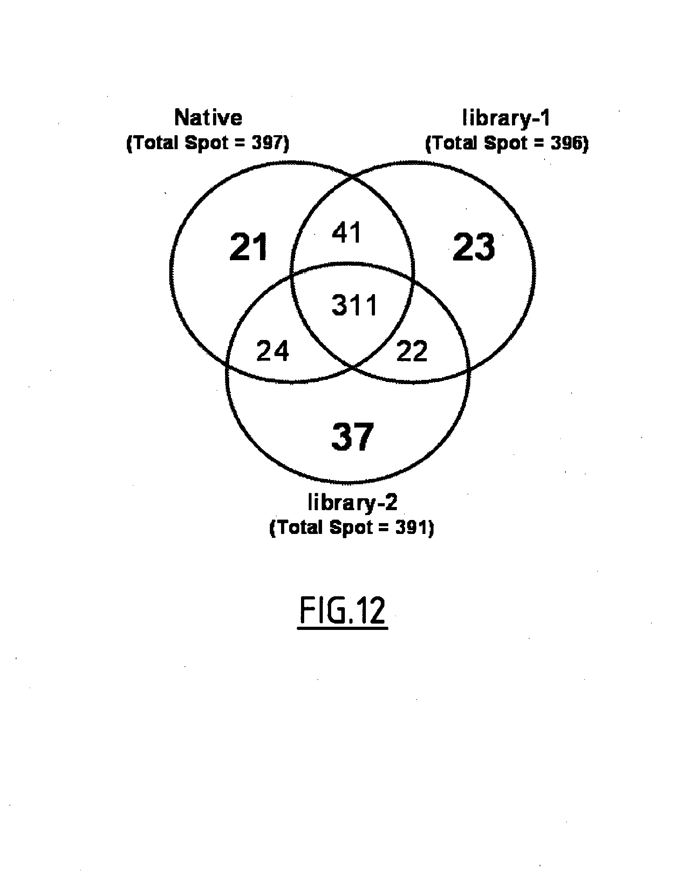

**Figure 12** shows the distribution of the total spot proteins detected with the 2-D gel analysis procedure, presented in Figures 10 and 11, for the comparison of the native (left upper circle), Library-1 (right upper circle) and Library-2 (middle lower circle) fractions. In bold is illustrated the spot number not common between fractions, with a series of new detected spots in Library-2 fraction (37 spots) and in Library-1 fraction (23 spots)

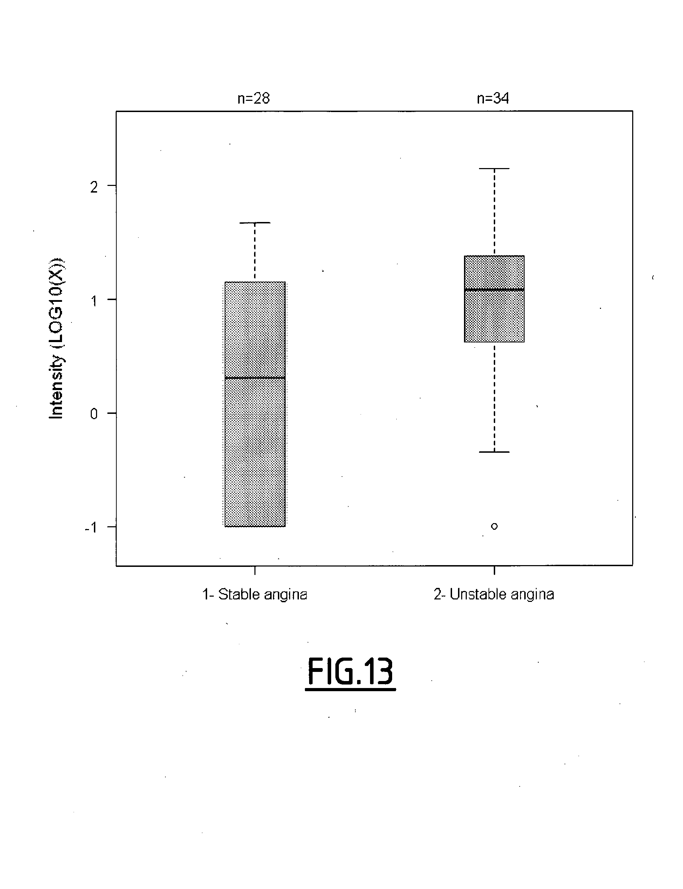

**Figure 13** shows the boxplot of circulating calponin-1 in stable angina (n=28) and unstable angina patients (n=34) described in Example 4. A logarithmic transformation was applied on the calponin-1 levels in plasmas. Using the Wilcoxon nonparametric test, a significant increase in plasma calponin-1 was obtained in unstable angina patients as compared to stable patients (p=0.009). Data are represented as median (horizontal bold line), interquartile range (Q3-Q1), minimun and maximun values. Circles are outliers.

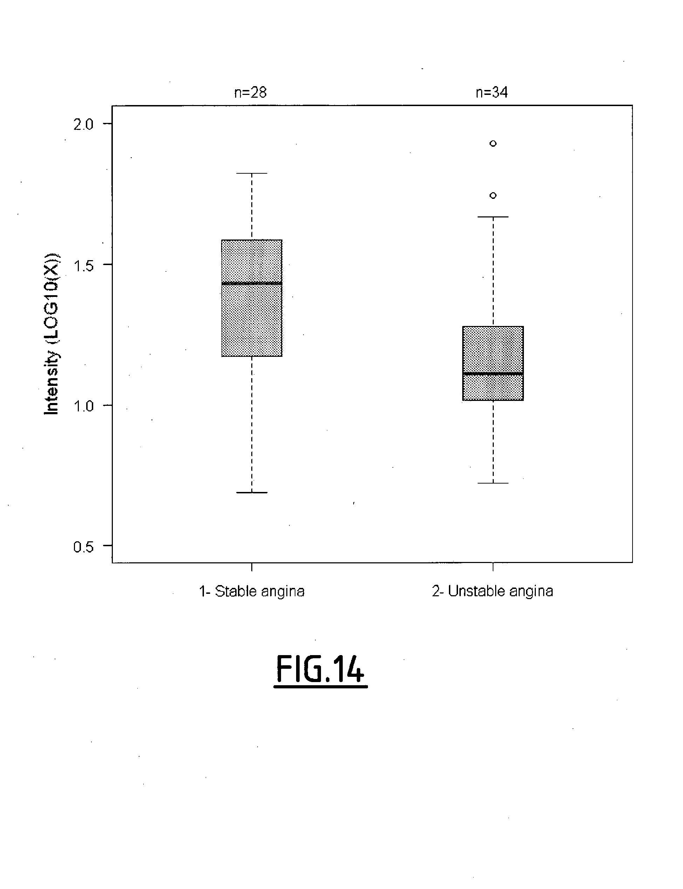

**Figure 14** shows the boxplot of circulating Interleukin-8 in stable angina (n=28) and unstable angina patients (n=34) described in Example 4. A logarithmic transformation was applied on the II-8 levels in plasmas. Using the Wilcoxon nonparametric test, a significant decrease in plasma II-8 was obtained in unstable angina patients as compared to stable patients (p=0.027). Data are represented as median (horizontal bold line), interquartile range (Q3-Q1), minimun and maximun values. Circles are outliers.

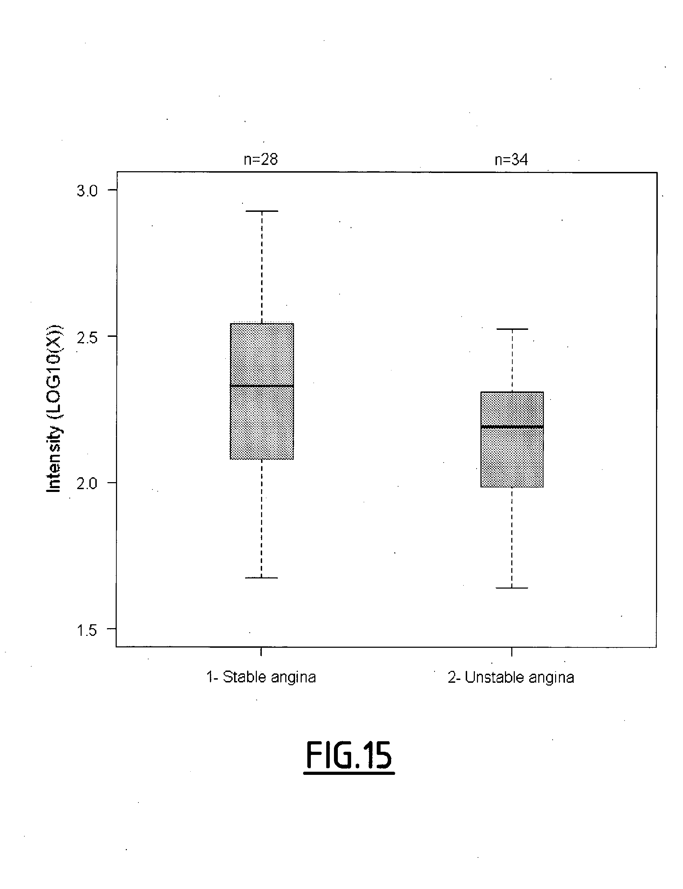

**Figure 15** shows the boxplot of circulating DJ-1 oncogene in stable angina (n=28) and unstable angina patients (n=34) described in Example 4, illustrating a decrease in plasma DJ-1 oncogene in unstable angina patients. A logarithmic transformation was applied on the DJ-1 oncogene levels in plasmas. Data are represented as median (horizontal bold line), interquartile range (Q3-Q1), minimun and maximun values.

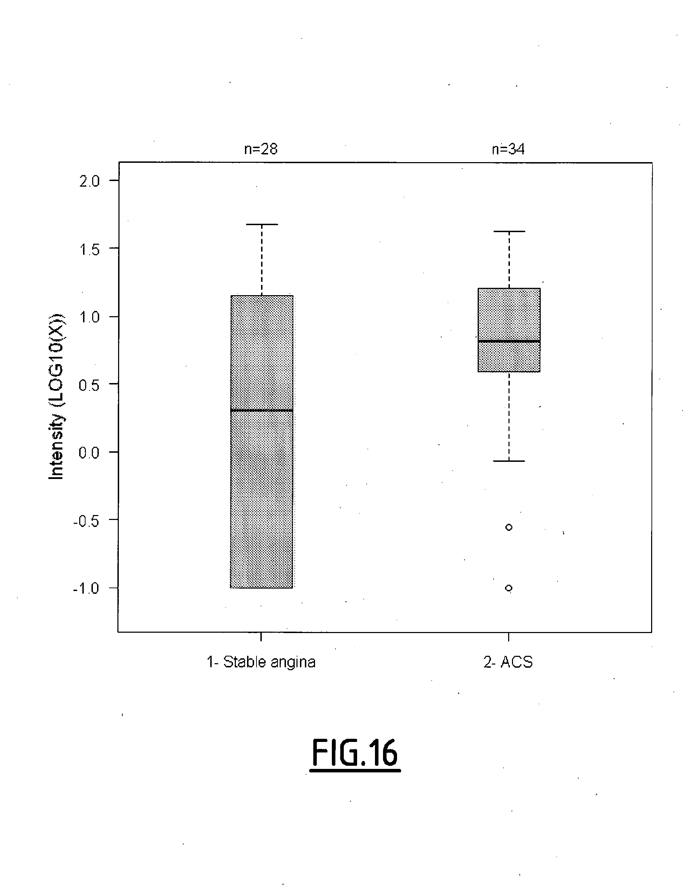

**Figure 16** shows the boxplot of circulating calponin-1 in stable angina (n=28) and ACS patients (n=34) described in Example 4. A logarithmic transformation was applied on the calponin-1 levels in plasmas. Using the Wilcoxon nonparametric test, a significant increase in plasma calponin-1 was obtained in ACS patients as compared to stable patients (p=0.021). Data are represented as median (horizontal bold line), interquartile range (Q3-Q1), minimun and maximun values. Circles are outliers.

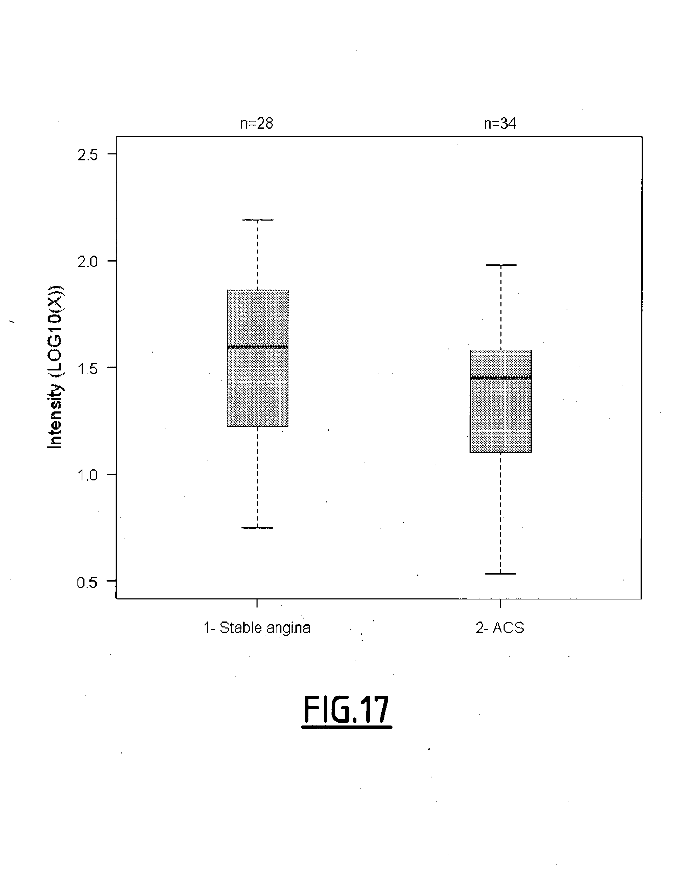

**Figure 17** shows the boxplot of circulating VEGF in stable angina (n=28) and ACS patients (n=34) described in Example 4. A logarithmic transformation was applied on the VEGF levels in plasmas. Using the Wilcoxon nonparametric test, a significant decrease in plasma VEGF was obtained in unstable angina patients as compared to ACS patients (p=0.032). Data are represented as median (horizontal bold line), interquartile range (Q3-Q1), minimun and maximun values.

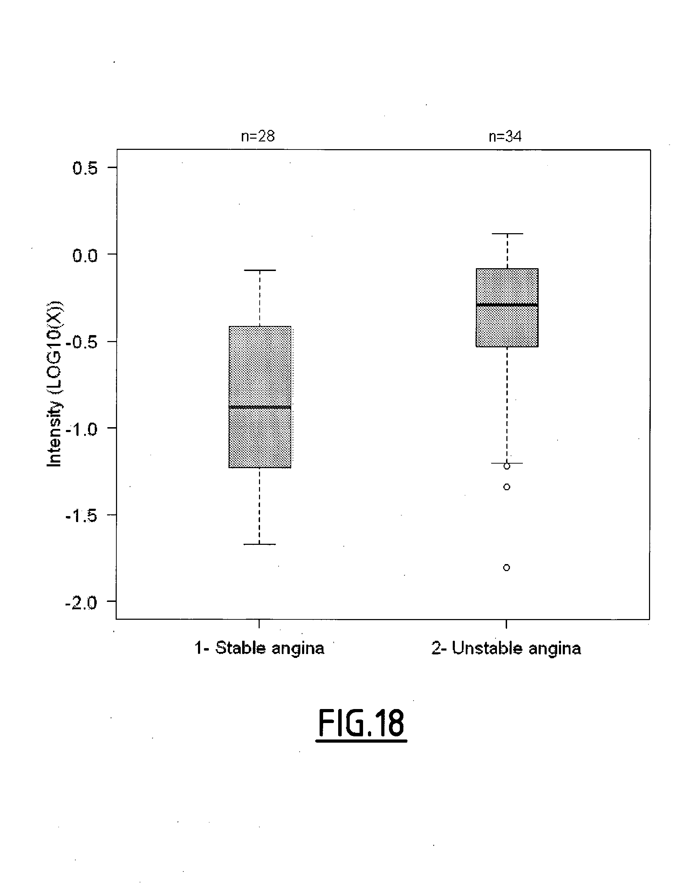

**Figure 18** shows the boxplot of the decision rule of calponin-1 combined to II-8 in stable angina (n=28) and unstable patients (n=34) described in Example 4. The new virtual marker (Z) from the multimarker equation (expressed in Log10) discriminated significantly stable and unstable patients (p=0.0004). Data are represented as median (horizontal bold line), interquartile range (Q3-Q1), minimun and maximun values. Circles are outliers.

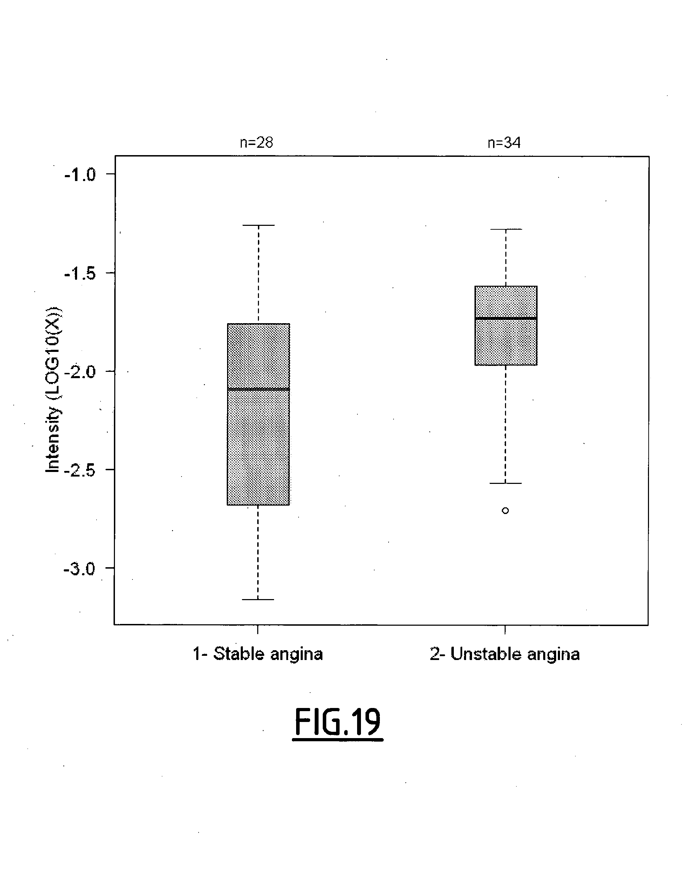

**Figure 19** shows the boxplot of the decision rule of calponin-1 combined to aciculin in stable angina (n=28) and unstable patients (n=34) described in Example 4. The new virtual marker (Z) from the multimarker equation (expressed in Log10) discriminated significantly stable and unstable patients (p=0.0017). Data are represented as median (horizontal bold line), interquartile range (Q3-Q1), minimun and maximun values. Circles are outliers.

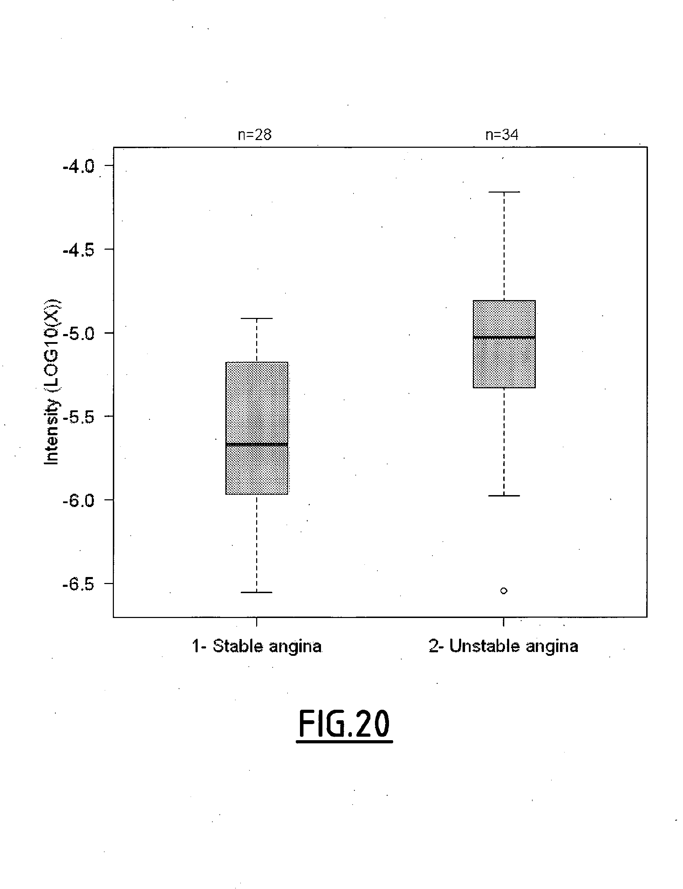

**Figure 20** shows the boxplot of the decision rule of calponin-1 combined to II-8 and MFAP-4 in stable angina (n=28) and unstable patients (n=34) described in Example 4.
The new virtual marker (Z) from the multimarker equation, expressed in Log10, had a good diagnostic accuracy (p<0.0002). Data are represented as median (horizontal bold line), interquartile range (Q3-Q1), minimun and maximun values. Circles are outliers.

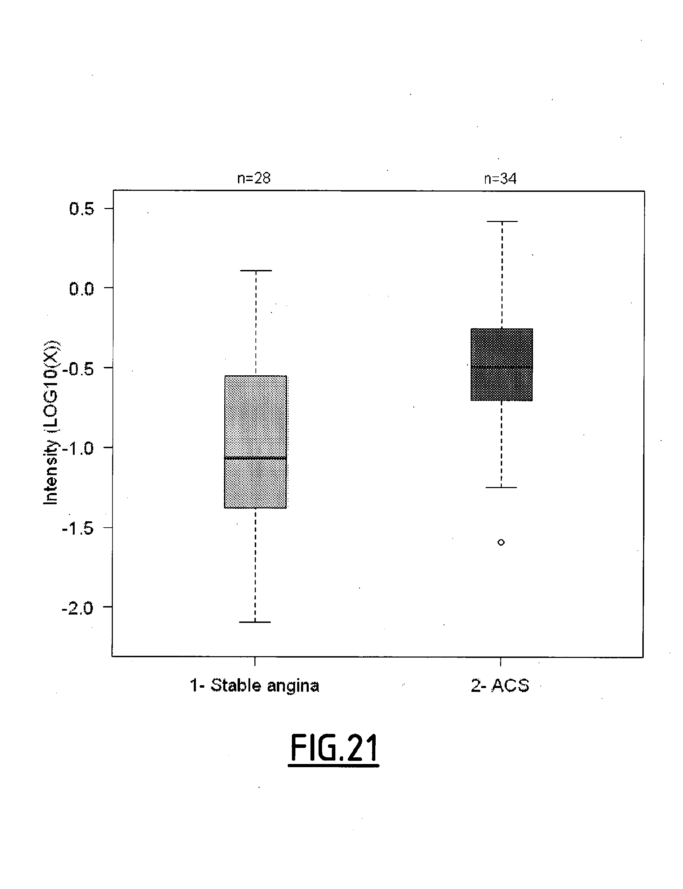

**Figure 21** shows the boxplot of the decision rule of calponin-1 combined to VEGF in stable angina (n=28) and ACS patients (n=34) described in Example 4. The new virtual marker (Z) from the multimarker equation (expressed in Log10) dissociated significantly ACS patients to stable individuals (p = 0.0007). Data are represented as median (horizontal bold line), interquartile range (Q3-Q1), minimun and maximun values. Circles are outliers.

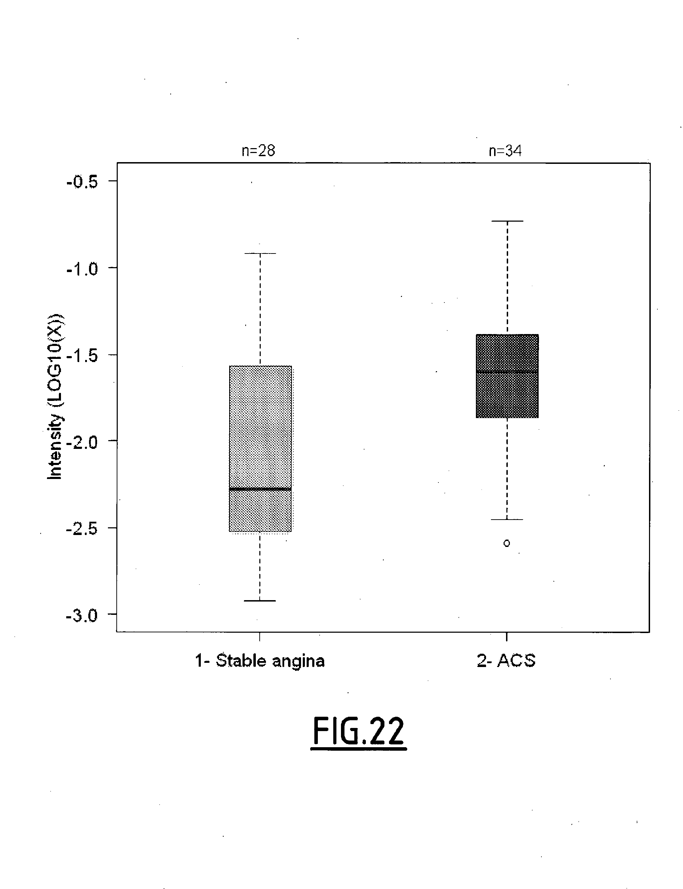

**Figure 22** shows the boxplot of the decision rule of calponin-1 combined to PEBP-1 in stable angina (n=28) and ACS patients (n=34) described in Example 4. The new virtual marker (Z, expressed in Log10) from the multimarker equation illustrated a robust diagnostic accuracy between stable and ACS patients (p=0.0018). Data are represented as median (horizontal bold line), interquartile range (Q3-Q1), minimun and maximun values. Circles are outliers.

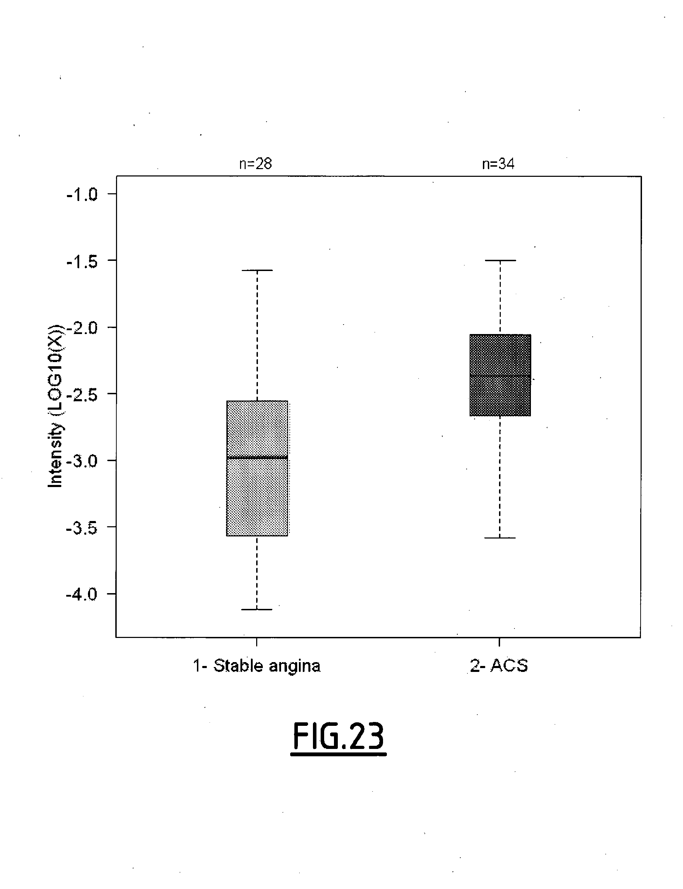

**Figure 23** shows the boxplot of the decision rule of calponin-1 combined to VEGF and PEBP-1 in stable angina (n=28) and ACS patients (n=34) described in Example 4. The new virtual marker (Z) from the multimarker equation was illustrated (expressed in Log10) with a significant discrimination (p=0.0001). Data are represented as median (horizontal bold line), interquartile range (Q3-Q1), minimun and maximun values.

### Examples

### Example 1

In this example, the inventors evaluated the impact of a ligand library bead treatment on protein diversity provided from carotid atherosclerotic plaques, and on differential protein expression, by studying proteins well known to be differentially expressed, such as heat shock protein 27 and hemoglobin, in non complicated (stable, NCP) and complicated (unstable, CP) plaques.

The inventors developed a new protocol to allow low abundant protein amplification from complex vascular tissues extracts, with limited impact on differential protein expression. The 2D electrophoresis reproducibility and compatibility with hexapeptide library treated plaque samples were also evaluated to reinforce the potential interest of pre-analytical treatment in biomarker discovery. In the present example, the inventors reported a new protein profiling and a differential expression amplification following ligand library bead treatment of carotid atherosclerotic plaques.

### Materials and Methods

### Atherosclerotic plaque samples

Human carotid atherosclerotic plaques were obtained from patients undergoing endarterectomy from Vascular surgery Department of Arnaud de Villeneuve Hospital (CHU Montpellier, France). The study was approved by Villeneuve Hospital Institutional board in accordance to institutional guidelines of Ethics Committee. Written informed consent was obtained for all included patients. Patients (females and males with a mean age of 74 years, range 42-96 years) presenting internal carotid artery stenosis greater than 70% were included. Following carotid atherosclerotic plaque endarterectomy, surgical specimens were extensively rinsed in ice-cold NaCl 0.9% solution. According to Stary's classification, previous studies on carotid plaques (Leclercq et al. (2007) Atherosclerosis 191:1-10; Lepedda *et al.* (2009)) and macroscopical observation, the inventors separated plaques having thick fibrous cap and low stenosis (named non-complicated plaque; NCP) from plaques with thin fibrous cap and necrotic core (stenosis) associated to hemorrhagic/thrombotic areas (named complicated plaque, CP). Dissected vascular tissues were rapidly snap frozen in liquid nitrogen and stored at -80°C until assessment.

### Soluble-oriented protein plaque extraction

To favour soluble-enriched species to be extracted in a buffer compatible with protein binding on ligand library beads, carotid plaques were subfractionated with sequential extraction: HEPES and urea CHAPS buffer as previously described (Kane et al. (2007) Methods Mol Biol. 357:87-90.). Briefly, carotid plaques were homogenized with an Ultra Turax™ homogenizer (in water bath in ice) by adding cold HEPES buffer, containing 25 mM HEPES pH 7.4, anti-proteases (Roche Diagnostics, Meylan, France) and anti-phosphatases (Sigma-Aldrich, St Louis, MO) cocktails (*i.e*., 0.15 g of tissue for 1 ml of HEPES buffer). Homogenates were incubated for 45 min at 4°C under agitation and then centrifuged at 18,000 g for 15 min at 4°C. Supernatants containing HEPES soluble-protein fractions were stored on ice and pellets were successively washed 3 times with cold HEPES. HEPES protein fractions were aliquoted and stored at - 80°C. Finally, insoluble tissue pellets were solubilized in a lysis buffer containing 8 M urea and 4% CHAPS (0.45 mg of tissue for 1 ml of lysis buffer) and incubated for 45 min at 4°C under agitation. After centrifugation, supernatants containing Urea/CHAPS protein fractions were collected and stored at -80°C.

### Ligand library beads for tissue protein samples

The combinatorial hexapeptide Library-1 (ProteoMiner™, Bio-Rad Laboratories, Hercules, CA) previously used for biological fluids, such as plasma (Sihlbom et al. (2008) J Proteome Res. 7(9):4191-8), serum (Sennels et al. (2007) J Proteome Res. 6(10):4055-62), cerebrospinal fluid (Mouton-Barbosa et al. (2010) Mol Cell Proteomics.) and urine (Candiano et al. (2009) Electrophoresis. 30(14):2405-11), as well as in cell extracts, such as erythrocytes (Roux-Dalvai et al. (2008) Mol Cell Proteomics. 7(11):2254-69), human platelets (Guerrier et al. (2007) J Proteome Res. 6(11):4290-303), was adapted to a heterogeneous vascular tissue: human atherosclerotic plaques with soluble-enriched features. In contrast, the carboxylated part hexapeptide Library (Library-2) was not yet used in biological fluids; a complete adaptation was performed on human atherosclerotic plaques with soluble-enriched features. Briefly, 2 mg of plaque proteins were incubated with 20 µl of phosphate buffer saline-equilibrated Library-1, into a spin-column, under gentle agitation for 3 hours at 4°C. After centrifugation (1020 g for 2 min), the flow-through fractions were incubated with 40 µl of phosphate buffer saline-equilibrated Library-2, into a same spin-column under agitation. In parallel, after washing six times with 1 ml of phosphate buffer saline pH 7.4, proteins bound to Library-1 were eluted three times with 20 µl of elution buffer pH 3.4 (8 M Urea, 2% CHAPS and 50 mM acetic acid) for maximal recovery. After 3 hours of incubation at 4°C and centrifugation (1020 g for 2 min), the Library-2 were also washed 6 times with 1 ml of phosphate buffer saline at pH 7.4. Finally, plaque proteins bound to Library-2 were eluted three times with 40 µl of elution buffer pH 3.4.

### 2D electrophoresis analysis

Protein 2D electrophoresis separations of HEPES fractions were performed in the first dimension by isoelectric focusing (IEF), using ReadyStrips (pH 3-10 non linear, 7 cm long; Bio-Rad Laboratories, Hercules, CA). The ReadyStrips were rehydrated overnight at room temperature in 125 µl of sample buffer (7 M urea, 2 M thiourea, 4% CHAPS, 100 mM dithiothreitol, 1:100 volume of Ampholyte pH 3-10, and a trace of bromophenol blue) containing 20 µg of protein. The IEF was performed using a PROTEAN IEF Cell system (Bio-Rad Laboratories) at 20°C with the following voltage gradient: 1 min at 200 V, 1 min at 300 V, 300-1000 V over 30 min, 1000-2000 V over 30 min and then 2000 V until 7.2 kVh was reached. After IEF, proteins were reduced for 10 min in equilibration buffer (50 mM Tris-HCl pH 8.8 containing 6 M urea, 30% glycerol and 2% SDS, 100 mM DTT and trace of bromophenol blue) and alkylated for 10 min in equilibration buffer with iodoacetamide (25 mg/ml) but without DTT. Proteins were separated in the 2^{nd} dimension by SDS-PAGE using 11% polyacrylamide gels. After protein fixation, 2D-gels were stained with Sypro Ruby (Bio-Rad Laboratories) and scanned with a Typhoon 9400 Scanner (Amersham Biosciences, GE Healthcare Life Sciences, Piscataway, NJ). 2D-gel images from native, Library-1 and Library-2 fractions were analyzed together with Progenesis SameSpots, V2.0 (Nonlinear USA Inc., Durham, NC). After gel alignments and protein spot detection and verification, ten new spots were created to determine the mean background values (percentage of normalized volumes); the mean background values plus 2 standard deviations (SD) were then calculated onto each gel. The values of percentage of normalized volumes obtained for each protein spots lower than the mean background values obtained plus 2 SD values were not considered as protein spots. For comparison, the total number of spots for each protein fraction was calculated and the presence or absence of protein spots in each fraction was also determined.

### Western-blot

Proteins were separated by SDS-PAGE and transferred onto a nitrocellulose membrane (Amersham Biosciences, GE Healthcare Life Sciences, Piscataway, NJ). Membranes were blocked with phosphate buffer saline containing 5 % of non-fat dry milk overnight at 4°C. Then, membranes were probed with either a mouse monoclonal anti-HSP-27 (SantaCruz Biotechnologie INC, Santa Cruz, CA), or a mouse monoclonal anti-HSA (Sigma-Aldrich, St Louis, MO), or a mouse monoclonal anti-β globin (kindly provided by B. Jardin from UMR CNRS-Bio-Rad 3145, Montpellier) or a goat polyclonal anti-polyvalent immunoglobulins (IgA, IgG, IgM) conjugated with Horseradish Peroxidase (HRP) (Sigma-Aldrich, St Louis, MO), for 2 h at room temperature in phosphate buffer saline containing 0.1 % Tween 20 (PBS-T) and 2% of non-fat dry milk. Membranes were washed with PBS-T, three times for 10 min. For HSP27, HSA and β globin, membranes were probed with an anti-mouse HRP-conjugated secondary antibody (Jackson Immunoresearch Laboratories INC, West Grove, PA) for 1 h at room temperature, in PBS-T containing 2% of non-fat dry milk. Protein detection was performed using ECL reagents (GE Healthcare Life Sciences, Piscataway, NJ) followed by exposure to autoradiography films (Kodak). Densitometry analyses were performed with Quantity-One Software (Bio-Rad Laboratories).

### Protein assays

Total protein contents from HEPES, Urea/CHAPS, Library-1 and Library-2 protein fractions were determined with BC assay according to the manufacturer's instructions (Interchim, Montlugon, France) and/or modified Bradford method. Protein profiles for each fraction were analysed with SDS-PAGE followed by Sypro Ruby staining (Bio-Rad Laboratories). Gels were scanned with Typhoon 9400 Scanner in a standardized manner. Hemoglobin content was determined by a colorimetric-enzymatic assay described by Montaño *et al.* (1999) (Montaño et al. (1999) J Immunol Methods 222:73-82). Briefly, plaque protein extracts (20 µg) were incubated with 0.2 M Tris-HCl, pH 7.2 buffer containing 0.1% (w/v) of 2-7 diaminofluorene (Sigma Aldrich, St Louis, MO). After 20 min of incubation at room temperature, the optical density was determined at 620 nm with an ELISA reader. Hemoglobin from bovine erythrocytes was used as standard.

### Statistical analysis

Results were expressed as means ± SEM. Student t-test was used to define difference between data sets from experimental groups. A p value ≤ 0.05 was considered as statistically significant.

### Results and discussion

According to its pathological stage, atherosclerotic plaque is a heterogeneous tissue, composed of circulating and tissular cells, extracellular lipid pools, extracellular matrix, intra-plaque hemorrhage and/or calcified nodule. Its proteomic analysis is complicated mainly due to the presence of high abundant species and lipids/calcified nodule. To favor soluble-enriched extract to better detect circulating proteins, the inventors applied soluble-oriented extraction compatible to 1- and 2-dimensional gel electrophoresis in non complicated and complicated carotid atherosclerotic plaques. Effective plaque extract fractionation was obtained when applying sequential extraction with HEPES and Urea/CHAPS buffers. Using sequential extraction, the inventors generated two different protein profiles as illustrated by 1 D SDS-PAGE analysis (**Figures 1 and 2**), with higher protein recovery amount in soluble HEPES fraction as compared to insoluble Urea/CHAPS fractions (73.60 ± 1.08% and 26.40 ± 1.08% for HEPES and Urea/CHAPS fractions respectively, **Table 1**).

**Table 1: Protein quantity recovery after sequential extraction with HEPES and Urea/CHAPS (UC) buffers**

| | **Total protein quantity (mg/g of tissue)** | | **Total protein quantity (%)** | |
|---|---|---|---|---|
| | HEPES | UC | HEPES | UC |
| **CP** | 6.54±0.96 | 2.34±0.21 | 73.60±1.08 | 26.40±1.08 |
| **NCP** | 7.25±1.38 | 2.33±0.14 | 75.32±4.66 | 24.68±4.66 |

Values are expressed as mean ± SEM. Total protein quantity is expressed either in mg/g of tissue or % of total protein. UC indicates extraction conditions with Urea/CHAPS buffer, CP complicated or unstable plaques, NCP non complicated or stable plaques.

Blot analyses reported that HEPES extraction had improved detection of soluble protein as albumin and β-globin in non complicated and complicated plaques as compared to Urea/CHAPS extraction, suggesting that HEPES extraction is more accurate to concentrate soluble proteins in vascular tissue. In accordance to these data, a previous study reported successful sub-fractionation of heart tissue thanks to HEPES buffer allowing separation of cytoplasmic-enriched extract to myofilament-enriched extracts (Kane et al. (2007) Methods Mol Biol. 357:87-90.). Ligand library beads have been developed for biological samples with large protein dynamic range (Boschetti and Righetti (2008) J Proteomics 71:255-264) to facilitate access to deep proteome for proteomic studies. Intra-plaque hemorrhage (Type VI in Stary's classification) is a complicated feature of unstable or complicated plaques (Virmani et al. (2005) Arterioscler Thromb Vasc Biol. 25(10):2054-61); Leclercq et al. (2007) J Leukoc Biol. 82(6):1420-9), suggesting a large protein dynamic range in atherosclerotic plaques, but nevertheless much lower than in plasma. After contacting 2 mg of protein extracts with peptides library beads, protein recovery was comparable between non complicated and complicated plaques in E-bead elution fraction (117.8 ± 13 versus 114.7 ± 17 µg proteins for complicated and non complicated fractions respectively, **Table 2).** The second sequential peptides library (Library-2) led to reduced protein recovery in both extracts **(Table 2).**

**Table 2: Protein quantity recovery after sequential equalization of 2 mg of proteins**

| | **Protein quantity (µg)** | | **CV (%)** | |
|---|---|---|---|---|
| | Library-1 | Library-2 | Library-1 | Library-2 |
| **CP** | 117.82±13.10 | 66.98±5.31 | 11.12 | 7.93 |
| **NCP** | 114.75±17.18 | 73.21±14.73 | 14.98 | 20.12 |

Values are expressed as mean ± SEM. Protein quantity is expressed in µg of tissue. CV indicates coefficient of variation, CP complicated or unstable plaques, NCP non complicated or stable plaques.

According to these numbers and corresponding CV % values, the peptide library beads process on atherosclerotic plaque was reproducible in both Library-1 and Library-2 elution fractions **(Table 2).** Using beads in human plasma, protein recovery was 2 to 3-fold lower than carotid plaques (Sihlbom *et al.* (2008)), probably because of the higher dynamic range of this protein in plasma.

To validate the reduced protein dynamic range in atherosclerotic plaques, the inventors analyzed by 1 D-gel native electrophoresis, Library-1 and Library-2 elution fractions **(****Figure 3****).** They clearly showed, as example, a reduction of highly abundant proteins (13 and 70 kDa bands) in native extracts and the appearance of others bands in Library-1 and Library-2 elution fractions (25 and 60 kDa bands). Previous protein profiling revealed that albumin, transferrin, hemoglobin and immunoglobulin were the most abundant proteins in atherosclerotic plaque extracts, representing around 70% of total protein content (Lepedda *et al.* (2009)). To evaluate the specific dynamic range reduction, the inventors performed immunoblot analyses in native condition of Library-1 and Library-2 fractions, using antibodies targeting albumin, immunoglobulin and β-globin proteins. They confirmed the high abundance of those proteins in native atherosclerotic plaques as illustrated in **Figures 4** **and** **5****,** profile comparable to flow through fraction. They reported the significant expression lowering in peptides library beads extracts for three abundant proteins. The lowered detection in bead extracts was, however, protein and beads specific, resulting from different hexapeptides affinity. For hemoglobin protein, a weak detection was reported in Library-1 fractions as compared to native fractions **(****Figures 4** **and** **5** lower panel).

To ensure that the amount of hemoglobin per sample (biological variability) was not altered by peptide beads, the inventors evaluated the hemoglobin expression in a series of samples (complicated and non complicated extracts, **Figures 6** **and** **7****).** The obtained results support that hemoglobin profile between samples (complicated and non complicated) was conserved as shown by similar expression pattern between native and Library-1 extracts **(****Figures 6** **and** **7****).** Peptides library beads-treated plaques were characterized by similar hemoglobin pattern, with higher amount of β-globin in complicated hemorrhagic plaques, supporting the unaltered differential expression after peptides library-beads processing. The inventors demonstrated that the dynamic range in protein concentration was successfully reduced following peptides library beads treatment of atherosclerotic plaques, while, unexpectedly, the differential expression of the high abundant hemoglobin was relatively conserved.

One limitation of sample depletion for biomarker discovery is the removal of proteins of interest and/or the impact on differential profiling between control and pathologic samples. To evaluate the impact of peptides library beads treatment on protein expression in atherosclerotic plaques, the inventors studied the expression of a previously reported biomarker, heat-shock protein 27 (HSP27) expressed in endothelial cells and vascular smooth muscle cells. Numerous previous studies reported the reduced expression of HSP-27 in complicated/unstable carotid plaques (Park *et al.* (2006); Lepedda *et al.* (2008)) as well as in carotid plaques incubated in conditioned media *(*Martin-Ventura et al. (2004) Circulation 110:2216-2219; Martin-Ventura et al. (2006) Arterioscler Thromb Vasc Biol 26:1337-1343). In the present example, native extracts of atherosclerotic plaques confirmed the significant decrease of HSP27 expression in complicated/stable plaques as compared to non complicated plaques **(****Figure 8****).** Based on immunoblot analyses, double peptides library beads treatment amplified HSP27 protein signal and maintained the differential expression between complicated and non complicated atherosclerotic plaques. Interestingly, the differential expression between complicated and non complicated plaques was significantly higher in beads treated extracts as compared to non-treated plaques **(****Figure 9****,** p<0.05). Based on HSP27 expression, it appeared that ligand library could amplify low abundant proteins and differential expression.

Further profiling differences in native and beads treated fractions were reported in 2DE analysis as illustrated in **Figures 10-12****.** When quantifying the spot number in native, Library-1 and Library-2 extracts using SameSpot software, the inventors obtained comparable total spot number (around 390 spots), with detected spots specific to native (21 spots), Library-1 (23 spots) and Library-2 (37 spots) fractions. A large amount of spots, however, were common (311 spots) between native and beads treated extracts. By reducing large protein dynamic range, the inventors thus highlighted specie diversity in atherosclerotic plaques.

The inventors thus demonstrated that soluble-enriched extraction combined to hexapeptides library beads potentialised specie diversity detection in carotid atherosclerotic plaques. Beads extracts seemed to have a lower protein dynamic range as compared to native complicated and non complicated plaques. When focusing on protein expression between pathological and control lesions, the inventors reported that peptides library beads treatment did not alter differential expression of low abundant proteins, as HSP27 and unexpectedly the more abundant hemoglobin. Consequently, the inventors showed that hexapeptides library bead technology, by reducing protein dynamic range, was relevant for new marker discovery of plaque vulnerability.

### Example 2

This example describes the identification, by the inventors, of the biochemical markers defined in the present application.

### Material and methods

Carotid atherosclerotic plaques obtained by endarterectomy were separated according to histological criteria in non-complicated plaques (NCP; fibrous, stable plaque) and complicated plaques (CP; instable plaques with a necrotic core and a thin fibrous layer).

Protein extracts of these two types of plaques were prepared as explained in Example 1, and were subjected to a depletion treatment as explained in Example 1, using ProteoMiner^{®} beads.

Proteins were then separated on 2D-PAGE gels and, after detection of the spots revealing a differential protein expression between NCP and CP, the proteins were analyzed by mass spectrometry. The analysis was then confirmed by Western blot using antibodies specific of the identified proteins. In some cases, the bands corresponding to the marker were quantified by measuring the optical density.

### Results

The following proteins were identified by the inventors as being differentially expressed in non-complicated and complicated plaques: calponin-1, phosphatidylethanolamine-binding protein 1, alpha B crystallin, aciculin, cathepsin K, osteoglycin, heat shock protein 27, peroxiredoxin-2, dihydropyrimidinase-like 3, serum amyloid P component, serpin B9, granzyme B/serpin B9 complex, pro-cathepsin D, thrombospondin-2, thrombospondin-1, pro-collagen C-endopeptidase enhancer-1, microfibril-associated protein-4, DJ-1 oncogene and tissue inhibitor of metalloproteinase 1.

The Western blot confirmation of some of these biochemical markers is shown herein below:

### Aciculin

The antibodies used to detect aciculin were mouse monoclonal antibodies, clone 14F8 (Santa Cruz).

**Table 3** displays the level of aciculin in complicated plaques and non-complicated plaques. The level of aciculin is represented by the measure of the optical density of the bands corresponding to aciculin in the blots.

**Table 3: Comparison of the level of expression of aciculin**

| | Mean | CV% mean | Median | CV% median | Fold mean | Fold median | T-test |
|---|---|---|---|---|---|---|---|
| **CP** | 47.34 | 49.87 | 43.39 | 54.41 | 0.18 | 0.16 | 0.00004 |
| **NCP** | 268.13 | 15.80 | 271.75 | 15.59 | | | |

### Granzyme B/Serpin B9 complex

The antibodies used to detect serpin B9 either free or complexed with Granzyme B were mouse monoclonal antibodies, clone 6D700 (Santa Cruz).

**Table 4** displays the level of Granzyme B/Serpin B9 complex in complicated plaques and non-complicated plaques. The level of Granzyme B/Serpin B9 complex is represented by the measure of the optical density of the bands corresponding to Granzyme B/Serpin B9 complex in the blots.

**Table 4: Comparison of the level of expression of Granzyme B/Serpin B9 complex**

| | Mean | CV% mean | Median | CV% median | Fold mean | Fold median | T-test |
|---|---|---|---|---|---|---|---|
| **CP** | 4.82 | 56.27 | 4.31 | 63.00 | 0.12 | 0.10 | 0.00952 |
| **NCP** | 39.89 | 47.70 | 42.75 | 44.50 | | | |

### Calponin 1

The antibodies used to detect calponin-1 were mouse monoclonal antibodies, clone 0.N.39 (Santa Cruz).

**Table 5** displays the level of calponin 1 in complicated plaques and non-complicated plaques. The level of calponin 1 is represented by the measure of the optical density of the bands corresponding to calponin 1 in the blots.

**Table 5: Comparison of the level of expression of calponin 1**

| | Mean | CV% mean | Median | CV% median | Fold mean | Fold median | T-test |
|---|---|---|---|---|---|---|---|
| **CP** | 12.32 | 78.65 | 8.96 | 108.16 | 0.08 | 0.06 | 0.02790 |
| **NCP** | 163.54 | 41.93 | 162.34 | 42.24 | | | |

### Alpha B crystallin

The antibodies used to detect alpha B crystallin were mouse monoclonal antibodies, clone 2E8 (Santa Cruz).

**Table 6** displays the level of alpha B crystallin in complicated plaques and non-complicated plaques. The level of alpha B crystallin is represented by the measure of the optical density of the bands corresponding to alpha B crystallin in the blots.

**Table 6: Comparison of the level of expression of alpha B crystallin**

| | Mean | CV% mean | Median | CV% median | Fold mean | Fold median | T-test |
|---|---|---|---|---|---|---|---|
| **CP** | 9.85 | 33.28 | 10.36 | 31.64 | 0.06 | 0.06 | 0.00561 |
| **NCP** | 168.44 | 51.54 | 177.90 | 48.80 | | | |

### Serum amyloid B component

The antibodies used to detect serum amyloid B component were mouse monoclonal antibodies, clone 6E6 (Santa Cruz).

**Table 7** displays the level of serum amyloid B component in complicated plaques and non-complicated plaques. The level of serum amyloid B component is represented by the measure of the optical density of the bands corresponding to serum amyloid B component in the blots.

**Table 7: Comparison of the level of expression of serum amyloid B component**

| | Mean | CV% mean | Median | CV% median | Fold mean | Fold median | T-fest |
|---|---|---|---|---|---|---|---|
| **CP** | 227.87 | 30.37 | 232.61 | 29.36 | 2.89 | 5.09 | 0.01050 |
| **NCP** | 77.82 | 81.74 | 45.69 | 139.22 | | | |

### Thrombospondin-2

The antibodies used to detect thrombospondin-2 were goat polyclonal antibodies, N-20 (Santa Cruz).

**Table 8** displays the level of thrombospondin-2 in complicated plaques and non-complicated plaques. The level of thrombospondin-2 is represented by the measure of the optical density of the bands corresponding to thrombospondin-2 in the blots.

**Table 8: Comparison of the level of expression of thrombospondin-2**

| | Mean | CV% mean | Median | CV% median | Fold mean | Fold median | T-test |
|---|---|---|---|---|---|---|---|
| **CP** | 9.37 | 52.26 | 10.38 | 47.15 | 0.10 | 0.16 | 0.02766 |
| **NCP** | 94.45 | 68.47 | 63.14 | 102.43 | | | |

### Peroxiredoxin-2

The antibodies used to detect peroxiredoxin-2 were mouse monoclonal antibodies, clone 9A1 (Santa Cruz).

**Table 9** displays the level of peroxiredoxin-2 in complicated plaques and non-complicated plaques. The level of peroxiredoxin-2 is represented by the measure of the optical density of the bands corresponding to peroxiredoxin-2 in the blots.

**Table 9: Comparison of the level of expression of peroxiredoxin-2**

| | Mean | CV% mean | Median | CV% median | Fold mean | Fold median | T-test |
|---|---|---|---|---|---|---|---|
| **CP** | 76.86 | 47.60 | 67.78 | 53.98 | 2.73 | 2.81 | 0.02194 |
| **NCP** | 28.19 | 59.96 | 24.13 | 70.04 | | | |

### Pro-Cathepsin D

The antibodies used to detect pro-cathepsin D were goat polyclonal antibodies, C-20 (Santa Cruz).

**Table 10** displays the level of pro-cathepsin D in complicated plaques and non-complicated plaques. The level of pro-cathepsin D is represented by the measure of the optical density of the bands corresponding to pro-cathepsin D in the blots.

**Table 10: Comparison of the level of expression of pro-cathepsin D**

| | Mean | CV% mean | Median | CV% median | Fold mean | Fold median | T-test |
|---|---|---|---|---|---|---|---|
| **CP** | 9.28 | 62.98 | 5.57 | 104.91 | 0.32 | 0.24 | 0.04734 |
| **NCP** | 28.57 | 61.18 | 23.70 | 73.74 | | | |

### Phosphatidylethanolamine-binding protein 1

The antibodies used to detect phosphatidylethanolamine-binding protein 1 were rabbit polyclonal antibodies, FL-187 (Santa Cruz).

**Table 11** displays the level of phosphatidylethanolamine-binding protein 1 in complicated plaques and non-complicated plaques. The level of phosphatidylethanolamine-binding protein 1 is represented by the measure of the optical density of the bands corresponding to phosphatidylethanolamine-binding protein 1 in the blots.

**Table 11: Comparison of the level of expression of phosphatidylethanolamine-binding protein 1**

| | Mean | CV% mean | Median | CV% median | Fold mean | Fold median | T-test |
|---|---|---|---|---|---|---|---|
| **CP** | 39.75 | 46.56 | 38.10 | 48.58 | 0.25 | 0.30 | 0.02846 |
| **NCP** | 161.90 | 56.92 | 128.74 | 71.58 | | | |

### Dihydropyrimidinase-like 3

The antibodies used to detect dihydropyrimidinase-like 3 were mouse monoclonal antibodies, clone 1 B8 (Abnova).

**Table 12** displays the level of dihydropyrimidinase-like 3 in complicated plaques and non-complicated plaques. The level of aciculin is represented by the measure of the optical density of the bands corresponding to dihydropyrimidinase-like 3 in the blots.

**Table 12: Comparison of the level of expression of dihydropyrimidinase-like 3**

| | Mean | CV% mean | Median | CV% median | Fold mean | Fold median | T-test |
|---|---|---|---|---|---|---|---|
| **CP** | 9.26 | 31.55 | 9.47 | 30.87 | 0.11 | 0.20 | 0.04331 |
| **NCP** | 81.96 | 78.31 | 48.24 | 133.05 | | | |

### Osteoglycin

The antibodies used to detect osteoglycin were goat polyclonal antibodies, K-14 (Santa Cruz).

**Table 13** displays the level of osteoglycin in complicated plaques and non-complicated plaques. The level of osteoglycin is represented by the measure of the optical density of the bands corresponding to aciculin in the blots.

**Table 13: Comparison of the level of expression of osteoglycin**

| | Mean | CV% mean | Median | CV% median | Fold mean | Fold median | T-test |
|---|---|---|---|---|---|---|---|
| **CP** | 32.15 | 59.23 | 26.55 | 71.72 | 0.37 | 0.28 | 0.02484 |
| **NCP** | 86.85 | 18.04 | 96.45 | 16.25 | | | |

### DJ-1 oncogene

The antibodies used to detect DJ-1 oncogene were mouse monoclonal antibodies, clone D-4 (Santa Cruz).

**Table 14** displays the level of DJ-1 oncogene in complicated plaques and non-complicated plaques. The level of DJ-1 oncogene is represented by the measure of the optical density of the bands corresponding to DJ-1 oncogene in the blots.

**Table 14: Comparison of the level of expression of DJ-1 oncogene**

| | Mean | CV% mean | Median | CV% median | Fold mean | Fold median | T-test |
|---|---|---|---|---|---|---|---|
| **CP** | 43.10 | 40.39 | 46.80 | 37.19 | 0.27 | 0.32 | 0.00778 |
| **NCP** | 161.83 | 40.17 | 145.95 | 44.54 | | | |

### Pro-Collagen C-endopeptidase enhancer-1

The antibodies used to detect Pro-Collagen C-endopeptidase enhancer-1 were mouse monoclonal antibodies, clone 10D9 (Santa Cruz).

**Table 15** displays the level of Pro-Collagen C-endopeptidase enhancer-1 in complicated plaques and non-complicated plaques. The level of Pro-Collagen C-endopeptidase enhancer-1 is represented by the measure of the optical density of the bands corresponding to Pro-Collagen C-endopeptidase enhancer-1 in the blots.

**Table 15: Comparison of the level of expression of Pro-Collagen C-endopeptidase enhancer-1**

| | Mean | CV% mean | Median | CV% median | Fold mean | Fold median | T-test |
|---|---|---|---|---|---|---|---|
| **CP** | 76.52 | 69.89 | 64.60 | 82.79 | 0.45 | 0.33 | 0.04909 |
| **NCP** | 168.67 | 48.47 | 196.09 | 41.69 | | | |

### Microfibril-associated protein-4

The antibodies used to detect microfibril-associated protein-4 were rabbit polyclonal antibodies (Abcam).

**Table 16** displays the level of microfibril-associated protein-4 in complicated plaques and non-complicated plaques. The level of microfibril-associated protein-4 is represented by the measure of the optical density of the bands corresponding to microfibril-associated protein-4 in the blots.

**Table 16: Comparison-of the level of expression of microfibril associated protein-4**

| | Mean | CV% mean | Median | CV% median | Fold mean | Fold median | T-test |
|---|---|---|---|---|---|---|---|
| **CP** | 23.70 | 24.30 | 23.11 | 24.92 | 0.35 | 0.34 | 0.01770 |
| **NCP** | 68.39 | 42.65 | 67.14 | 43.45 | | | |

### Tissue inhibitor of metalloproteinase 1

**Table 17** displays the level of tissue inhibitor of metalloproteinase 1 in complicated plaques and non-complicated plaques. The level of tissue inhibitor of metalloproteinase 1 is represented by the measure of the normalized volumes of 2D gel spots corresponding to tissue inhibitor of metalloproteinase 1 protein, in the 2D gel differential analysis.

**Table 17: Comparison of the level of expression of tissue inhibitor of metalloproteinase 1**

| | Mean | CV% mean | Median | CV% median | Fold mean | Fold median | T-test |
|---|---|---|---|---|---|---|---|
| **CP** | 97640 | 21.58 | 92314 | 22.82 | 0.53 | 0.49 | 0.00027 |
| **NCP** | 183290 | 20.03 | 188389 | 19.49 | | | |

### Other Biochemical markers:

Using the same approach as for the above mentioned markers, the inventors also observed differential expression of Serpin B9, thrombospondin 1 and HSP27 (level of expression is decreased in CP) and cathepsin K (level of expression is increased in CP).

Antibodies used: Serpin B9: mouse monoclonal antibody clone 6D700 (Santa Cruz); HSP27: mouse monoclonal antibodies, clone F-4 (Santa Cruz); thrombospondin 1: rabbit polyclonal antibodies; cathepsin K: mouse monoclonal antibodies, clone E-7 (Santa Cruz).

### Example 3

This example describes the identification, by the inventors, of other biochemical markers defined in the present application.

### Material and methods

Fluorescent bead-based Bio-Plex Cytokine assays were carried out to determine the level of the markers.

Briefly, cytokines, chemokines and growth factors were measured using the Bio-Plex cytokine assays (human 21-Plex and 27-Plex cytokine panels) (Bio-Rad, Hercules, CA, USA) according to the manufacturer's instructions. 25 µg of protein extracts from complicated plaques (unstable plaques, CP) and non complicated plaques (stable plaques, NCP) were diluted in the sample diluent and used to measure each concentration of analytes.

Eight standards ranging from 2 to 32,000 pg/ml were used to determine the concentration of each analyte.

Results were expressed as pg/mg of protein extracts

### Results

The following proteins were identified by the inventors as being differentially expressed in non-complicated and complicated plaques: IFN-α2, IL-1α, SDF-1α, IL-2ra, IL-12, VEGF, IL-8, G-CSF, GRO alpha, IL-13, LIF and IL-1 β.

The comparison of the levels of expression of these markers in complicated and non-complicated plaques is shown herein below.

### IFN-α2

**Table 18: Comparison of the level of expression of IFN-α2**

| | Mean | CV% mean | Median | CV% median | Fold mean | Fold median | Wilcoxon test |
|---|---|---|---|---|---|---|---|
| **CP** | 26.06 | 18.76 | 26.18 | 18.68 | 0.70 | 0.68 | 0.0001 |
| **NCP** | 36.98 | 12.72 | 38.26 | 12.29 | | | |

### IL-1α

**Table 19: Comparison of the level of expression of IL-1α**

| | Mean | CV% mean | Median | CV% median | Fold mean | Fold median | Wilcoxon test |
|---|---|---|---|---|---|---|---|
| **CP** | 0.72 | 25.36 | 0.72 | 25.48 | 0.62 | 0.65 | 0.0001 |
| **NCP** | 1.17 | 25.62 | 1.10 | 27.23 | | | |

### SDF-1α

**Table 20: Comparison of the level of expression of SDF-1α**

| | Mean | CV% mean | Median | CV% median | Fold mean | Fold median | Wilcoxon test |
|---|---|---|---|---|---|---|---|
| **CP** | 43.96 | 14.34 | 46.63 | 13.52 | 0.84 | 0.89 | 0.0004 |
| **NCP** | 52.21 | 6.45 | 52.22 | 6.44 | | | |

### IL-1ra

**Table 21: Comparison of the level of expression of IL-1 ra**

| | Mean | CV% mean | Median | CV% median | Fold mean | Fold median | Wilcoxon test |
|---|---|---|---|---|---|---|---|
| **CP** | 985.55 | 76.29 | 621.10 | 121.06 | 8.14 | 11.35 | 0.0001 |
| **NCP** | 121.08 | 93.99 | 54.70 | 208.04 | | | |

### IL-12

**Table 22: Comparison of the level of expression of IL-12**

| | Mean | CV% mean | Median | CV% median | Fold mean | Fold median | Wilcoxon test |
|---|---|---|---|---|---|---|---|
| **CP** | 1.66 | 28.70 | 1.58 | 30.17 | 2.42 | 2.68 | 0.0001 |
| **NCP** | 0.69 | 47.66 | 0.59 | 55.45 | | | |

### VEGF

**Table 23: Comparison of the level of expression of VEGF**

| | Mean | CV% mean | Median | CV% median | Fold mean | Fold median | Wilcoxon test |
|---|---|---|---|---|---|---|---|
| **CP** | 13.40 | 33.47 | 12.82 | 34.98 | 3.65 | 5.76 | 0.0001 |
| **NCP** | 3.67 | 85.60 | 2.23 | 141.33 | | | |

### IL-8

**Table 24: Comparison of the level of expression of IL-8**

| | Mean | CV% mean | Median | CV% median | Fold mean | Fold median | Wilcoxon test |
|---|---|---|---|---|---|---|---|
| **CP** | 17.85 | 52.23 | 13.67 | 68.23 | 3.97 | 3.68 | 0.0001 |
| **NCP** | 4.50 | 55.27 | 3.72 | 66.94 | | | |

### G-CSF

**Table 25: Comparison of the level of expression of G-CSF**

| | Mean | CV% mean | Median | CV% median | Fold mean | Fold median | Wilcoxon test |
|---|---|---|---|---|---|---|---|
| **CP** | 5.02 | 35.18 | 5.18 | 34.10 | 2.90 | 3.33 | 0.0001 |
| **NCP** | 1.73 | 45.87 | 1.56 | 51.02 | | | |

### GRO-alpha

**Table 26: Comparison of the level of expression of GRO-alpha**

| | Mean | CV% mean | Median | CV% median | Fold mean | Fold median | Wilcoxon test |
|---|---|---|---|---|---|---|---|
| **CP** | 14.79 | 24.04 | 15.12 | 23.52 | 1.69 | 1.53 | 0.0001 |
| **NCP** | 8.77 | 41.67 | 9.87 | 37.03 | | | |

### IL-13

**Table 27: Comparison of the level of expression of IL-13**

| | Mean | CV% mean | Median | CV% median | Fold mean | Fold median | Wilcoxon test |
|---|---|---|---|---|---|---|---|
| **CP** | 1.63 | 36.45 | 1.50 | 39.56 | 1.93 | 1.76 | 0.0001 |
| **NCP** | 0.84 | 20.95 | 0.85 | 20.78 | | | |

### LIF

**Table 28: Comparison of the level of expression of LIF**

| | Mean | CV% mean | Median | CV% median | Fold mean | Fold median | Wilcoxon test |
|---|---|---|---|---|---|---|---|
| **CP** | 10.91 | 56.38 | 12.35 | 49.81 | 2.97 | 3.50 | 0.0001 |
| **NCP** | 3.68 | 81.06 | 3.53 | 84.38 | | | |

### IL-1β

**Table 29: Comparison of the level of expression of IL-1β**

| | Mean | CV% mean | Median | CV% median | Fold mean | Fold median | Wilcoxon test |
|---|---|---|---|---|---|---|---|
| **CP** | 1.17 | 54.45 | 0.90 | 70.74 | 3.16 | 3.60 | 0.0001 |
| **NCP** | 0.37 | 75.50 | 0.25 | 111.73 | | | |

### Example 4

This example demonstrates the usefulness of detecting the biochemical markers identified by the inventors in the plasma of coronary patients.

### Material and methods

### Patient population

96 patients (mean age, 74.8 ± 11.4 years) were enrolled in the coronary artery disease study. Patients admitted in the emergency medicine department with symptoms such as acute chest pain with suspected acute coronary syndrome were included in the study. All subjects, older than 18 years, gave written informed consent before enrolment in the study protocol approved by the institutional review committee. Following admission, an electrocardiogram (12-lead ECG) and blood level of cardiac Troponin I assay were performed to discriminate proven unstable angina patients from patients with Acute Coronary Syndrome (ACS). Patients without elevation of ST-segment, anginous chest pain at rest and normal to slight increase in plasma troponin levels were included and classified as unstable angina patients (34 unstable patients). Patients presenting chest pain and ischemia type discomfort but not diagnosed for ACS (*i.e.* presenting a normal electrocardiogram and normal Troponin I) were defined as stable angina patients (28 patients). A group composed of 34 patients diagnosed for ACS with elevated plasma cardiac troponin I (> 0.04 ng/ml) at admission was included in the study. For all patients, classical demographic characteristics are recorded in Table 30.

For each patient, 1 ml of venous blood was collected in standard EDTA and dry tubes. The tubes were labelled with a code identifying the patient, date and time of sampling. EDTA plasma samples from all groups were centrifuged and stored at -80°C for biomarker assessment. All biomarker analyses on human samples were performed without clinical information by blinded operator. Baseline levels for each biomarker are presented in Table 31 for stable angina patients.

### Biomarker measurement

Up to 23 biochemical variables were measured using commercial or in-house developed immunoassays in all EDTA plasma as follows.

### a) Commercial tests

Briefly, cardiac Troponin I (cTnI, Access AccuTnl, Beckman Coulter) was measured to validate preliminary Troponin I testing and inclusion criteria at emergency admission. Assays for cytokines, chemokines and growth factors (Bio-Plex Pro Human Cytokine 21-Plex and 27-Plex Assays, Bio-Rad Laboratories), HSP-27 (HSP27 ELISA Kit, Merck), peroxiredoxin-2 (ELISA Kit for Peroxiredoxin-2, USCN Life Science Inc.), TIMP-1 (Human TIMP-1 Quantikine ELISA Kit, R&D Systems), PCPE-1 (ELISA Kit for PCPE-1, USCN Life Science Inc.), DJ-1 oncogene (Circulex Human DJ-1/PARK7 ELISA Kit, MBL International Corporation), osteoglycin (OGN) (ELISA Kit for osteoglycin, USCN Life Science Inc.) and TSP-2 (Human thrombospondin-2 Quantikine ELISA Kit, R&D Systems), were conducted on the samples using commercial immunoassays according to manufacturer's instructions.

Before the coronary plasma assessment, all commercial kits were previously validated in EDTA plasmas from healthy volunteers from the Etablissement Français du Sang (EFS, Montpellier, France) to evaluate the immunoassay reproducibility. Inter and intra variability was reported to be around 10-15% for all commercial immunoassays.

### b) Developed tests

In parallel, a large series of commercial polyclonal and monoclonal antibodies were tested to develop sensitive and specific immunoassays for calponin-1, aciculin, MFAP-4 and PEBP-1.

Due to the lack of robust biologics tools for detecting adequately calponin-1, aciculin, MFAP-4 and PEBP-1 in human plasma samples, the inventors generated monoclonal antibodies for those four proteins.

Full-length purified recombinant protein of *Homo sapiens* were purchased from Abnova Corporation (Aciculin with GST tag, Walnut, CA, USA) or from Origene Technologies (calponin-1, MFAP-4 and PEBP-1 with Myc-tag, Rockville, MD, USA). Mice (Balb/c strain females, 5 weeks old, ref: SIFE055, Charles River, MA, USA) were immunised using one of the following recombinant proteins in order to produce monoclonal antibodies. The experimental protocol on animals was reviewed by the Institutional Animal Ethics Committee. Animal immunization, lymphocyte fusion between spleen cells of the immunised mouse and myeloma SP2 cells were conducted with conventional technical methods known in the art. All clones were tested for their sensitivity and specificity to detect proteins of interest in human plasmas. Selected antibodies were used to develop sandwich immunoassays carried out on a solid support (96-well plate) based on pair antibodies with acceptable sensitivity and specificity.

Developed immunoassays were assessed on EDTA plasmas as follows. The specificity, reproducibility, linearity and limit of detection for developed assays were determined. Specificity of each assay was determined by spiking plasmas with the protein of interest and 2 to 3 irrelevant proteins at different concentrations. Reproducibility of each assay was determined by calculation of the variation coefficient of within-plate, between-plate and between-day experiments using concentration range of recombinant proteins and plasmas. Limit of detection and linearity for each assay were also determined using a concentration range of recombinant proteins. Inter and intra variability was reported to be around 5-10% for all developed immunoassays.
- *Human plasma aciculin detection:* Maxisorp 96-well plates were coated with 100 µl of a home-made anti-aciculin monoclonal antibody solution, enabled to detect aciculin in plasma, at 4°C overnight. After washing and blocking steps, 50 µl of standard range of aciculin recombinant protein or plasmas were mixed with 50 µl of a home-made anti-aciculin monoclonal antibody coupled to peroxidase solution, enabled to detect aciculin in plasma. Then, 100 µl of mixed solution were dispensed into Maxisorp 96-well plates and incubated 1h30 at 37°C. After the final wash step, 100 µl of chromogenic substrate solution (tetra-methylbenzidine; TMB) were dispensed. 50 µl of H₂SO₄ 4N solution were added after 20 minutes of reaction. Optical densities were measured at 450 nm with a plate reader and aciculin plasma concentrations were determined using the dose response curve of the aciculin standard.
- *Human plasma MFAP-4 detection:* Maxisorp 96-well plates were coated with 100 µl of a home-made anti-MFAP-4 monoclonal antibody solution, enabled to detect MFAP-4 in plasma, at 4°C overnight. After washing and blocking steps, 50 µl of standard range of MFAP-4 recombinant protein or plasmas were mixed with 50 µl of a home-made anti-MFAP4 monoclonal antibody coupled to peroxidase solution, enabled to detect MFAP-4 in plasma. Then, 100 µl of mixed solution were dispensed into Maxisorp 96-well plates and incubated 1h30 at 37°C. After the final wash step, 100 µl of TMB substrate solution were dispensed. 50 µl of H₂SO₄ 4N solution were added after 20 minutes of reaction. Optical densities were measured at 450 nm with a plate reader and MFAP-4 plasma concentrations were determined using the dose response curve of the MFAP-4 standard.

- *Human plasma PEBP-1 detection:* Maxisorp 96-well plates were coated with 100 µl an anti-PEBP-1 monoclonal antibody solution enabled to detect PEBP-1 in plasma, at 4°C overnight. After washing and blocking steps, 50 µl of standard range of PEBP-1 recombinant protein or plasmas were mixed with 50 µl of a home-made anti-PEBP-1 monoclonal antibody coupled to peroxidase solution, enabled to detect PEBP-1 in plasma. Then, 100 µl of mixed solution were dispensed into Maxisorp 96-well plates and incubated 1h30 at 37°C. After the final wash step, 100 µl of TMB substrate solution were dispensed. 50 µl of H₂SO₄ 4N solution were added after 20 minutes of reaction. Optical densities were measured at 450 nm with a plate reader and PEBP-1 plasma concentrations were determined using the dose response curve of the PEBP-1 standard.
- *Human plasma calponin-1 detection:* Maxisorp 96-well plates were coated with 100 µl of a home-made anti-calponin-1 monoclonal antibody solution, enabled to detect calponin-1 in plasma, at 4°C overnight. After washing and blocking steps, 50 µl of standard range of calponin-1 recombinant protein or plasmas were mixed with 50 µl of purified rabbit polyclonal antibody solution enabled to detect calponin-1 in plasma. Then, 100 µl of mixed solution were dispensed into Maxisorp 96-well plates and incubated 1h30 at 37°C. After washing step, 100 µl of anti-rabbit IgG coupled to peroxidase solution were dispensed and incubated for 1 hour at 37°C. After the final wash step, 100 µl of TMB substrate solution were dispensed. 50 µl of H₂SO₄ 4N solution were added after 20 minutes of reaction. Optical densities were measured at 450 nm with a plate reader and calponin-1 plasma concentrations were determined using the dose response curve of the calponin-1 standard.

The levels of the various markers (single and combined markers) for each patient were then correlated to the potential occurrence of vascular events (plaque rupture) in said patient, according to statistical methods well known to the man skilled in the art.

### Statistical Analysis

All statistical tests were performed with "R" open source software (version 2.12.2) on SAS 9.2 software (SAS Institute Inc.). The differences between groups were analyzed by the non-parametric Wilcoxon rank sum test and by the parametric Welch's test. A p value less than 0.05 was considered as statistically significant. Values are presented as mean ± standard deviation (SD). A logarithmic (log10) transformation was applied on the biomarker expression levels in plasmas to have distribution normality assumptions. All data distributions are illustrated as medians and box plots for each biomarker. Differences among patient groups for demographic and clinical characteristics were evaluated by Chi-square test on ANOVA test.

The marker diagnostic performance could be characterised by: sensitivity, which represents its ability to detect the diseased population and specificity which represents its ability to detect the control population. The results of the evaluation of a diagnostic test can be summarised in a 2x2 contingency table comparing these two well-defined populations. By forming a cut-off, the two populations could be classified into categories according to the results of the test, categorised as either positive or negative. So for a particular marker, a subjects with a positive test result among the "cases" population (*the "True Positive": TP)* and *b* subjects with a positive test result among the "controls" population *(the "True Negative": TN*) are observed. In the same fashion, *c* subjects with a negative test result among the cases *(the "False Positive": FP)* and *d* subjects with a negative test result among the controls *(the "False Negative": FN)* are observed. Sensitivity is defined as TP/(TP+FN); which is herein referred to as the "true positive rate". It is calculated in the sample of cases. The sensitivity (Se) of a test at a particular cut-off thus corresponds to the proportion of positive results among the cases. Specificity is defined as TN/(TN+FP); which is herein referred to as the "true negative rate". It is calculated in the sample of controls. The specificity (Sp) of a test at a particular cut-off thus corresponds to the proportion of negative results among the controls.

The sensibility and specificity were calculated for a defined cut-off. The marker concentrations with the highest diagnostic accuracy (minimal false-negative and false positive results) were selected as a defined cut-off or threshold. AUC (Area Under the Curve) illustrates the overall index for the diagnostic performance of the respective single markers or a combination of markers.

Receiving operating characteristic (ROC) analysis was performed for single biomarker and for multimarker combination (multiple ROC, mROC), when comparing patients diagnosed for stable angina to patients diagnosed for unstable angina or ACS with ROC software well described by Kramar et al. (Comput. Methods Programs Biomed. 2001;66:199-207; Revue d'Epidémiologie et Santé Publique 1999;47:376-83). mROC is a dedicated program calculating the linear combination (Su et al. Journal of the American Statistical Association 1993;88:1350-1355), to maximize the AUC (Area Under the Curve) ROC for all markers selected and also for all two and three marker combinations among the selected markers (Staack et al. BMC Urology 2006;6:1-12). The equation for the respective combination is provided and can be used as a new virtual marker. For a marker combination and for a sample selected, the cut-off is the result value of the corresponding linear equation and calculated by the mROC program:
Marker combination cut-off = a x Marker₁ + b x Marker₂ + c x Marker₃,
where a, b, c are calculated coefficients and Marker_{1,2,3} are individual marker cut-offs.

A logistic regression model was also applied using the *"proc logistic"* procedure in SAS, to evaluate the predictive relationship between biomarker expression in plasma and plaque rupture in coronary patients. Coefficients were tested for significance for selection or elimination from the model with Wald test, Likelihood-Ratio test and Hosmer-Lemshow Goodness of Fit test. Clinical characteristics (age, sex and race, Table 30) were not included in the regression model. Presence of plaque rupture in unstable angina and ACS patients was used as the dependent variable and differential biomarker expression (p<0.20) between stable angina patients and unstable/ACS patients as the independent variable. The logistic regression model was applied for univariable and multivariable conditions. Sensitivity, specificity, and accuracy were measured in the same datasets.

### Results

### Demographic analysis of patient population

As reported in table 30, the coronary patients diagnosed at admission in the emergency medicine department for stable angina (n=28), unstable angina (n=34) and acute coronary syndrome (n=34) did not differ in age, gender and race.

**Table 30: Baseline demographic of stable angina, unstable angina and ACS individuals**

| | Stable Angina | Unstable angina | ACS | Stable *vs* unstable angina *vs* AC p-value |
|---|---|---|---|---|
| Mean aqe, year | 75.54 ± 11.47 | 76.41 ± 11.81 | 72.68 ± 10.98 | 0.377 |
| Age Min-Max, year | 48-93 | 50-94 | 52-94 | |
| Gender, male/female (%) | 13/15 (46.43/53.57) | 18/16 (52.94/47.06) | 15/19 (44.12/55.88) | 0.753 |
| Ethnicity Caucasian, n (%) | 20 (71.43) | 22 (64.71) | 25 (73.53) | 0.823 |
| Ethnicity A American, n (%) | 7 (25.00) | 9 (26.47) | 6 (17.65) | |
| Ethnicity Black, n (%) | 1 (3.57) | 2 (05.88) | 3 (8.82) | |
| Ethnicity Hispanic, n (%) | 0 (0) | 1 (2.94) | 0 (0) | |

*Data are means ± Standard deviation. ACS: acute coronary syndrome.*

### Baseline levels of circulating biomarkers

Based on proteins differentially expressed in atherosclerotic plaques which are listed in the present application, a large series of proteins were successfully detected in plasma patients using validated immunoassays. Table 31 reports the baseline levels of circulating biomarkers in stable angina patients.

**Table 31: Baseline levels of proteins in plasmas from stable angina patients**

| **Marker** | **Mean** | **Median** | **Stand. Dev.** | **1st Quartile** | **3rd Quartile** |
|---|---|---|---|---|---|
| TSP-2 (ng/ml) | 38.33 | 30.92 | 27.10 | 18.83 | 47.15 |
| PCPE-1 (ng/ml) | 133.5 | 104.6 | 108.8 | 42.91 | 183.9 |
| TIMP-1 (ng/ml) | 266.3 | 237.8 | 153.6 | 152.2 | 337.7 |
| DJ-1 (ng/ml) | 204.8 | 170.1 | 141.4 | 112.2 | 255.4 |
| PRDX2 (ng/ml) | 12.45 | 12.48 | 6.46 | 8.54 | 15.92 |
| OGN (ng/ml) | 32.27 | 27.20 | 20.51 | 18.26 | 38.04 |
| HSP27 (ng/ml) | 3.35 | 2.65 | 2.71 | 1.38 | 4.55 |
| MFAP-4 (ng/ml) | 1089.1 | 1117.3 | 223.6 | 973.0 | 1253.5 |
| PEBP-1 (ng/ml) | 342.5 | 211.1 | 392.8 | 142.3 | 345.0 |
| Calponin-1 (ng/ml) | 13.81 | 5.72 | 22.71 | 0.80 | 16.13 |
| Aciculin (nq/ml) | 327.8 | 297.4 | 171.9 | 213.7 | 391.2 |
| GRO alpha (pg/ml) | 44.65 | 36.78 | 35.52 | 27.83 | 52.10 |
| IL-1 alpha (pg/ml) | 2.09 | 1.23 | 5.59 | 0.89 | 1.76 |
| LIF (pg/ml) | 29.58 | 24.34 | 25.16 | 14.64 | 34.14 |
| SDF-1a (pg/ml) | 127.3 | 116.2 | 77.53 | 91.77 | 134.7 |
| IFN-a2 (pg/ml) | 99.89 | 96.93 | 27.22 | 80.20 | 110.5 |
| IL-1 beta (pg/ml) | 1.82 | 1.45 | 1.71 | 0.93 | 1.92 |
| IL-1ra (pg/ml) | 428.3 | 285.5 | 784.7 | 163.2 | 473.4 |
| IL-8 (pg/ml) | 33.46 | 17.27 | 74.35 | 10.93 | 34.14 |
| IL-12 (p70) (pg/ml) | 15.22 | 10.45 | 15.60 | 4.95 | 16.80 |
| IL-13 (pg/ml) | 5.77 | 4.12 | 7.38 | 2.31 | 6.08 |
| G-CSF (pg/ml) | 321.8 | 41.99 | 1287.2 | 24.28 | 56.68 |
| VEGF (pg/ml) | 47.64 | 39.28 | 42.07 | 17.85 | 61.20 |

All circulating levels of proteins were determined by validated immunoassays on 28 stable patients with less than 10-15% of variability. Protein levels in human EDTA plasmas are detectable in the range of pg/ml to ng/ml.

### Univariable analysis of biomarkers

### a) Stable angina patients versus unstable angina patients

The level of the biochemical markers listed in Table 31 was determined and compared in patients with stable and unstable angina.

As example, when comparing patients with stable angina (n=28) to unstable angina (n=34), circulating calponin-1, interleukin-8 and DJ-1 oncogene levels were significantly altered (Figures 13, 14 and 15). A significant 6-fold increase of plasma calponin-1 was detected in patients diagnosed for unstable angina as compared to stable angina patients (p=0.009). Circulating interleukin-8 and DJ-1 oncogene decreased in unstable patients as compared to stable individuals. Based on Receiving-Operating-Characteristic (ROC) analysis for circulating marker levels, the area under the curve (AUC) for individual marker, such as calponin-1, interleukin-8 and DJ-1 oncogene, allowed discriminating patients diagnosed for stable angina from unstable angina patients (Table 32).

**Table 32: Discriminative performance of single marker when comparing unstable angina patients to stable angina patients**

| **Markers** | **AUC** | **Threshold** | **Se %** | **Sp %** | **PPV %** | **NPV %** | **CI 95%** |
|---|---|---|---|---|---|---|---|
| Calponin-1 | 0.696 | 0.617 | 79.4 | 67.9 | 75.0 | 73.1 | [0.544 ; 0.815] |
| IL8 | 0.662 | -1.305 | 79.4 | 60.7 | 71.0 | 70.8 | [0.506 ; 0.790] |
| DJ1 | 0.638 | -2.357 | 82.4 | 50.0 | 66.6 | 70.1 | [0.486 ; 0.767] |

The accuracy of each marker and its discriminatory power was evaluated using a Receiving Operating Characteristics (ROC) analysis. ROC curves are the graphical visualization of the reciprocal relation between the sensitivity (Se) and the specificity (Sp) of a test for various values. AUC means area under the curve, with its confidence interval (CI). ROC Curves are based on models of prediction of plaque vulnerability by calculating optimal threshold of sensitivity (Se %) and specificity (Sp%) for single marker. Positive (PPV, %) and negative (NPV, %) predictive values for single marker were calculated to evaluate the proportion of true presence [true positive /(true positive+ false positive] and true absence [true negative /(true negative+ false negative)] of plaque rupture in unstable patients.

### b) Stable angina patients versus ACS patients

When comparing stable angina patients (n=28) to ACS patients (n=34), calponin-1 was for example significantly increased (p=0.021, Figure 16) and VEGF significantly decreased (p=0.032, Figure 17). Using ROC analysis for circulating marker levels, the AUC for individual marker, such as calponin-1, VEGF and PCPE-1, discriminated patients diagnosed for stable angina from acute coronary syndrome patients. Table 33 reports the discriminative performance of single markers in comparing stable patients to ACS patients.

**Table 33: Discriminative performance of single marker when comparing stable angina patients to ACS patients**

| **Markers** | **AUC** | **Threshold** | **Se %** | **Sp %** | **PPV %** | **NPV %** | **CI 95%** |
|---|---|---|---|---|---|---|---|
| Calponin-1 | 0.671 | 0.593 | 76.5 | 67.9 | 74.3 | 70.4 | [0.513 ; 0.798] |
| VEGF | 0.655 | -1.6 | 82.4 | 50.0 | 66.6 | 70.1 | [0.503 ; 0.781] |
| PCPE1 | 0.640 | -1.951 | 70.6 | 60.7 | 68.5 | 63.0 | [0.491 ; 0.766] |

The accuracy of each marker and its discriminatory power was evaluated using a Receiving Operating Characteristics (ROC) analysis. ROC curves are the graphical visualization of the reciprocal relation between the sensitivity (Se) and the specificity (Sp) of a test for various values. AUC means area under the curve, with its confidence interval (CI). ROC Curves are based on models of prediction of plaque vulnerability by calculating optimal threshold of sensitivity (Se %) and specificity (Sp %) for single marker. Positive (PPV, %) and negative (NPV, %) predictive values for single marker were calculated to evaluate the proportion of true presence [true positive /(true positive+ false positive] and true absence [true negative /(true negative+ false negative)] of plaque rupture in unstable patients.

### Multivariable analysis of biomarkers

Multimarker analysis with mROC (multiple Receiving-Operating-Characteristic) approach improved significantly AUC when comparing stable to unstable patients. The marker combination associating for example calponin-1 to II-8, aciculin, DJ-1 , SDF1a, VEGF, TIMP1, HSP27, IL12, MFAP4, PEBP1, PCPE1 or osteoglycin (OGN) has a predictive value for higher risk of cardiovascular events in unstable patients as reported by the higher sensitivity and specificity (Table 34). When associating a third marker, the predictive value was significantly improved as illustrated in the Table 35. The statistical analysis combining two or three markers generated a series of decision rules; a new virtual marker (Z) was calculated for each combination as illustrated in Figures 18, 19 and 20 (stable patients versus unstable patients) and Figures 21, 22 and 23 (stable patients versus ACS patients).

Based on the combination of 2 markers, the virtual marker, transposing markers from the multivariate conditions into a univariate setting, discriminated significantly unstable patients from stable patients (Z = 0.474x[Calponin-1] - 0.688x[IL8] with a p value at 0.0004 ; Z = 0.495x[Calponin-1] - 0.903x[Aciculin] with a p value at 0.0017, Figure 18 and 19 respectively), and discriminated significantly ACS patients from stable cases (Z = 0.547x[Calponin1] - 0.705x[VEGF] with a p value at 0.0007 and Z = 0.505x[Calponin1] - 0.914x[PEBP1] with a p value at 0.0018, Figures 21 and 22 respectively).

In the same way, combining three markers generated a new virtual marker with good diagnostic accuracy between unstable and stable patients (Z = 0.500x[Calponin-1] - 0.649x[IL8] - 1.588x[MFAP4] with a p value at 0.0002 Figure 20 ; Z = 0.470x[Calponin1] - 0.726x[IL8] - 0.277x[HSP27] with a p value at 0.0004), and discriminated significantly ACS patients from stable cases (Z = 0.580x[Calponin1] - 0.672x[VEGF] - 0.878x[PEBP1] with a p value at 0.0001, Figure 23 ; Z = 0.541x[Calponin-1] - 1.061x[PEBP1] - 1.036x[IL1ra] with a p value at 0.0001).

Tables 34 and 35 report a series of multimarker evaluation giving the opportunity to improve vulnerability diagnosis at admission in the emergency department, by discriminating safely and rapidly stable patients from unstable patients. Similar multimarker analyses were performed to illustrate the significant improvement of predicting cardiovascular event in ACS patients (Tables 36 and 37).

**Table 34: Biomarker performance using multivariable analysis with 2 markers (stable versus unstable patients)**

| **Marker combination** | **AUC** | **Threshold** | **Se %** | **Sp %** | **PPV %** | **NPV %** | **CI 95%** |
|---|---|---|---|---|---|---|---|
| Calponin-1 + IL8 | 0.763 | -0.526 | 76.5 | 71.4 | 76.4 | 71.5 | [0.624 ; 0.861] |
| Calponin-1 + Aciculin | 0.737 | -1.997 | 82.4 | 67.9 | 75.7 | 76.1 | [0.588 ; 0.847] |
| Calponin-1 + SDF1 a | 0.729 | -1.273 | 79.4 | 67.9 | 75.0 | 73.1 | [0.586 ; 0.837] |
| Calponin-1 + DJ1 | 0.724 | -1.799 | 91.2 | 57.1 | 72.0 | 84.3 | [0.570 ; 0.838] |
| Calponin-1 + VEGF | 0.723 | -0.212 | 79.4 | 67.9 | 75.0 | 73.1 | [0.577 ; 0.833] |
| Calponin-1 + TIMP1 | 0.720 | 1.084 | 79.4 | 67.9 | 75.0 | 73.1 | [0.574 ; 0.830] |
| Calponin-1 + HSP27 | 0.714 | 0.028 | 88.2 | 60.7 | 73.1 | 80.9 | [0.806 ; 0.882] |
| Calponin-1 + GROalpha | 0.714 | -0.498 | 73.5 | 67.9 | 73.5 | 67.9 | [0.570 ; 0.825] |
| Calponin-1 + IL12p70 | 0.711 | 0.108 | 82.4 | 71.4 | 77.7 | 77.0 | [0.560 ; 0.826] |
| Calponin-1 + MFAP4 | 0.709 | -5.059 | 79.4 | 64.3 | 72.9 | 72.0 | [0.561 ; 0.823] |
| Calponin-1 + PEBP1 | 0.709 | -0.419 | 82.4 | 60.7 | 71.8 | 74.0 | [0.562 ; 0.823] |
| Calponin-1 + PCPE1 | 0.708 | -0.27 | 79.4 | 60.7 | 71.0 | 70.8 | [0.564 ; 0.820] |
| Calponin-1 + OGN | 0.707 | 0.414 | 82.4 | 64.3 | 73.7 | 75.1 | [0.556 ; 0.823] |

The accuracy of multimarker panel and its discriminatory power was evaluated using a Receiving Operating Characteristics (ROC) analysis. ROC curves are the graphical visualization of the reciprocal relation between the sensitivity (Se) and the specificity (Sp) of a test for various values. AUC means area under the curve, with its confidence interval (CI). ROC Curves are based on models of prediction of plaque vulnerability by calculating optimal threshold of sensitivity (Se %) and specificity (Sp %) for multimarker panel. Positive (PPV, %) and negative (NPV, %) predictive values for combined marker were calculated to evaluate the proportion of true presence [true positive /(true positive+ false positive] and true absence [true negative /(true negative+ false negative)] of plaque rupture in unstable patients. As example, the equations for the marker combinations calculated by the mROC program were: a) Z = 0.474x[Calponin-1] - 0.688x[IL8], b) Z = 0.495x[Calponin-1] - 0.903x[Aciculin], c) Z = 0.477x[Calponin] - 0.752x[SDF1a], d) Z = 0.431x[Calponin1] - 0.818x[DJ1], e) Z = 0.487x[Calponin1] - 0.345x[VEGF], f Z = 0.462x[Calponin1] + 0.346x[TIMP1], g) Z = 0.442x[Calponin1] - 0.247x[HSP27], h) Z = 0.417x[Calponin1] - 0.513x[GROalpha], i) Z = 0.462x[Calponin1] - 0.156x[IL12p70], j) Z = 0.478x[Calponin1] - 1.752x[MFAP4], k) Z = 0.456x[Calponin1] - 0.286x[PEBP1], I) Z = 0.439x[Calponin1] - 0.261x[PCPE1] and m) Z = 0.443x[Calponin1] + 0.130x[OGN]. Using these equations a new virtual marker (Z) was calculated with significant diagnostic accuracy (p<0.05).

**Table 35: Biomarker performance using multivariable analysis with 3 markers (stable versus unstable patients)**

| **Marker combination** | **AUC** | **Threshold** | **Se %** | **Sp %** | **PPV %** | **NPV %** | **CI 95%** |
|---|---|---|---|---|---|---|---|
| Calponin-1 + IL8 + MFAP4 | 0.778 | -5.262 | 73.5 | 75.0 | 78.1 | 70.0 | [0.646 ; 0.871] |
| Calponin-1 + IL8 + HSP27 | 0.776 | -0.592 | 70.6 | 82.1 | 82.7 | 69.7 | [0.636 ; 0.873] |
| Calponin-1 + IL8 + TSP2 | 0.77 | -0.668 | 76.5 | 75.0 | 78.8 | 72.5 | [0.632 ; 0.867] |
| Calponin-1 + IL8 + PCPE1 | 0.769 | -1.171 | 76.5 | 75.0 | 78.8 | 72.5 | [0.631 ; 0.866] |
| Calponin-1 + IL8 + TIMP1 | 0.769 | -0.009 | 79.4 | 71.4 | 77.1 | 74.1 | [0.633 ; 0.865] |
| Calponin-1 + IL8 + DJ1 | 0.769 | -2.736 | 82.4 | 75.0 | 80.0 | 77.9 | [0.626 ; 0.869] |
| Calponin-1 + IL8 + GROalpha | 0.767 | -1.57 | 79.4 | 67.9 | 75.0 | 73.1 | [0.632 ; 0.863] |
| Calponin-1 + IL8 + IL1 beta | 0.765 | -0.462 | 70.6 | 78.6 | 80.0 | 68.8 | [0.629 ; 0.862] |
| Calponin-1 + IL8 + IL13 | 0.765 | -0.511 | 76.5 | 71.4 | 76.4 | 71.4 | [0.627 ; 0.862] |
| Calponin-1 + IL8 + VEGF | 0.764 | -0.603 | 79.4 | 71.4 | 77.1 | 74.1 | [0.625 ; 0.862] |
| Calponin-1 + IL8 + IL12p70 | 0.763 | -0.399 | 70.6 | 75.0 | 77.4 | 67.8 | [0.625 ; 0.861] |
| Calponin-1 + IL8 + IL1alpha | 0.763 | -0.842 | 85.3 | 64.3 | 74.3 | 78.3 | [0.623 ; 0.862] |
| Calponin-1 + IL8 + LIF | 0.763 | -0.46 | 76.5 | 71.4 | 76.5 | 71.4 | [0.624 ; 0.861] |
| Calponin-1 + IL8 + Aciculin | 0.762 | -2.929 | 73.5 | 75.0 | 78.1 | 70.0 | [0.618 ; 0.863] |
| Calponin-1 + IL8 + IFNa2 | 0.761 | -1.104 | 76.5 | 71.4 | 76.4 | 71.5 | [0.623 ; 0.859] |
| Calponin-1 + Aciculin + VEGF | 0.761 | -2.631 | 73.5 | 71.4 | 75.7 | 69.0 | [0.617 ; 0.862] |
| Calponin-1 + IL8 + IL1ra | 0.757 | 0.412 | 70.6 | 75 | 77.4 | 67.8 | [0.620 ; 0.856] |
| Calponin-1 + IL8 + OGN | 0.755 | -0.362 | 76.5 | 71.4 | 76.4 | 71.5 | [0.616 ; 0.856] |
| Calponin-1 + IL8 + SDF1 a | 0.755 | -1.739 | 76.5 | 71.4 | 76.4 | 71.5 | [0.618 ; 0.855] |
| Calponin-1 + Aciculin + DJ1 | 0.755 | -4.322 | 94.1 | 60.7 | 74.4 | 89.5 | [0.606 ; 0.861] |
| Calponin-1 + IL8 + PEBP1 | 0.754 | -2.035 | 85.3 | 67.9 | 76.3 | 79.2 | [0.609 ; 0.858] |
| Calponin-1 + IL8 + GCSF | 0.754 | -0.501 | 79.4 | 67.9 | 75 | 73.1 | [0.612 ; 0.857] |
| Calponin-1 + IL8 + PRDX2 | 0.752 | -0.569 | 85.3 | 64.3 | 74.3 | 78.3 | [0.611 ; 0.854] |
| Calponin-1 + Aciculin + PEBP1 | 0.747 | -3.128 | 82.4 | 71.4 | 77.7 | 77.0 | [0.601 ; 0.852] |

The accuracy of multimarker panel and its discriminatory power was evaluated using a Receiving Operating Characteristics (ROC) analysis. ROC curves are the graphical visualization of the reciprocal relation between the sensitivity (Se) and the specificity (Sp) of a test for various values. AUC means area under the curve, with its confidence interval (CI). ROC Curves are based on models of prediction of plaque vulnerability by calculating optimal threshold of sensitivity (Se %) and specificity (Sp %) for multimarker panel. Positive (PPV, %) and negative (NPV, %) predictive values for combined marker were calculated to evaluate the proportion of true presence [true positive /(true positive+ false positive] and true absence [true negative /(true negative+ false negative)] of plaque rupture in unstable patients. As example, the equations for the marker combinations calculated by the mROC program were: a) Z = 0.500x[Calponin-1] - 0.649x[IL8] - 1.588x[MFAP4], b) Z = 0.470x[Calponin1] - 0.726x[IL8] - 0.277x[HSP27], c) Z = 0.465x[Calponin1] - 0.722x[IL8] - 0.316x[PCPE1], d) Z = 0.471x[Calponin1] - 0.692x[IL8] - 0.125x[TSP2], e) Z = 0.484x[Calponin1] - 0.672x[IL8] + 0.247x[TIMP1], f) Z = 0.459x[Calponin1] - 0.757x[IL8] - 0.914x[DJ1], g) Z = 0.440x[Calponin1] - 0.710x[IL8] - 0.587x[GROalpha], h) Z = 0.466x[Calponin1] - 0.787x[IL8] - 0.378x[IL1beta], i) Z = 0.470x[Calponin1] - 0.692x[IL8] + 0.039x[IL13], j) Z = 0.481x[Calponin1] - 0.640x[IL8] - 0.075x[VEGF], k) Z = 0.466x[Calponin1] - 0.749x[IL8] + 0.109x[IL12p70], I) Z = 0.466x[Calponin1] - 0.691x[IL8] - 0.156x[IL1alpha], m) Z = 0.477x[Calponin1] - 0.686x[IL8] + 0.067x[LIF], n) Z = 0.527x[Calponin1] - 0.721x[IL8] - 0.963x[Aciculin], o) Z = 0.467x[Calponin1] - 0.674x[IL8] - 0.297x[IFNa2], p) Z = 0.542x[Calponin1] - 0.956x[Aciculin] - 0.381 x[VEGF], q) Z = 0.488x[Calponin] - 0.886x[IL8] + 0.449x[IL1ra*], r) Z = 0.469x[Calponin1] - 0.689x[IL8] + 0.143x[OGN], s) Z = 0.498x[Calponin1] - 0.658x[IL8] - 0.640x[SDF1a], t) Z = 0.482x[Calponin1] - 0.964x[Aciculin] - 0.865x[DJ1], u) Z = 0.494x[Calponin1] - 0.801x[IL8] - 0.470x[PEBP1], v) Z = 0.474x[Calponin1] - 0.800x[IL8] + 0.160x[GCSF] and w) Z = 0.455x[Calponin1] - 0.671x[IL8] + 0.248x[PRDX2] and x) Z = 0.513x[Calponin1] - 1.001x[Aciculin] - 0.386x[PEBP1]. Using these equations a new virtual marker (Z) was calculated with significant diagnostic accuracy (p<0.05).

**Table 36: Biomarker performance using multivariable analysis with 2 markers (stable versus ACS patients)**

| **Marker combination** | **AUC** | **Threshold** | **Se %** | **Sp %** | **PPV %** | **NPV %** | **CI 95%** |
|---|---|---|---|---|---|---|---|
| Calponin-1 + VEGF | 0.757 | -0.653 | 73.5 | 75.0 | 78.1 | 70.0 | [0.615 ; 0.859] |
| Calponin-1 + PEBP1 | 0.746 | -2.06 | 94.1 | 60.7 | 74.4 | 89.5 | [0.597 ; 0.853] |
| Calponin-1 + HSP27 | 0.734 | -0.14 | 88.2 | 60.7 | 73.1 | 80.9 | [0.583 ; 0.845] |
| Calponin-1 + SDF1a | 0.732 | -2.939 | 73.5 | 67.9 | 73.5 | 67.9 | [0.585 ; 0.841] |
| Calponin-1 + PCPE1 | 0.727 | -1.045 | 70.6 | 60.7 | 68.5 | 63.0 | [0.588 ; 0.832] |
| Calponin-1 + IL1ra | 0.726 | -1.765 | 76.5 | 67.9 | 74.3 | 70.4 | [0.576 ; 0.837] |
| Calponin-1 + IL12p70 | 0.722 | -0.273 | 88.2 | 57.1 | 71.4 | 80.0 | [0.570 ; 0.835] |
| IL13 + VEGF | 0.705 | -1.48 | 97.1 | 42.9 | 67.3 | 92.4 | [0.558 ; 0.819] |
| Calponin-1 + IL8 | 0.699 | -0.165 | 70.6 | 71.4 | 75.0 | 66.7 | [0.544 ; 0.818] |
| Calponin-1 + TSP2 | 0.696 | -0.668 | 85.3 | 57.1 | 70.7 | 76.2 | [0.543 ; 0.816] |
| Calponin-1 + GCSF | 0.694 | -0.06 | 76.5 | 67.9 | 74.3 | 70.4 | [0.544 ; 0.812] |
| Calponin-1 + LIF | 0.691 | 0.001 | 76.5 | 64.3 | 72.2 | 69.3 | [0.538 ; 0.811] |
| IL1ra + PCPE1 | 0.691 | -3.581 | 82.4 | 57.1 | 70.0 | 72.8 | [0.539 ; 0.811] |
| VEGF + PCPE1 | 0.686 | -2.1 | 94.1 | 50.0 | 69.5 | 87.5 | [0.528 ; 0.810] |
| Calponin-1 + PRDX2 | 0.685 | 0.423 | 82.4 | 64.3 | 73.7 | 75.1 | [0.526 ; 0.810] |
| Calponin-1 + GROalpha | 0.684 | -0.856 | 85.3 | 53.6 | 69.0 | 75.0 | [0.532 ; 0.805] |
| Calponin-1 + DJ1 | 0.682 | -0.498 | 91.2 | 50.0 | 68.9 | 82.4 | [0.524 ; 0.807] |
| Calponin-1 + TIMP1 | 0.682 | 1.144 | 79.4 | 60.7 | 71.0 | 70.8 | [0.527 ; 0.805] |
| Calponin-1 + MFAP4 | 0.676 | -3.45 | 76.5 | 67.9 | 74.3 | 70.4 | [0.518 ; 0.803] |
| Calponin-1 + IL13 | 0.675 | 0.292 | 82.4 | 60.7 | 71.8 | 74.0 | [0.518 ; 0.801] |
| Calponin-1 + Aciculin | 0.670 | 0.231 | 76.5 | 67.9 | 74.3 | 70.4 | [0.514 ; 0.796] |
| Calponin-1 + OGN | 0.671 | -0.138 | 76.5 | 60.7 | 70.2 | 68.1 | [0.519 ; 0.794] |

The accuracy of multimarker panel and its discriminatory power was evaluated using a Receiving Operating Characteristics (ROC) analysis. ROC curves are the graphical visualization of the reciprocal relation between the sensitivity (Se) and the specificity (Sp) of a test for various values. AUC means area under the curve, with its confidence interval (CI). ROC Curves are based on models of prediction of plaque vulnerability by calculating optimal threshold of sensitivity (Se %) and specificity (Sp %) for multimarker panel. Positive (PPV, %) and negative (NPV, %) predictive values for combined marker were calculated to evaluate the proportion of true presence [true positive /(true positive+ false positive] and true absence [true negative /(true negative+ false negative)] of plaque rupture in ACS patients. As example, the equations for the marker combinations calculated by the mROC program were: a) Z = 0.547x[Calponin1] - 0.705x[VEGF], b) Z = 0.505x[Calponin1] - 0.914x[PEBP1], c) Z = 0.485x[Calponin1] - 0.650x[HSP27], d) Z = 0.544x[Calponin1] - 1.585x[SDF1a], e) Z = 0.504x[Calponin1] - 0.679x[PCPE1], f) Z = 0.490x[Calponin1] - 0.833x[IL1ra], g) Z = 0.521x[Calponin1] - 0.456x[IL12p70], h) Z = 1.264x[IL13] - 1.302x[VEGF], i) Z = 0.469x[Calponin1] - 0.397x[IL8], j) Z = 0.477x[Calponin1] - 0.502x[TSP2], k) Z = 0.453x[Calponin1] - 0.202x[GCSF], I) Z = 0.460x[Calponin1] - 0.194x[LIF], m) Z = -0.898x[IL1ra] - 0.689x[PCPE1], n) Z = -0.507x[VEGF] - 0.592x[PCPE1], o) Z = 0.459x[Calponin1] + 0.174x[PRDX2], p) Z = 0.447x[Calponin1] - 0.641x[GROalpha], q) Z = 0.455x[Calponin1] - 0.249x[DJ1], r) Z = 0.488x[Calponin1] + 0.386x[TIMP1], s) Z = 0.467x[Calponin1] - 1.226x[MFAP4], t) Z = 0.458x[Calponin1] + 0.112x[IL13], u) Z = 0.474x[Calponin1] - 0.297x[OGN] and v) Z = 0.466x[Calponin1] - 0.016x[Aciculin]. Using these equations a new virtual marker (Z) was calculated with significant diagnostic accuracy (p<0.05).

**Table 37: Biomarker performance using multivariable analysis with 3 markers (stable versus ACS patients)**

| **Marker combination** | **AUC** | **Threshold** | **Se %** | **Sp %** | **PPV %** | **NPV %** | **CI 95%** |
|---|---|---|---|---|---|---|---|
| Calponin-1 + PEBP1 + IL1ra | 0.814 | -4.75 | 85.3 | 67.9 | 76.3 | 79.2 | [0.675 ; 0.902] |
| Calponin-1 + VEGF + PCPE1 | 0.789 | -2.165 | 76.5 | 71.4 | 76.4 | 71.5 | [0.658 ; 0.879] |
| Calponin-1 + VEGF + PEBP1 | 0.788 | -2.91 | 91.2 | 60.7 | 73.8 | 85.1 | [0.650 ; 0.881] |
| Calponin-1 + VEGF + IL13 | 0.786 | -1.053 | 88.2 | 75.0 | 81.1 | 84.0 | [0.639 ; 0.884] |
| Calponin-1 + PEBP1 + IL12p70 | 0.784 | -2.377 | 82.4 | 67.9 | 75.7 | 76.1 | [0.641 ; 0.880] |
| Calponin-1 + PCPE1 + IL1ra | 0.783 | -3.805 | 79.4 | 75.0 | 79.4 | 75.0 | [0.646 ; 0.877] |
| Calponin-1 + PEBP1 + SDF1 a | 0.782 | -4.802 | 79.4 | 67.9 | 75.0 | 73.1 | [0.645 ; 0.876] |
| Calponin-1 + VEGF + SDF1 a | 0.780 | -4.315 | 79.4 | 75.0 | 79.4 | 75.0 | [0.643 ; 0.875] |
| Calponin-1 + HSP27 + IL1ra | 0.777 | -2.326 | 85.3 | 67.9 | 76.3 | 79.2 | [0.629 ; 0.878] |
| Calponin-1 + HSP27 + SDF1 a | 0.773 | -3.22 | 70.6 | 78.6 | 80.0 | 68.8 | [0.631 ; 0.872] |
| Calponin-1 + SDF1a + IL1ra | 0.771 | -5.809 | 88.2 | 67.9 | 76.9 | 82.6 | [0.625 ; 0.872] |
| Calponin-1 + IL8 + PCPE1 | 0.769 | -1.497 | 61.8 | 82.1 | 80.7 | 63.9 | [0.633 ; 0.865] |
| Calponin-1 + IL8 + PEBP1 | 0.769 | -2.98 | 91.2 | 64.3 | 75.6 | 85.8 | [0.623; 0.870] |
| Calponin-1 + PEBP1 + HSP27 | 0.765 | -1.684 | 76.5 | 71.4 | 76.4 | 71.5 | [0.619 ; 0.867] |
| Calponin-1 + PEBP1 + TSP2 | 0.764 | -2.71 | 94.1 | 60.7 | 74.4 | 89.5 | [0.618 ; 0.866] |
| Calponin-1 + VEGF + HSP27 | 0.764 | -0.879 | 82.4 | 67.9 | 75.7 | 76.1 | [0.621 ; 0.864] |
| Calponin-1 + PEBP1 + MFAP4 | 0.764 | -7.512 | 91.2 | 57.1 | 72.0 | 84.3 | [0.618 ; 0.866] |
| Calponin-1 + PCPE1 + SDF1 a | 0.763 | -3.512 | 70.6 | 71.4 | 75.0 | 66.7 | [0.627 ; 0.860] |

The accuracy of multimarker panel and its discriminatory power was evaluated using a Receiving Operating Characteristics (ROC) analysis. ROC curves are the graphical visualization of the reciprocal relation between the sensitivity (Se) and the specificity (Sp) of a test for various values. AUC means area under the curve, with its confidence interval (CI). ROC Curves are based on models of prediction of plaque vulnerability by calculating optimal threshold of sensitivity (Se %) and specificity (Sp %) for multimarker panel. Positive (PPV, %) and negative (NPV, %) predictive values for combined marker were calculated to evaluate the proportion of true presence [true positive /(true positive+ false positive] and true absence [true negative /(true negative+ false negative)] of plaque rupture in ACS patients. As example, the equations for the marker combinations calculated by the mROC program were: a) Z = 0.541x[Calponin-1] - 1.061x[PEBP1] - 1.036x[IL1ra], b) Z = 0.593x[Calponin1] - 0.756x[VEGF] - 0.737x[PCPE1], c) Z = 0.580x[Calponin1] - 0.672x[VEGF] - 0.878x[PEBP1], d) Z = 0.544x[Calponin1] - 1.518x[VEGF] + 1.254x[IL13], e) Z = 0.560x[Calponin1] - 0.918x[PEBP1] - 0;460x[IL12p70], f) Z = 0.542x[Calponin1] - 1.046x[IL1ra] - 0.830x[PCPE1], g) Z = 0.578x[Calponin1] - 0.892x[PEBP1] - 1.511x[SDF1a], h) Z = 0.638x[Calponin1] - 0.751x[VEGF] - 1.750x[SDF1a], i) Z = 0.514x[Calponin1] - 0.730x[HSP27] - 0.929x[IL1ra], j) Z = 0.566x[Calponin1] - 0.676x[HSP27] - 1.652x[SDF1 a], k) Z = 0.514x[Calponin1] - 0.900x[PEBP1] - 0.452x[TSP2], I) Z = 0.509x[Calponin1] - 0.454x[IL8] - 0.716x[PCPE1], m) Z = 0.513x[Calponin1] - 0.534x[IL8] - 1.011x[PEBP1], n) Z = 0.583x[Calponin1] - 1.834x[SDF1a] - 0.948x[IL1ra], o) Z = 0.557x[Calponin1] - 0.651x[VEGF] - 0.571x[HSP27], p) Z = 0.513x[Calponin1] - 0.793x[PEBP1] - 0.470x[HSP27],, q) Z = 0.508x[Calponin1] - 0.981x[PEBP1] - 1.724x[MFAP4] and r) Z = 0.563x[Calponin1] - 0.599x[PCPE1] - 1.315x[SDF1a]. Using these equations a new virtual marker (Z) was calculated with significant diagnostic accuracy (p<0.05).

### Logistic regression: univariable and multivariable analyses

Logistic regression was carried out using forward and backward stepwise elimination for variable selection to identify the most important explanatory variables that significantly distinguished stable to unstable patients or stable patients to ACS patients. As example, a series of markers were significantly altered in ACS patients as compared to stable angina patients (Table 38). Multimarker analysis (with 0.5 as threshold) using logistic regression confirmed previous statistical analysis; Logit analysis revealed that combination of atherosclerotic marker improved discrimination between stable patients and unstable or ACS patients (Table 39).

**Table 38: Diagnostic accuracy of single marker using logistic regression (stable versus ACS patients) with backward stepwise elimination (p<0.20)**

| **Analysis of Maximum Likelihood Estimates** | | | | | |
|---|---|---|---|---|---|
| Parameter | DF | Estimate | Standard Error | Wald Chi-Square | Pr > ChiSq |
| Intercept | 1 | 7.2837 | 8.9390 | 0.6639 | 0.4152 |
| TSP2 | 1 | -4.2060 | 2.0583 | 4.1756 | 0.0410 |
| HSP27 | 1 | -2.7321 | 1.4189 | 3.7074 | 0.0542 |
| Calponin-1 | 1 | 1.8420 | 0.6162 | 8.9366 | 0.0028 |
| IFNa2 | 1 | 8.5517 | 5.0970 | 2.8150 | 0.0934 |
| IL1ra | 1 | -3.1982 | 1.5793 | 4.1009 | 0.0429 |
| IL13 | 1 | 3.0253 | 1.5622 | 3.7504 | 0.0528 |
| VEGF | 1 | -2.2682 | 1.3631 | 2.7687 | 0.0961 |

The accuracy of single marker and its discriminatory power was evaluated using a logistic regression, using a differential biomarker expression at p<0.20.

**Table 39: Diagnostic accuracy of multimarker panel (3 markers) using logistic regression (stable versus ACS patients) with backward stepwise elimination (p<0.20)**

| **LOGIT regression** | **AUC ROC** | **Se (%)** | **Sp (%)** |
|---|---|---|---|
| Calponin-1+PEBP1+IL1ra | 0.815 | 82.4 | 60.7 |
| Calponin-1+PEBP1+IL12p70 | 0.787 | 76.5 | 57.1 |
| Calponin-1+PEBP1+VEGF | 0.787 | 76.5 | 64.3 |
| Calponin-1+PCPE1+IL1ra | 0.785 | 79.4 | 53.6 |
| Calponin-1+PCPE1+VEGF | 0.785 | 76.5 | 50.0 |
| Calponin-1+IL13+VEGF | 0.784 | 88.2 | 67.9 |
| Calponin-1+PEBP1+SDF1a | 0.780 | 82.4 | 53.6 |
| Calponin-1+SDF1a+VEGF | 0.778 | 79.4 | 64.3 |
| Calponin-1+PCPE1+IL8 | 0.776 | 76.5 | 50.0 |
| Calponin-1+HSP27+IL1ra | 0.776 | 82.4 | 64.3 |

The accuracy of multimarker panel and its discriminatory power were evaluated using a logistic regression. Receiving Operating Characteristics (ROC) curves are the graphical visualization of the reciprocal relation between the sensitivity (Se) and the specificity (Sp) of a test for various values. AUC means area under the curve.

### Example 5

This example shows the results of a clinical study in post-mortem coronary patients.

### Material and methods

### Clinical population and marker testing

The study protocol was approved by the institutional review committee and human samples were provided by CVPath Institute Inc. (Gaithersburg, MD, US). After sudden death, coronary arteries were dissected for histological analysis to detect the presence or absence of acute thrombosis, the degree of luminal stenosis, disruption of fibrous cap and the presence of inflammatory cells. Death from coronary causes was evaluated to exclude non-cardiac causes of death. According to Burke et al. (N Engl J med 1997; 336:1276-1282), a vulnerable plaque was characterized when thin fibrous cap (>65 µm) and macrophages were present with or without rupture. Stable plaque was defined as severe coronary disease with cross-sectional luminal narrowing >75% with no thrombosis. Using this classification, 30 cases of acute thrombosis resulting from plaque rupture of a vulnerable plaque and 30 cases of stable plaque were included in the study. EDTA plasmas from patients with coronary disease who died suddenly were assessed to determine the levels of circulating biomarkers. The clinical characteristics of individuals are reported in Table 40.

All markers listed in the Table 31 except Calponin-1, Aciculin, HSP27, MFAP4, PEBP1, PRDX2 and OGN were measured using validated immunoassays in EDTA plasmas from males and females who died suddenly. Statistical analyses (univariate and multivariate analyses) were performed extensively to compare stable plaque to ruptured plaque conditions. Large univariable and multivariable analyses were applied on patients who died suddenly, by comparing the cases with acute thrombosis (ruptured plaque) to cases with severe coronary disease (stable plaque).

### Results

### Demographic analysis of individuals who died suddenly

As illustrated in Table 40, cases classified with severe coronary disease (stable plaque) were statistically comparable to cases identified as having a ruptured plaque for age, body weight, height, total plaque burden, gender and race.

**Table 40: Baseline demographic of individuals who died suddenly while having a stable or ruptured plaque**

| | **Stable** | **Rupture** | **Stable versus Rupture** |
|---|---|---|---|
| | Mean ± SD | Mean ± SD | p-value |
| Mean age,year (Min-Max) | 53.17 ± 09.66 (36-77) | 51.31 ± 13.95 (31-87) | 0.556 |
| Body weight (Min-Max) | 206.20 ± 43.70 (126-301) | 212.90 ± 51.96 (147-386) | 0.591 |
| Height (Min-Max) | 69.57 ± 03.30 (62-78) | 69.57 ± 03.27 (62-75) | 1.000 |
| Total plaque burden (Min-Max) | 234.31 ± 64.50 (80-320) | 232.04 ± 76.49 (0-305) | 0.905 |
| Gender, male/female (%) | 30/0 (100.0/0.0) | 27/03 (90.0/10.0) | 0.075 |
| Ethnicity A American, n (%) | 02 (6.67) | 0(0) | ND |
| Ethnicity caucasian, n (%) | 15 (50.00) | 25 (83.33) | 0.113 |
| Ethnicity Black, n (%) | 12 (40.00) | 05 (16.67) | 0.089 |
| Ethnicity Hispanic, n (%) | 1 (3.33) | 0(0) | ND |

| | | | |
|---|---|---|---|
| *SD: standard deviation. ND: not determined.* | | | |

### Univariable and multivariable analyses of biomarkers

As example, when comparing cases with stable plaques (n=30) to cases with ruptured plaques (n=30), circulating IL-13, GCSF, IL-7, II-12 and TIMP-1 were significantly altered. Based on Receiving-Operating-Characteristic (ROC) analysis for circulating marker levels, the area under the curve (AUC) for individual marker, such as IL-13, GCSF, IL-7, II-12 and TIMP-1, allowed discriminating individuals with stable plaque from those with ruptured plaque (Table 41). As illustrated in the next tables, marker combination improved the diagnostic accuracy between..stable and ruptured plaques (Tables 42 and 43).

**Table 41: Discriminative performance of single marker in cases who died suddenly while having a stable or ruptured plaque**

| **Markers** | **AUC** | **threshold** | **Se %** | **Sp %** | **PPV %** | **NPV %** | **CI 95%** |
|---|---|---|---|---|---|---|---|
| IL13 | 0.693 | 0.905 | 73.3 | 70.0 | 71.0 | 72.4 | [0.542 ; 0.812] |
| GCSF | 0.677 | -2.201 | 83.3 | 53.3 | 64.1 | 76.1 | [0.529 ; 0.796] |
| TIMP1 | 0.628 | 2.458 | 73.3 | 56.7 | 62.9 | 68.0 | [0.479 ; 0.756] |
| IL12p70 | 0.628 | 1.602 | 43.3 | 86.7 | 76.5 | 60.5 | [0.480 ; 0.755] |
| VEGF | 0.628 | 1.872 | 70.0 | 56.7 | 61.8 | 65.4 | [0.479 ; 0.755] |

The accuracy of each marker and its discriminatory power was evaluated using a Receiving Operating Characteristics (ROC) analysis. ROC curves are the graphical visualization of the reciprocal relation between the sensitivity (Se) and the specificity (Sp) of a test for various values. AUC means area under the curve, with its confidence interval (CI). ROC Curves are based on models of prediction of plaque vulnerability by calculating optimal threshold of sensitivity (Se %) and specificity (Sp%) for single marker. Positive (PPV, %) and negative (NPV, %) predictive values for single marker were calculated to evaluate the proportion of true presence [true positive /(true positive+ false positive] and true absence [true negative /(true negative+ false negative)] of plaque rupture in unstable patients.

**Table 42: Discriminative performance of two-marker combination in cases who died suddenly while having a stable or ruptured plaque**

| **Marker combination** | **AUC** | **threshold** | **Se %** | **Sp %** | **PPV %** | **NPV %** | **CI 95%** |
|---|---|---|---|---|---|---|---|
| IL8 + GCSF | 0.749 | -0.182 | 60.0 | 80.0 | 75.0 | 66.7 | [0.609 ; 0.851] |
| TIMP1 + GCSF | 0.729 | 2.278 | 80.0 | 60.0 | 66.7 | 75.0 | [0.588 ; 0.835] |
| TIMP1 + IL13 | 0.722 | 3.897 | 66.7 | 80.0 | 76.9 | 70.6 | [0.575 ; 0.833] |
| IL13 + GCSF | 0.721 | -0.394 | 66.7 | 66.7 | 66.7 | 66.7 | [0.580 ; 0.829] |
| PCPE1 + GCSF | 0.720 | -3.37 | 63.3 | 80.0 | 76.0 | 68.6 | [0.577 ; 0.829] |
| IL1beta + IL13 | 0.716 | 0.59 | 66.7 | 73.3 | 71.4 | 68.8 | [0.570 ; 0.827] |
| IL12p70 + GCSF | 0.714 | -0.712 | 73.3 | 56.7 | 62.9 | 68.0 | [0.573 ; 0.824] |
| VEGF + GCSF | 0.708 | -0.772 | 73.3 | 56.7 | 62.9 | 68.0 | [0.565 ; 0.819] |
| DJ1 + GCSF | 0.698 | -3.6313 | 86.7 | 50.0 | 63.4 | 79.0 | [0.553 ; 0.812] |

As example, the equations for the marker combinations calculated by the mROC program were: a) Z = 0.863x[IL8] - 1.171x[GCSF], b) Z = 1.612x[TIMP1] - 0.818x[GCSF], c) Z = 1.175x[TIMP1] + 0.948x[IL13], d) Z = 0.725x[IL13] - 0.583x[GCSF], e) Z =-0.578x[PCPE1] - 0.674x[GCSF], f) Z = -0.370x[IL1beta] + 0.901x[IL13], g) Z = 0.510x[IL12p70] - 0.657x[GCSF], h) Z = 0.253x[VEGF] - 0.615x[GCSF] and i) Z =-0.389x[DJ1] - 0.684x[GCSF]. Using these equations a new virtual marker (Z) was calculated with significant diagnostic accuracy (p<0.05).

**Table 43: Discriminative performance of multimarker panel in cases who died suddenly while having a stable or ruptured plaque**

| **Marker combination** | **AUC** | **threshold** | **Se %** | **Sp %** | **PPV %** | **NPV %** | **CI 95%** |
|---|---|---|---|---|---|---|---|
| PCPE1 + IL8 + GCSF | 0.788 | -2.606 | 70.0 | 80.0 | 77.8 | 72.7 | [0.652 ; 0.880] |
| IL8 + IL13 + GCSF | 0.780 | 0.482 | 70.0 | 76.7 | 75.0 | 71.9 | [0.644 ; 0.874] |
| DJ1 + IL8 + GCSF | 0.780 | -5.754 | 76.7 | 66.7 | 69.7 | 74.1 | [0.646 ; 0.873] |
| TIMP1 + IL13 + GCSF | 0.776 | 3.857 | 56.7 | 86.7 | 81.0 | 66.7 | [0.642 ; 0.869] |
| TIMP1 + IL8 + GCSF | 0.767 | 2.617 | 73.3 | 73.3 | 73.3 | 73.3 | [0.629 ; 0.864] |
| GROalpha + IL8 + GCSF | 0.762 | -1.226 | 80.0 | 63.3 | 68.6 | 76.0 | [0.624 ; 0.861] |
| IL8 + IL12p70 + GCSF | 0.761 | 0.059 | 56.7 | 86.7 | 81.0 | 66.7 | [0.624 ; 0.860] |
| TIMP1 + IL13 + GROalpha | 0.761 | 3.336 | 50.0 | 93.3 | 88.2 | 65.1 | [0.623 ; 0.860] |

As example, the equations for the marker combinations calculated by the mROC program were: a) Z = -0.650x[PCPE1] + 0.890x[IL8] - 1.194x[GCSF], b) Z = 0.818x[IL8] + 0.669x[IL13] - 1.067x[GCSF], c) Z = -1.008x[DJ1] + 1.093x[IL8] - 1.349x[GCSF], d) Z = 1.758x[TIMP1] + 0.799x[IL13] - 0.739x[GCSF], e) Z = 1.306x[TIMP1] + 0.741x[IL8] - 1.222x[GCSF], f) Z = -0.518x[GROalpha] + 0.974x[IL8] - 1.075x[GCSF], g) Z = 0.689x[IL8] + 0.310x[IL12p70] - 1.064x[GCSF] and h) Z = 2.114x[TIMP1] + 0.899x[IL13] - 1.186x[GROalpha]. Using these equations a new virtual marker (Z) was calculated with significant diagnostic accuracy (p<0.05).

## Claims

1. A method for determining the presence of an unstable atherosclerotic plaque in an individual, comprising the steps of:
a1) measuring the presence and/or level of at least one biochemical marker in a biological sample obtained from said individual, said biochemical marker being microfibril-associated protein-4;
b1) based on the result of the measurement in step a1), determining the presence of an unstable atherosclerotic plaque in the individual.

2. The method according to claim 1, wherein step a1) comprises measuring the presence and/or level of (i) calponin-1 and (ii) at least one biochemical marker in a biological sample obtained from said individual, said biochemical marker being microfibril-associated protein-4.

3. The method according to claim 2, wherein step a1) comprises measuring the presence and/or level of
(i) calponin-1,
(ii) microfibril-associated protein-4, and
(iii) at least one other biochemical marker selected from the group consisting of:
- alpha B crystallin,
- phosphatidylethanolamine-binding protein 1,
- aciculin,
- mature cathepsin K,
- osteoglycin,
- peroxiredoxin-2,
- dihydropyrimidinase-like 3,
- serum amyloid P component,
- serpin B9,
- serpin B9 -granzyme B complex,
- pro-cathepsin D,
- thrombospondin-2,
- thrombospondin-1,
- pro-collagen C-endopeptidase enhancer-1,
- DJ-1 oncogene,
- tissue inhibitor of metalloproteinase 1,
- heat shock protein 27,
- IL-12,
- vascular endothelial growth factor,
- IL-8,
- IL-1ra,
- granulocyte colony-stimulating factor,
- growth-regulated alpha protein,
- IL-13,
- leukemia inhibitory factor,
- IL-1 alpha,
- IL-1 beta,
- interferon alpha-2, and
- stromal cell derived factor 1 alpha.

4. The method according to any one of claims 1 to 3, wherein step a1) is followed by a step a2) of comparing the presence and/or level of the at least one biochemical marker with at least one predetermined value, and wherein step b1) consists in determining the presence of an unstable atherosclerotic plaque in the individual, based on the comparison in step a2).

5. The method according to any one of claims 1 to 4, wherein the biological sample is a liquid biological sample.

6. The method according to any one of claims 1 to 4, wherein the biological sample is a tissue sample, preferably an artery sample.

7. A method for diagnosing a condition in an individual, said method comprising the step of determining the presence of an unstable atherosclerotic plaque using the method as defined in any one of claims 1 to 6, the presence of an unstable atherosclerotic plaque being indicative of said condition, wherein said condition is (i) a vascular or metabolic disease, or (ii) an increased predisposition to an adverse outcome.

8. A method for evaluating the likelihood that an individual will benefit from a treatment with an agent to reduce the risk of cardiovascular disease, comprising the step of determining the presence of an unstable atherosclerotic plaque by the method as defined in any one of claims 1 to 6, the presence of an unstable atherosclerotic plaque being indicative of the likelihood of a benefit from the treatments.

## Patentansprüche

1. Verfahren zum Ermitteln des Vorhandenseins einer instabilen atherosklerotischen Plaque bei einem Individuum, das die Schritte umfasst:
a1) Messen des Vorhandenseins und/oder des Spiegels zumindest eines biochemischen Markers in einer biologischen Probe, die vom Individuum erhalten wurde, wobei der biochemische Marker mikrofibrillenassoziiertes Protein-4 ist;
b1) Ermitteln des Vorhandenseins einer instabilen atherosklerotischen Plaque beim Individuum auf Basis des Ergebnisses der Messung in Schritt a1).

2. Verfahren nach Anspruch 1, wobei Schritt a1) das Messen des Vorhandenseins und/oder des Spiegels von (i) Calponin-1 und (ii) zumindest eines biochemischen Markers in einer biologischen Probe umfasst, die vom Individuum erhalten wurde, wobei der biochemische Marker mikrofibrillenassoziiertes Protein-4 ist.

3. Verfahren nach Anspruch 2, wobei Schritt a1) das Messen des Vorhandenseins und/oder des Spiegels umfasst von:
(i) Calponin-1
(ii) mikrofibrillenassoziiertem Protein-4, und
(iii) zumindest einem anderen biochemischen Marker, der aus der Gruppe ausgewählt ist, bestehend aus:
- Alpha-B-Kristallin
- phosphatidylethanolaminbindendem Protein 1
- Aciculin
- reifem Kathepsin K
- Osteoglycin
- Peroxiredoxin-2
- Dihydropyrimidinase-like 3
- Serum-Amyloid-P-Komponente
- Serpin B9
- Serpin-B9-Granzym-B-Komplex
- Prokathepsin D
- Thrombospondin-2
- Thrombospondin-1
- Prokollagen-C-Endopeptidase-Enhancer-1
- DJ-1-Onkogen
- Gewebehemmer von Metalloproteinase 1
- Hitzeschockprotein 27
- IL-12
- vaskulärem endothelialem Wachstumsfaktor
- IL-8
- IL-1ra
- Granulozytenkolonie stimulierendem Faktor
- wachstumsreguliertem Alpha-Protein
- IL-13
- Leukämiehemmungsfaktor
- IL-1 alpha
- I L-1 beta
- Interferon alpha 2, und
- stromazellenabgeleitetem Faktor 1 alpha.

4. Verfahren nach einem der Ansprüche 1 bis 3, wobei auf Schritt a1) Schritt a2) des Vergleichens des Vorhandenseins und/oder des Spiegels des zumindest einen biochemischen Markers mit zumindest einem vordefinierten Wert folgt, und wobei Schritt b1) aus dem Ermitteln des Vorhandenseins einer instabilen atherosklerotischen Plaque beim Individuum auf Basis des Vergleichs in Schritt a2) besteht.

5. Verfahren nach einem der Ansprüche 1 bis 4, wobei die biologische Probe eine flüssige biologische Probe ist.

6. Verfahren nach einem der Ansprüche 1 bis 4, wobei die biologische Probe eine Gewebeprobe ist, vorzugsweise eine Arterienprobe.

7. Verfahren zum Diagnostizieren eines Zustands bei einem Individuum, wobei das Verfahren den Schritt des Ermittelns des Vorhandenseins einer instabilen atherosklerotischen Plaque unter Verwendung des Verfahrens nach einem der Ansprüche 1 bis 6 umfasst, wobei das Vorhandensein einer instabilen atherosklerotischen Plaque für den Zustand indikativ ist, wobei der Zustand (i) eine Gefäß- oder Stoffwechselkrankheit oder (ii) eine erhöhte Prädisposition für ein unerwünschtes Ergebnis ist.

8. Verfahren zum Beurteilen der Wahrscheinlichkeit, dass ein Individuum von einer Behandlung mit einem Mittel profitiert, um das Risiko für Herz-Kreislauf-Krankheiten zu verringern, das den Schritt des Ermittelns des Vorhandenseins einer instabilen atherosklerotischen Plaque unter Verwendung des Verfahrens nach einem der Ansprüche 1 bis 6 umfasst, wobei das Vorhandensein einer instabilen atherosklerotischen Plaque für die Wahrscheinlichkeit, dass aus der Behandlung ein Nutzen entsteht, indikativ ist.

## Revendications

1. Procédé de détermination de la présence d'une plaque athérosclérotique instable chez un individu, comprenant les étapes suivantes :
a1) la mesure de la présence et/ou du niveau d'au moins un marqueur biochimique dans un échantillon biologique obtenu à partir dudit individu, ledit marqueur biochimique étant la protéine 4 associée aux microfibrilles ;
b1) à partir du résultat de la mesure à l'étape a1), la détermination de la présence d'une plaque athérosclérotique instable chez l'individu.

2. Procédé selon la revendication 1, dans lequel l'étape a1) comprend la mesure de la présence et/ou du niveau de (i) la calponine-1 et (ii) au moins un marqueur biochimique dans un échantillon biologique obtenu à partir dudit individu, ledit marqueur biochimique étant la protéine 4 associée aux microfibrilles.

3. Procédé selon la revendication 2, dans lequel l'étape a1) comprend la mesure de la présence et/ou du niveau de
(i) la calponine-1,
(ii) la protéine 4 associée aux microfibrilles et
(iii) au moins un autre marqueur biochimique choisi dans le groupe constitué par :
- l'alpha-B-cristalline,
- la protéine 1 de liaison à la phosphatidyléthanolamine,
- l'aciculine,
- la cathepsine K mature,
- l'ostéoglycine,
- la peroxyrédoxine 2,
- la dihydropyrimidinase-like 3,
- le composant sérique amyloïde P,
- la serpine B9,
- le complexe serpine B9-granzyme B,
- la pro-cathepsine D,
- la thrombospondine 2,
- la thrombospondine 1,
- l'amplificateur 1 d'endopeptidase C pro-collagène,
- l'oncogène DJ-1,
- l'inhibiteur tissulaire de métalloprotéinase 1,
- la protéine de choc thermique 27,
- IL-12,
- le facteur de croissance endothélial vasculaire,
- IL-8,
- IL-1ra,
- le facteur de stimulation des colonies granulocytaires,
- la protéine alpha régulée par la croissance,
- IL-13,
- le facteur d'inhibition de la leucémie,
- IL-1 alpha,
- IL-1 bêta,
- l'interféron alpha-2 e
- le facteur 1 alpha dérivé de cellules stromales.

4. Procédé selon l'une quelconque des revendications 1 à 3, dans lequel l'étape a1) est suivie par une étape a2) de comparaison de la présence et/ou du niveau dudit au moins un marqueur biologique avec au moins une valeur prédéterminée, et dans lequel l'étape b1) consiste en la détermination de la présence d'une plaque athérosclérotique instable chez l'individu à partir de la comparaison à l'étape a2).

5. Procédé selon l'une quelconque des revendications 1 à 4, dans lequel l'échantillon biologique est un échantillon biologique liquide.

6. Procédé selon l'une quelconque des revendications 1 à 4, dans lequel l'échantillon biologique est un échantillon de tissu, de préférence un échantillon d'artère.

7. Procédé de diagnostic d'un état chez un individu, ledit procédé comprenant l'étape de détermination de la présence d'une plaque athérosclérotique instable en utilisant le procédé tel que défini dans l'une quelconque des revendications 1 à 6, la présence d'une plaque athérosclérotique instable étant indicatrice dudit état, ledit état étant (i) une maladie vasculaire ou métabolique ou (ii) une prédisposition augmentée à un résultat négatif.

8. Procédé d'évaluation de la probabilité qu'un individu bénéficie d'un traitement avec un agent pour réduire le risque de maladie cardiovasculaire, comprenant l'étape de détermination de la présence d'une plaque athérosclérotique instable en utilisant le procédé tel que défini dans l'une quelconque des revendications 1 à 6, la présence d'une plaque athérosclérotique instable étant indicatrice de la probabilité de bénéficier du traitement.
